# NEW EUROPEAN PATENT SPECIFICATION

(11) **EP 1 444 346 B2**
(45) Date of publication and mention of the opposition decision: **24.06.2015**
(45) Mention of the grant of the patent: 12.03.2008
(21) Application number: 02776950.4
(22) Date of filing: 29.08.2002
(51) Int. Cl.: C12N 15/11, C12N 5/10, C12N 7/00, A61K 31/713

(54) **SIRNA KNOCKOUT ASSAY METHOD AND CONSTRUCTS**
SIRNA KNOCKOUT PRÜFVERFAHREN UND KONSTRUKTE
MÉTHODE DE TEST 'KNOCKOUT' À PETIT ARN INTERFÉRANT ET CONSTRUCTIONS

(30) Priority: 01.09.2001 US 317229 P; 04.06.2002 US 385733 P
(43) Date of publication of application: 11.08.2004
(73) Proprietor: Galapagos N.V., 2800 Mechelen (BE)
(72) Inventor: ARTS, Gert-Jan, NL-2403 ES Alphen AD Rijn (NL); LANGEMEIJER, Ellen, Vera, NL-2628 TG Delft (NL); PIEST, Ivo, NL-3645 VS Vinkeveen (NL); VAN ES, Helmuth, Hendrikus, Gerardus, NL-2011 ZZ Haarlem (NL); MICHIELS, Godefridus, Augustinus, Maria, NL-2352 EP Leiderdorp (NL)
(74) Representative: Nichol, Maria Zenarosa
(86) International application number: PCT/EP2002/009670
(87) International publication number: WO 2003/020931

(56) References cited:
- WO-A-00/11154
- WO-A-00/49035
- WO-A-00/52188
- WO-A-96/29097
- WO-A-99/64582
- WO-A-03/012082
- WO-A1-99/53050
- HUTVAGNER GYORGY ET AL: "A cellular function for the RNA-interference enzyme dicer in the maturation of the let-7 small temporal RNA." SCIENCE (WASHINGTON D C), vol. 293, no. 5531, 3 August 2001 (2001-08-03), pages 834-838, XP002204649 ISSN: 0036-8075
- PASQUINELLI AMY E ET AL: "Conservation of the sequence and temporal expression of let-7 heterochronic regulatory RNA." NATURE (LONDON), vol. 408, no. 6808, 2000, pages 86-89, XP002204652 ISSN: 0028-0836 cited in the application
- ELBASHIR SAYDA M ET AL: "RNA interference is mediated by 21- and 22-nucleotide RNAs." GENES & DEVELOPMENT, vol. 15, no. 2, 15 January 2001 (2001-01-15), pages 188-200, XP002204651 ISSN: 0890-9369
- MEDINA M F C ET AL: "RNA POLYMERASE III-DRIVEN EXPRESSION CASSETTES IN HUMAN GENE THERAPY" CURRENT OPINION IN MOLECULAR THERAPEUTICS, vol. 1, no. 5, October 1999 (1999-10), pages 580-594, XP001055319 ISSN: 1464-8431
- PADDISON PATRICK J ET AL: "Short hairpin RNAs (shRNAs) induce sequence-specific silencing in mammalian cells." GENES & DEVELOPMENT, vol. 16, no. 8, 15 April 2002 (2002-04-15), pages 948-958, XP002204653 ISSN: 0890-9369
- YU JENN-YAH ET AL: "RNA interference by expression of short-interfering RNAs and hairpin RNAs in mammalian cells." PROCEEDINGS OF THE NATIONAL ACADEMY OF SCIENCES OF THE UNITED STATES, vol. 99, no. 9, 30 April 2002 (2002-04-30), pages 6047-6052, XP002204654 ISSN: 0027-8424
- RAYKOV Z ET AL: "Transient suppression of transgene expression by means of antisense oligonucleotides: A method for the production of toxin-transducing recombinant viruses." GENE THERAPY, vol. 9, no. 5, March 2002 (2002-03), pages 358-362, XP002253225 ISSN: 0969-7128
- YU, J-Y ET AL.: 'RNA interference by expression of short-interfering RNAs and hairpin RNAs in mammalian cells' PNAS USA vol. 99, no. 9, 30 April 2002, pages 6047 - 6052
- BRUMMELKAMP T. R. ET AL.: 'A system for stable expression of short interfering RNAs in mammalian cells' SCIENCE vol. 296, no. 5567, 19 April 2002, pages 550 - 553
- MICHAEL T. ET AL.: 'Gene silencing using micro-RNA designed hairpins' RNA vol. 8, no. 6, 06 June 2002, pages 842 - 850
- SUI G. ET AL.: 'A DNA vector-based RNAi technology to suppress gene expression in mammalian cells' PNAS USA vol. 99, no. 8, 16 April 2002, pages 5515 - 5520

## Description

### Field of the Invention

The present invention relates to polynucleotide vector constructs, methods for their preparation, and preparations for their use in methods that lower the amount of RNA and/or protein production in cells based on the intracellular expression of small interfering polyribonucleic acid molecules.

Genomics research over the last decades has resulted in a nearly complete map of all human genes and opened-up new directions in medical research. In this post-genomics era new disciplines of science have emerged such as proteomics and functional genomics. Traditional pharmaceutical companies with substantial R&D budgets are interested in getting access to new functional genomics and proteomics platform technologies. What is needed is better screening technologies for identification of new therapeutic targets as well as better target validation approaches. With applications in all disciplines of modern medicine, functional genomics has the potential to make a significant difference for the treatment of all human diseases.

Pharmaceutical companies are interested in reliable knockdown based technologies since their drug screens with small molecules are based on inhibiting the activity and effect of an expressed protein. Therefore, blocking expression or function of a potential target, either through screening in a cellular assay or through single gene validation will provide an important data set regarding drug-ability of the target early on in the drug development process. This data set forms a strong basis for the start of a drug development program, based on a compound, antibody or biological, with the aim to develop an effective therapy.

The study of gene function in vertebrates is hampered by the complexity of the genome, the multicellular nature and the lack of extensive genetic tools. The techniques to generate stable transgenic cell-lines or transgenic mice are powerful but very time- and labor-intensive approaches that cannot be easily performed at high throughput.

Various knockdown or knockout approaches are used to study gene function in mammalian cells (e.g. antisense, antibodies, ribozymes, aptamers, zinc finger proteins, chimeric RNA-DNA oligos, etc.). However, these technologies are not robust and efficient nor they can be generically applied to all genes and all cell types.

RNA interference (RNAi) is the post-transcriptional process of gene silencing mediated by double stranded RNA (dsRNA) that is homologous in sequence to the silenced RNA and is observed in animals and plants. The dsRNA is processed into 21-23 nucleotides (nts) molecules, called small interfering RNAs (siRNAs), which guide the sequence-specific degradation of the target RNA (Sharp, 2001).

The initial discovery of RNA interference in C. *elegans* (Fire et al., 1998) has been followed by numerous examples of organisms where introduction of dsRNA can induce the sequence specific silencing effect.

The development of an *in vitro* system using extracts of embryos or cultured cells of drosophila has accelerated the insight into the mechanism behind the sequence specific silencing effect (Elbashir et al., 2001b; Hammond et al., 2000; Zamore et al., 2000). The dsRNA that is introduced into these extracts are processed into 21-23 nts fragments. The extracts are now "programmed" to degrade target RNAs with sequences overlapping to the dsRNA fragments. The target RNA is cleaved in both strands, sense and antisense, at 21-23 nts intervals. These smaller species are referred to as short interfering RNAs (siRNAs). Al though longer dsRNA species appear to be more potent than shorter RNAs, the specific silencing effect is obtained by transfecting the 21-23 nts siRNA directly into the cells (Elbashir et al., 2001 b).

Mature siRNA duplexes are precisely processed to form a duplexed RNA of 21-23 nts long with 3' overhangs of 2- or 3-nts; they do not contain modified nucleotides and have a 5' phosphate (not essential for its function) and a 3' hydroxyl group. A siRNA duplex with 2- or 3-nts overhangs is more active than duplexes with blunt ends or 4-nts overhangs. Extensions at the 3' terminus of 17 or-up nts at either the sense strand or the antisense strand results in a loss of activity to cleave the complementary target strand. This indicates that the correct 3' terminus of the antisense strand is essential for the maintaining the activity of the siRNA duplex to degrade the sense target RNA (Elbashir et al., 2001 b).

Genetic studies have linked 151A.1 to transposon silencing in C. *elegans.* Co-suppression by posttranslational gene silencing (PTGS) in plants seems to function by a related mechanism mediated by small guide RNAs of approximately 22 nts.

Several protein factors have been associated with RNAi, based on genetic and biochemical studies (Sharp, 2001). The RDB-1 gene family consists of a large number of members (24 in *C*. *elegans*) that is well conserved. Members of the 9.DE-1 family contain conserved PIWI- and PAZ-domains of unknown functions.

RDE-1 homologues are found in various species and RDE-1 family members have been implicated to act in various processes; RNAi, PTGS in plants, embryogenesis in Drosophila, expression and regulation of small temporal RNAs.

The Dicer protein is a member of the RNase III family that is conserved in several species. Dicer contains a helicase domain, 1-2 dsRNA binding domains, 2 RNase III type domains and a PAZ domain. Dicer is required for the generation of the functional 21 nucleotides long siRNAs from longer dsRNA complexes (Bernstein et al., 2001).

In *C*. *elegans let-7* and *lin-4*, small temporal RNAs (stRNAs) of 21-22 nts, play a regulatory role during development. They do so by recognizing sequences in the 3' untranslated regions of their target transcripts resulting in strong repression of expression (Reinhart et al., 2000). The stRNAs are processed from longer precursor transcripts. Mature *let*-*7* RNA has also been detected in humans and precursors with conserved secondary structures have been predicted (Pasquinelli et al., 2000).

For the correct processing of the stRNA let-7, Dicer is required (Grishok et al., 2001; Hutvagner et al., 2001). Therefore, Dicer has a dual function: processing of dsRNA into siRNA as well as processing of let-7 precursors into mature let-7 stRNA. The products, the stRNA and the siRNA, have some characteristics that are different and some characteristics that are shared.

The differences between stRNA and siRNA include (1) both sense and antisense strands of mature siRNAs are present in the cell; only the antisense strand of mature stRNAs is detectable, (2) the duplexed region of stRNAs contains some G-U base pairs and mismatches in contrast to siRNAs that have 100% complementary duplexes, and (3) the mode of action for siRNA is RNA degradation, while let-7 stRNA is believed to result in a translational block.

stRNAs and siRNAs share the characteristics that (1) both RNA species are involved in repression of gene expression, (2) both, mature siRNAs and mature stRNAs are 21-22 nts long, (3) both are produced from duplexed, longer precursor RNAs, and (4) the processing into active forms of both stRNA and siRNA is mediated by the same enzyme, Dicer.

### Reported Developments

RNAi provides researchers with an additional genetic tool to study gene functions. In C. *elegans,* chromosomes I and III have now systematically been analyzed for phenotypic effects. The RNAi approach creates extra possibilities in developmental studies. Classical knockouts with lethal effects during development could never be analyzed in later developmental stages. With RNAi, the onset of the effect may be varied and roles in later stages of development may be studied.

The use of RNAi in mammalian cells has been problematic since introduction of long (>30 base pairs) dsRNA results in two major intracellular responses: activation of the double stranded RNA dependent protein kinase PKR, which results in a general block of protein synthesis, and activation via 2'-5'-oligoadenylate synthetase of RNase L, which attacks all mRNAs.

In mammals, the appearance of dsRNA in the cell, often generated during viral infections, results in strong cellular responses. A major activity is mediated by the interferon-inducible dsRNA-dependent protein kinase (PKR) that binds to dsRNA. This results in autophosphorylation and activation of PKR. The activated PKR phosphorylates the alpha subunit of the eukaryotic translation initiation factor 2 (elF2-alpha) at position serine-51. elF2 bound to GTP delivers the initiator tRNA methionine to the small ribosomal subunit and elF2 is released as the GDP-bound form. In order to get continuous ongoing translation, elF2 has to be recycled from the GDP- to the GTP-bound state. Phosphorylation of elF2-alpha by PKR prevents this recycling and thereby blocks the initiation of translation. As a consequence, dsRNA leads to a general translational block in mammalian cells.

dsRNA is also known to activate the interferon-induced (2'-5') oligoadenylate synthetase. Upon activation, this enzyme polymerizes ATP into 2'-5'-linked nucleotide oligomers (also indicated by 2-5A). The 2-5A oligomers activate the ribonuclease RNase L that results in RNA degradation.

Further, in mammals some mRNAs are edited by the nuclear dsRNA-specific adenosine deaminase (ADAR). Although ADAR acts selectively on particular substrates, like mRNAs for brain glutamate receptors (gluR), its activity shows very little sequence specificity and can act on any dsRNA molecule above a certain minimum length. This generic modifying activity results in deamination of adenosine- into inosine-residues resulting in unwinding of the dsRNA helix.

Indeed, transfection approaches of dsRNA that worked for drosophila-cultured cells failed for various cultured cells from mammalian origin. However, microinjection experiments in mouse embryos and oocytes showed that under these conditions RNAi effects could be observed. This suggested that RNAi in mammalian systems is possible.

Recently, it has been demonstrated that RNAi can be used in a panel of mammalian cell lines (Elbashir *et al*., 2001a). The approach is based on direct transfection of the 21-23 nts siRNA duplexes into the cells. This circumvents the intracellular responses mentioned above and results in sequence-specific silencing of endogenous and heterologous genes.

An important bottleneck in the siRNA transfection approach is its limited applicability to target different cell types, especially primary cells. Primary cells are closest to the in vivo situation and often have the highest physiological relevance. Non-viral DNA or siRNA transfection technologies have severe limitations with regard to these cells and are not efficient and reliable. Practical use of these approaches needs significant optimisation of conditions, and in general lack the robustness necessary for large-scale applications. The gene transfer reagents used are often toxic, yielding lower levels of viable transduced cells. In essence, they do not allow a generic siRNA application for a wide variety of cell types, including primary cell types such as T cells, B cells, mast cells, endothelial cells, synoviocytes and lung epithelial cells. Furthermore, transfection of the siRNA gives a short knock-down effect. For a prolonged knock-down effect in cells several additional transfections are necessary.

Therefore, a broad application of the siRNA technology will require further research and development to overcome these limitations. Genomics scale implementation of knocking down genes in mammalian cells has been hampered by the lack of a reliable, robust and efficient gene transfer technology (see above) applicable in a wide range of cell lines and primary cell types.

Hutvagner et al ((2001) Science 293:834-838) discloses a candidate RNA for the let-7 precursor which does form a hair pin structure, however, it differs from the present invention in having first and third sequences (RNA) which are 28-29 nt in length which contain mismatches thereby resulting in bulges.

The present invention overcomes the limitations recognized by the prior art, and finds applications in numerous fields, such as genomics studies, viral production and protein production.

The production of recombinant viruses is sometimes complicated by the expression of exogenous sequences that encode lethal or toxic proteins that interfere with viral production. The prior art discloses systems for the temporary shut-down of protein production including the Tet-repression system and the Bodyson system. These systems are however time-consuming and involve difficult cloning steps to introduce the constructs into the vectors. Another disadvantage of the prior art repression systems is that to express the exogenous gene, one often must add a compound that suppresses the suppressor system itself to turn on gene expression. The present invention may be applied to every viral packaging and protein production system to improve production by the selective knock-down of lethal or recombinant proteins during the viral or producing cell production phases respectively.

### Summary of the Invention

The present invention relates to a vector useful for transfecting host cells comprising an isolated polynucleotide useful for the degradation of a specific mRNA molecule in a host cell, comprising a first guide polynucleotide sequence consisting of 19 to 22 nucleotides and complementary to 19 to 22 nucleotides of said specific mRNA sequence in said host cell, said first sequence being covalently linked to a second sequence capable of forming a stem-loop structure when said second sequence is an RNA sequence, wherein said first sequence consists essentially of a single stranded DNA equivalent of an RNA sequence, and wherein said polynucleotide comprises further a third sequence consisting of 19 to 22 nucleotides complementary to said first sequence and covalently linked to the distal end of said second sequence and wherein all nucleotides in said first and third sequences base pair with each other, all of which nucleotides of said first and third sequences correspond to a sequence found in said specific mRNA, and wherein a pol III promoter is positioned upstream of said first sequence.

Another embodiment of the present invention relates to such vectors including a self-complementing single stranded polynucleotide, wherein said second nucleotide sequence comprises a stem-loop forming region having a sequence derived from naturally occurring RNA sequences found in RNA molecules and that does not functionally target a specific RNA molecule in a host cell. Most preferably the second sequences are derived from RNA molecules other than mRNA.

Another aspect of the present invention relates to an *in vitro* method for reducing the amount of at least one RNA molecule having a unique sequence present in a host cell comprising transfecting said cell with a vector according to the invention, wherein said polynucleotide comprises a first sequence which is complementary to said RNA sequence.

Another aspect of the present disclosure relates to a method for preparing a self-complementing single stranded polynucleotide including complementary sequences covalently linked by a polynucleotide sequence forming a stem loop structure, comprising treating a single stranded polynucleotide consisting essentially of a first polynucleotide sequence covalently linked to a second polynucleotide sequence that includes two nucleotide sequences capable of complementary base pairing and thereby forming a stem-loop structure that has a 3' OH terminus, under conditions such that said first sequence serves as a template starting a the 3' OH terminus for the synthesis of a complementary sequence thereto.

Another aspect of the present invention relates to a method of preparing a vector according to the invention including the sequence of a polynucleotide, wherein said self-complementing polynucleotide is a DNA sequence and further comprises a fourth sequence linked to the free end of said first sequence, and wherein said polynucleotide is denatured, converted into a double stranded polynucleotide, and ligated into a vector capable of transfecting a host cell and transcribing said polynucleotide.

Another aspect of the present invention relates to a method of determining the function of a naturally occurring polynucleotide sequence comprising transfecting a host cell with a vector according to the invention, said vector including a polynucleotide sequence complementary to said naturally occurring polynucleotide and detecting a change in cellular phenotype.

Other aspects of the invention relate to libraries of vectors according to the invention. Further aspects of the disclosure relate to methods of lowering the amounts of RNA or protein translated from RNA in a subject, comprising the administration of a vector according to the present invention, and transfecting cells in said subject, in an amount effective to lower the amounts of said RNA in said transfected cells.

The present invention provides for the temporary knock-down of proteins, such as lethal proteins, during virus or recombinant protein production, thereby allowing (1) the replication and packaging of virus that include sequences encoding for lethal proteins, or (2) the optimal production of recombinant protein. Knock-down constructs described herein below are transfected into any selected packaging cell and such transfected cells are used directly. The knock- down system uses virus constructs that are used directly to infect cells and no further compound is required by the method to induce virus or protein production.

### Brief Description of the Drawings

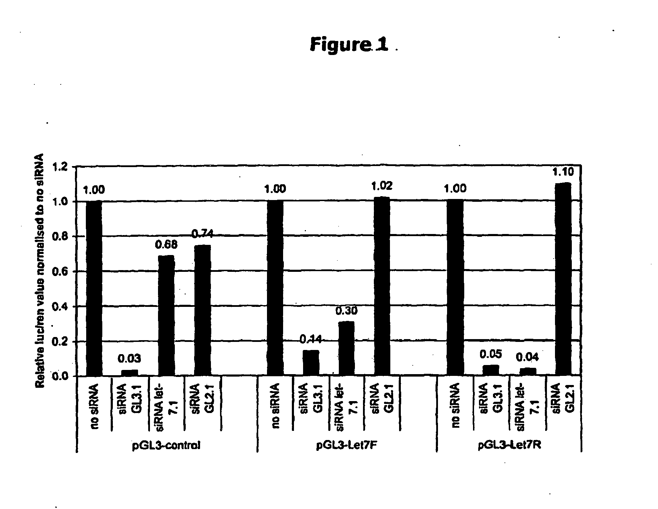

Figure 1 shows the repression of luciferase activity of pGL3-fusion constructs containing let-7 target sequences by let-7 siRNAs in PER.C6/E2A cells. PER.C6/E2A cells are transiently transfected with the pGL3-fusion constructs containing let-7 target sequences in either orientation (pGL3-tLet-7F, pGL3-tLet-7R) or pGL3-control lacking let-7 sequences, in combination with each of the siRNA duplexes siRNA GL3.1, siRNA let-7.1, siRNA GL2.1, or no siRNA. Co-transfection of siRNA let-7.1 specifically represses luciferase activity of the reporters pGL3-tLet7F and pGL3- tLet7R, but not the pGL3-control. Co-transfection of the positive control siRNA GL3.1 shows repression of all the reporter constructs (pGL3-tLet-7F, pGL3-tLet-7R, pGL3-control).
Figure 2 is a table presenting the results of a DNA database search using the C. *elegans* let-7 guide sequence as a probe. Three perfect matches were found on the human genome, in chromosomes 9, 11, 19, 21, 22 and X.
Figure 3 describes plasmid plPspAdapt Let-7 gene D formed by the insertion of the Xba I/Hind III fragment of Let- 7 gene D into the Avr II/Hind III sites of plPspAdapt6-deltaPolyA.

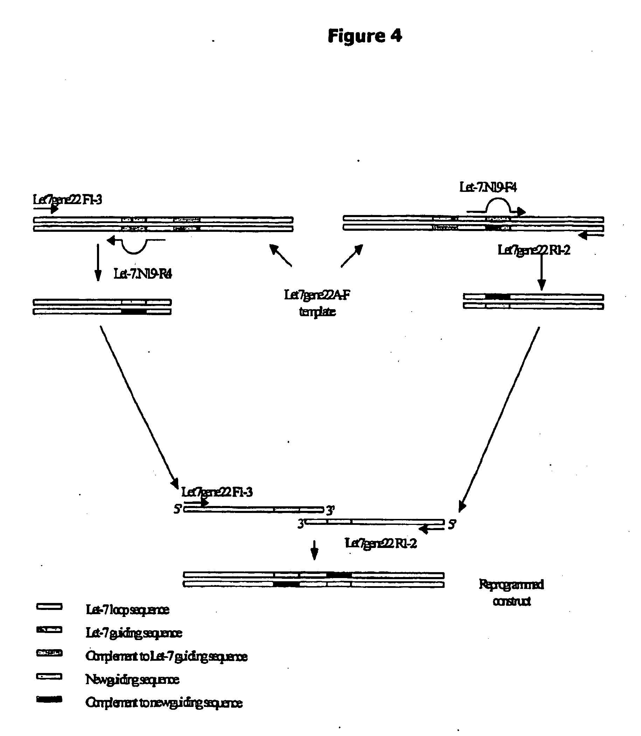

Figure 4 depicts the reprogramming of Let-7 RNA to another target sequence. Plasmid constructs containing Let7gene22A-F are used as templates for two separate PCR reactions. Primer Let-7.N19-. R4 is used in combination with a forward primer, for instance, Let7gene22 F1-3. Primer Let-7.N19-F4 is used in combination with a reverse primer, for instance, Let7gene22 R1-2. The products of these two separate PCR reactions are used as template for a final PCR reaction. Only the outside primers Let7gene22 F1-3 and Let7gene22 R1-2 are used in this final PCR reaction. The PCR products obtained from this final reaction are cloned into plPspAdapt6-deltaPolyA using the same strategy as described for the Let7gene22A-F fragments.
Figure 5 shows the process generating a library from randomly produced/isolated sRNAs.
Figure 6 shows the process for the preparation of constructs that code for individual chimera sRNA that may include RNA associated with unknown function and that is automatable and useful in the construction of a library of chimera sRNA.
Figure 7 is a schematic representation of the construction and use of an adenoviral chimeric sRNA library.
Figure 8 is a schematic representation of the luciferase-based reporter constructs and siRNA constructs used in example 2
Figure 9 depicts down-regulation of the reporters containing the target sequences that corresponds to the sequences of the co-transfected RNAs.
Figure 10 is a comparison of the wild-type let-7 loop, which is 30 nucleotides in length, and a shorter, 12-nucleotide loop based on the wild type let-7 loop. The bolded nucleotides represent those nucleotides deleted from the wild- type let-7 loop to generate the 12 nucleotide Loop 12 (L12).
Figure 11 depicts additional loop sequences tested for knock-down efficiency. These loops (L1, L2, L3, L4, L5, L6, L11, and L12) vary in size from 11 to 16 nucleotides.
Figure 12 shows the knock-down efficiencies of constructs comprising loop sequences varying in length from 11 to 30 nucleotides. These constructs are synthesized in the context of a GL2 target/guide. The knock-down efficiencies were measured in a transient transfection experiment as described in Example 3.
Figure 13A is a schematic illustration of human Let-7 genomic constructs.
Figure 13B is a schematic illustration of Let-7-based expression plasmids to replace the RNAs.
Figure 14 shows transient transfection of let-7 based expression plasmids on PER.C6/E2A cells, and a comparison of knock-down efficiency of different length loop sequences of let-7 based expression plasmids.
Figure 15 is a schematic representation of the reprogrammed let-7 chimeric clones and luciferase-based reporter constructs pGL3-control, or pGL3-tLet-7F, or pGL3-tLet-7R, or pGL2.
Figure 16 depicts luciferase reporter levels for the reprogrammed let-7 chimeric clones.
Figure 17 depicts northern blots showing the expression of the RNA species derived from the construct plPspAdapt- Let-7-gGL3 in PER.C6.E2A cells.
Figure 18 depicts the expression plasmids containing sequences other than let-7 or GL3 that also express chimeric RNA molecules with the correct length and expected sequence.
Figure 19 depicts how the Let-7 promoter is replaced by elements of the human U6 snRNA promoter. For efficient promoter activity the first nucleotide of the transcript is a G and the transcription termination signal is a string of 5 or more Ts. The expressed RNA contains a guide sequence of 19-21 nts (directed against a target) and a sequence able to base pair with the guide sequence connected by a loop sequence.
Figure 20 shows northern blots of samples of cells transfected with the U6-based promoter expression adenoviral vectors. The blots show expression of the RNA species and processing into a species of a size comparable to that of endogenous Let-7 RNA. Lane 1 contains uninfected cells, lane 2 contains cells infected with U6 (+1) L12 gLet7 lane 3 contains cells infected with U6 (+1) L13 gLet7.
Figure 21 is a comparison of knock-down efficiency of the reporter plasmids with the target Let-7 sequences with different promoters and different loops sequences. The loop L12 (SEQ ID NO: 30) is depicted in Figures 10 and 11. The loop L13 (SEQ ID NO: 66) is 14 nucleotides in length.
Figure 22 is a transfection experiment with adenoviral superinfection of Ad-EGFP. Infection of adenovirus had no effect on the knock-down activity obtained by the transiently transfected plasmids under the conditions used in this example.

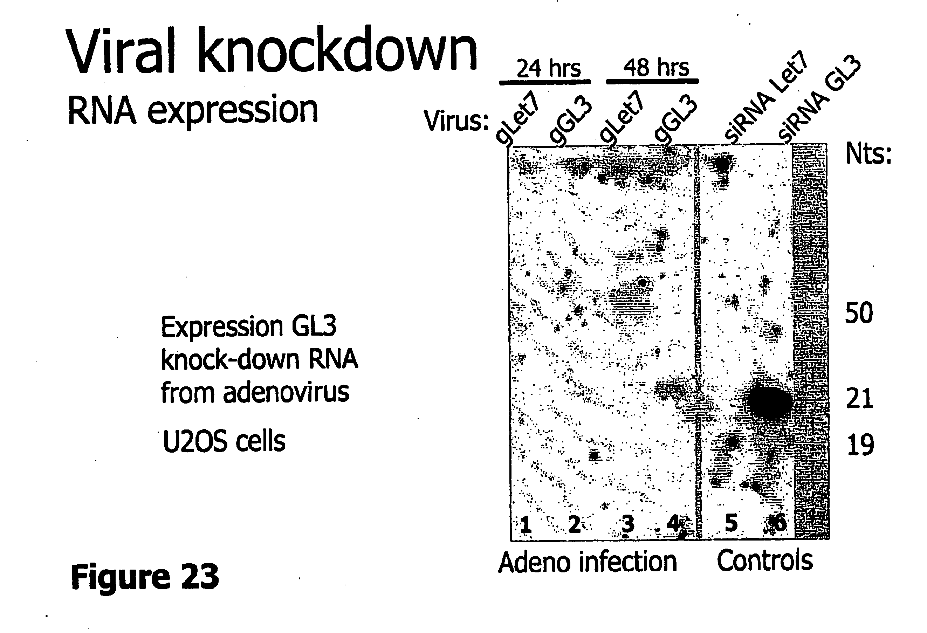

Figure 23 depicts northern blots of samples of cells infected with the adenoviral Let-7 based promoter expression constructs showing expression of the RNA species and processing into a species of a size comparable to that of synthetic siRNAs.
Figure 24 shows a knock-down efficiency comparison of two viral U6-promoter based expression constructs with different loop sequences, and the successful knock-down by viral expression constructs.
Figure 25A is a schematic representation of the cloning strategy for library construction showing utilization of SapI sites and an *E. coli*. death gene.
Figure 25B is a schematic representation of the cloning strategy for library construction. Adenoviral vector development for 56 nt inserts.
Figure 26 is a schematic representation of the cloning strategy for library construction. Adenoviral vector development for 51 nt inserts.
Figure 27 shows the successful knock-down of endogenous GNAS by adenoviral knock-down constructs as measured by real time PCR. The results show that the knock-down effect is dependent on MOI.
Figure 28 shows the specificity of the adenoviral knock-down constructs targeted against endogenous GNAS.
Figure 29 A-C show the successful knock-down of several endogenous mRNA by adenoviral knock-down constructs as measured by real time PCR. The knock-down effect is dependent on MOI and time.
Figure 30 shows the functional knock-down of GNAS. Adenoviral constructs encoding sRNA targeted against GNAS give a specific knock-down of GNAS on the functional level.

### Detailed Description

The following definitions are used in the description and examples to assist in understanding the scope of the present invention.

"Chimeric RNA" as used herein means an RNA molecule constructed from at least two polynucleotide sequences that covalently linked together and that derived from at least two different RNA molecules that may or may not be in the same or different species.

"Guide sequence" as used herein means a polynucleotide sequence that is complementary to a target sequence.

"Lethal protein" means proteins that may kill the cell in which the protein is produced, if produced in a lethal amount. Lethal proteins include proteins that induce apoptosis, such as Bax, Bcl-Xs, Bad and Bak, Fas, and Casp1, and proteins that inhibit viral replication, inhibit proliferation or inhibit protein synthesis both at the level of transcription or translation. Further specific examples of toxic proteins are full length Tiam, Rac, Rho, and Ras.

"siRNA" as used herein means a double stranded short interfering RNA molecule of no larger than about 23 nucleotides in length. The scientific literature describes siRNA as mediating the sequence specific degradation of a target mRNA.

"sRNA" as used herein means a single or double stranded RNA molecule of less than about 25 nucleotides. sRNA comprises both stRNA and siRNA molecules.

"stRNA" as used herein means a single stranded small temporal RNA molecule that is complementary to a 3' untranslated region in RNA in a host cell.

"Stem-loop" as used herein means a single stranded polynucleotide including two sequences of base pairs that complement each other and that permit the formation of a complementing duplex structure in the single-stranded polyribonucleotide, and a non-complementing loop sequence linking said two sequences of base pairs. The complementing base pairs making up the stem portion of the loop consist of at least two, and more preferably at least three base pairs in length. In certain special embodiments, where the stem base pair(s) correspond to the complementing sequence of the first and third sequences, one or more of the second sequence complementing stem base pairs can double as a first and third sequence complementing base pair. Under these special circumstances, the stem portion of the second sequence would be considered to contain only one complementing base pair, or no complementing base pairs.

"Target sequence" as used herein means a polyribonucleotide sequence present in mRNA in a host cell.

"Transfecting" as used herein means any way of introducing a nucleic acid into a cell as is known by a person skilled in the art. It includes but is not limited to transduction by e.g. calcium phosphate or liposomes based reagents, infection by e.g. viral vectors, phages, electroporation, via a soaking process, or introduction of the nucleic acid using a physical method like micro-injection or DNA coated particle bombardment.

The self-complementing single stranded polynucleotide included in a vector according to the present invention comprises a first guide sequence and a second sequence capable of forming a stem-loop structure within said second sequence when said second sequence is RNA, a third sequence, which complements the first guide sequence and is covalently linked to the distal end of the second sequence, wherein all nucleotides in said first and third sequences base pair. The preferred self-complementing polynucleotides comprise a second nucleotide sequence that comprises a stem-loop forming region derived from RNA molecules other than mRNA.

The present invention provides the first sequence to be a guide sequence that functions to direct the stRNA, siRNA, sRNA or chimeric RNA encoded by the single stranded polynucleotide of the vector to an mRNA having a complementary sequence in the host cell system. The first and third polynucleotide sequences have a length consisting of19 to 22 nucleotides, and most preferably about 19 or about 21 nucleotides, all of which correspond to a sequence found in a specific mRNA. The RNA in the host cell is mRNA. The mRNA in the host cell may be a known mRNA coding for a protein of known function, or may not be known to be associated with any particular protein or cellular function.

Preferred stem-loop sequences are based on stem-loop regions known to those persons skilled in the art to be present in RNA molecules such as, tRNA, snRNA, rRNA, mtRNA, or structural RNA sequences. Persons skilled in the art can readily identify stem-loop RNA structures using predictive computer modeling programs such as Mfold (M. Zuker, D.H. Mathews & D.H. Turner Algorithms and Thermodynamics for RNA Secondary Structure Prediction: A Practical Guide In RNA Biochemistry and Biotechnology, 11-43, J. Barciszewski & B.F.C. Clark, eds, NATO ASI Series, Kluwer Academic Publishers, (1999)), RNAstructure (Mathews, D.H.; Sabina, J.; Zuker, M.; and Turner, D.H., "expanded se- quence dependence of thermodynamic parameters improves prediction of RNA secondary structures", Journal of Molecular Biology, 1999, 288, 911-940), RNAfold in the Vienna RNA Package (Ivo Hofacker, Institut für theoretische Chemie, Währingerstr. 17,A-1090 Wien, Austria), Tinoco plot (Tinoco, I.Jr., Uhlenbeck, O.C. & Levine, M.D. (1971) Nature 230, 363-367), ConStruct, which seeks conserved secondary structures (Lück, R., Steger, G. & Riesner, D. (1996), Thermodynamic prediction of conserved secondary structure: Application to RRE-element of HIV, tRNA-like element of CMV, and mRNA of prion protein. J. Mol. Biol. 258, 813-826; and Lück, R., Gräf, S. & Steger, G. (1999), ConStruct: A tool for thermodynamic controlled prediction of conserved secondary structure. Nucleic Acids Res. 21, 4208-4217.), FOLDA- LIGN, (J. Gorodkin, L. J. Heyer and G. D. Stormo. Nucleic Acids Research, Vol. 25, no. 18 pp 3724-3732, 1997a; and J. Gorodkin, L. J. Heyer, and G. D. Stormo. ISMB 5; 120-123, 1997b), and RNAdraw (Ole Matzura and Anders Wennborg Computer Applications in the Biosciences (CABIOS), Vol. 12 no. 3 1996, 247-249).

RNA stem-loop structures are also found in databases such as the Small RNA Database (Karthika Perumal, Jian Gu, Yahua Chen and Ram Reddy Department of Pharmacology, Baylor College of Medicine, USA), Database of non-coding RNAs (Erdman VA, Barciszewska MZ, Szymanski M, Hochberg A. the non-coding RNAs as riboregulators (2001) Nucleic Acids Res. 29: 189-193), large subunit rRNA database (Wuyts J., De Rijk P., Van de Peer Y., Winkelmans T., De Wachter R. (2001) The European Large Subunit Ribosomal RNA database. Nucleic Acids Res. 29(1): 175-177), the small subunit rRNA database (Wuyts, J., Van de Peer, Y., Winkelmans, T., De Wachter R. (2002) The European database on small subunit ribosomal RNA. Nucleic Acids Res. 30, 183-185), snoRNA Database for budding yeast (Lowe and Eddy, Science 283: 1168-1171, 1999) for Archaea (Omer, Lowe, Russel, Ebhardt, Eddy and Dennis Science 288: 517-522, 2000), for *Arabidopsis thaliana:* (Brown, Clark, Leader, Simpson and Lowe RNA 7:1817-1832, 2001), tRNA sequences and sequences of tRNA genes (Mathias Sprinzl, Konstantin S. Vassilenko, http://www.uni-bayreuth.de/departments/biochemie/trna/), the 5S ribosomal RNA database (Szymanski M, Barcizewska MZ, Erdman VA, Barciszewski J, "5S ribosomal RNA database" (2002) Nucleic Acid Res. 30: 176-178), The Nucleic Acid Database Project (NDB) at Rutgers University (http://ndbserver.rutgers.edu/NDB/), The RNA Structure Database (www.RNABase.org).

Using these programs, one can identify an RNA stem-loop sequence, which can then be modified to eliminate multiple loop regions to result in a shorter, more easily synthesized stem-loop sequence. More preferred stem loop sequences are derived from the let-7 nucleotide sequence, or some portion thereof, or an artificially generated polynucleotide sequence based thereon.

Most preferred stem-loop regions consist essentially of a let-7 sequence found in the host cell or some portion thereof, some other naturally occurring RNA sequence or some portion thereof, or an artificial polynucleotide sequence capable of forming a loop structure when such polynucleotide is RNA. By eliminating the multiple loop segments of these sequences that are predicted by the aforesaid computer programs, stem loop sequences that are more easily synthesized and handled are prepared. Most preferred stem loop sequences are derived from the *let-7* nucleotide sequences.

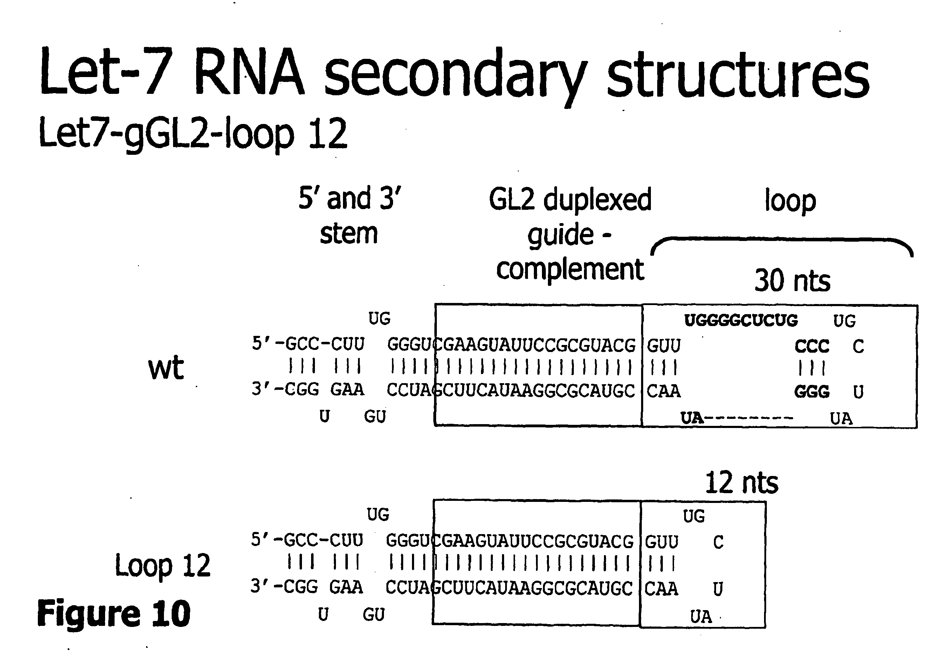

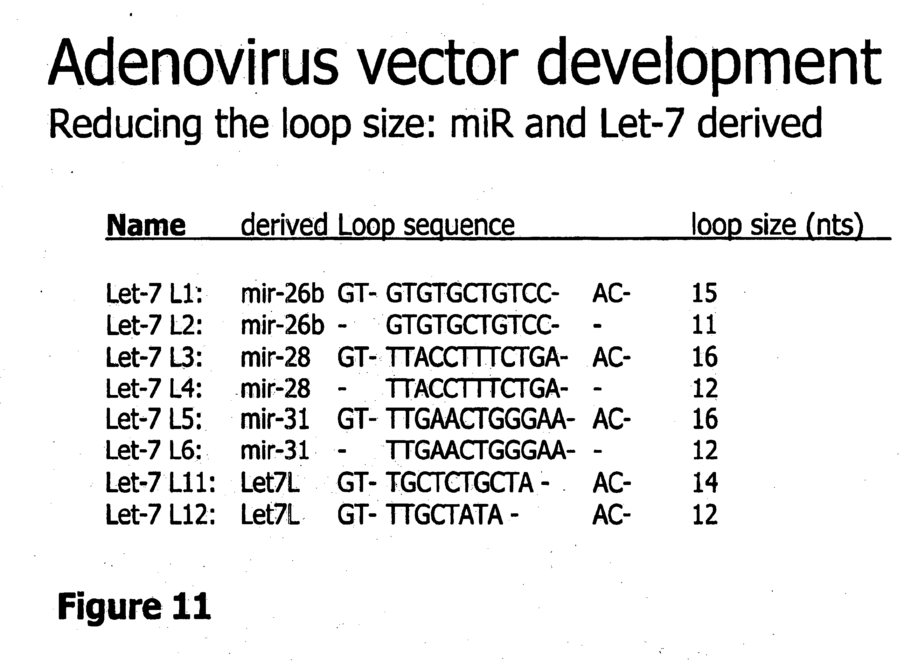

The loop structure is preferably about 4 to about 30 nucleotides in length, a more preferred length is about 4 to about 13 nucleotides, and a most preferred length is about 6 to about 12 nucleotides in length. A special embodiment of loop sequences comprise those artificial sequences based on known RNA loop sequences consisting of about 11 to about 16 nucleotides. Examples of preferred loop sequences are listed in Figures 10 and 11.

A particularly preferred polynucleotide of the present disclosure further comprises a fourth nucleotide sequence consisting essentially of a single stranded DNA equivalent of an RNA sequence, wherein said fourth sequence functions to permit the directional cloning thereof into a vector, and wherein said fourth sequence is covalently linked to a free end of either the first or third sequence, and wherein said RNA sequence is capable of being cleaved enzymatically in the host cell thereby resulting in the in situ preparation of a RNA polynucleotide that has first or third sequences with a free 3' and 5'-end.

Said fourth nucleotide sequence can be preferably derived from precursor RNAs, such as ribozymes, precursor tRNA, precursor rRNA, precursor microRNAs, RNAs recognized by ribozymes, or RNAs recognized by RNase P. Ribozymes cleave themselves such that a free 3'-or 5'-end at the said first or third nucleotide sequence is produced. Alternatively, ribozymes cleave the RNA sequences recognized by them, thereby producing free 3'-or 5'-end at the said first or third nucleotide sequence. Enzymes present in the host cell process precursor RNAs. Such fourth nucleotide sequences are preferably designed such that they are cleaved by enzymes present in the host cell.

The aforesaid fourth sequences may also be derived from "overhang" sequences found naturally, such as sequence that extend beyond the complementing portion of RNAs in the "microRNA" family. MicroRNAs (miRNAs) belong to an expanding class of non-coding RNAs of 21-24 nucleotides With let-7 RNA and lin-4 RNA as founding members. MicroRNA molecules are found in the genomes of a variety of species, including worms, flies, humans and plants, and are typically expressed from approximately 70 nts long hairpin-structured RNA precursors. These hairpin-structured precursors can also exist in a cluster of several precursors. Normally, after processing, only one strand of the duplexed region of the precursor accumulates in the cell as 21-24 nucleotides RNA. Examples of identified miRNAs are described in Lagos-Quintana, M et al. Science (2001) 294: 853, Lau, et al. Science (2001) 294: 858, Lee and Ambros Science (2001) 294: 862.

A most preferred dsDNA polynucleotide comprises a fourth sequence that functions to permit the directional cloning thereof into a DNA vector according to the invention, as described in more detail below. The dsDNA polynucleotide sequences may contain restriction sites at either end that are susceptible for cleavage by one restriction enzyme or two different restriction enzymes to enable efficient cloning. The resulting termini of the dsDNA oligonucleotides preferably have overhanging nucleotide sequences (either 5' or 3' overhanging) that match the vector insertion sites. The dsDNA polynucleotide containing the cleavable restriction sites at the termini can be generated by standard molecular biological techniques, for example, by annealing two complementary ssDNA oligonucleotides. Alternatively, two annealed DNA oligonucleotides, with only restriction sites at their 5' termini, can be enzymatically extended at their complementary 3' termini to make the fully complementary double stranded DNA. Furthermore, the person skilled in the art is able to utilize blunt end cloning techniques and PCR to develop alternative routes of synthesis to achieve the directional cloning described herein.

The aforesaid fourth and fifth sequences may or may not be transcribed from the DNA sequence present in a DNA vector of the present invention, but may function as part of the upstream promoter or downstream termination signal. Consequently the fourth sequence may incorporate the start signal for RNA polymerase to transcribe, and the fifth sequence comprise a stop signal, such as a multiple "T" sequence, that transcribes into an RNA fifth sequence of multiple "U" nucleotides. Upon transcription the fourth sequence may not be transcribed into RNA except for one to about five, and more preferably one to about three "G" nucleotides.

In a particular aspect of the present disclosure, the process of preparing the self-complementing polynucleotide uses an intermediate polynucleotide consisting essentially of a first sequence consisting of about 19 to about 22 nucleotides, said first sequence covalently linked to a second sequence capable of forming a stem-loop structure, when said second sequence is an RNA sequence, wherein said first sequence consists essentially of a RNA sequence, a single stranded DNA equivalent thereof, or a RNA or DNA sequence complementary to said RNA sequence. mRNA sequences, that are sequences that code for protein, are a special embodiment of the methods and compositions according to the present disclosure.

The self-complementing single-stranded polynucleotides may be prepared by chemically synthesis. The process of synthesis requires that the target sequence of the RNA be known, a 19 to 22 nt sequence corresponding thereto prepared, and the synthesis continued to add the stem-loop sequence of about 4 to about 30 nucleotides, more preferably, from 6 to about 13 nucleotides. The isolated synthetic polynucleotide may be used to prepare a vector for use as an intermediate in the practice of the present invention, or further lengthened to include the complementing third sequence.

In practice, from about two to about five sequences are chosen from a single RNA sequence for the preparation of a corresponding number of vectors containing the self-complementing polynucleotides. The present method, described in more detail below, uses this "redundant" set of vectors containing the self-complementing single stranded polynucleotides, to determine the optimum choice of RNA sequence that targets only one unique RNA that may exist among a family of RNA having homologous sequence regions. Alternatively, one sequence targeted against multiple RNA targets can be designed when knock-down of more than one RNA target, e.g. RNAs belonging to a family, is desired.

Another method of preparing the self-complementing polynucleotide including said third sequence involves treating a single stranded polynucleotide consisting essentially of a first polynucleotide sequence covalently linked to a second polynucleotide sequence that includes two nucleotide sequences capable of complementary base pairing and thereby forming a stem-loop structure and that has a 3' OH terminus, under conditions such that said first sequence serves as a template starting at the 3' OH terminus for the synthesis of a complementary sequence thereto.

The polynucleotide produced as a result of the extension reaction using the template or by chemical synthesis comprises a first nucleotide sequence and a third nucleotide sequence covalently linked by a second nucleotide sequence capable of forming a stem-loop structure such that all nucleotides in said first sequence and said third sequences are capable of base pairing with each other. The third sequence complementary to said first sequence is covalently linked to the distal end of said second sequence.

In a special embodiment of the intermediate polynucleotide of vectors of the present invention, said second nucleotide sequence contains at least one nucleotide sequence capable of being cleaved enzymatically. A more preferred embodiment comprises a second sequence have at least two enzymatic cleavage sites. A particularly preferred intermediate polynucleotide comprises a second polynucleotide sequence encoding the stem portion of said stem-loop structure including at least one of said enzymatic cleavage sites.

Another special embodiment comprises a polynucleotide intermediate wherein at least one enzymatic cleavage site is at the 5' and/or 3' ends of said second sequence. Enzymatic cleavage sites consist of nucleotide sequences containing at least four to about eight base pairs and are known to persons skilled in the art. Such sequences may add from about two to about twenty additional nucleotides to the length of the second sequence and be substituted for the complementing nucleotides defining the 5' and 3' ends of the loop sequences. Such elongated sequences may consist of from about twelve to about 50 nucleotides. Preferred elongated artificial loop sequences consist of from about ten to about 36 nucleotides.

The present invention relates to vector constructs comprising the self-complementing polynucleotide and a pol III promoter sequence positioned upstream of the first sequence of said polynucleotide. The self-complementing DNA polynucleotide sequence of the present invention may be inserted preferably into a plasmid DNA vector, an adenovirus DNA viral vector, an adeno-associated virus vector, or a herpes vector. The DNA plasmid vector may be delivered alone or complexed with various vehicles. The DNA, DNA/vehicle complexes, or the recombinant virus particles are locally administered to the site of treatment, as discussed below. Preferably, recombinant vectors capable of expressing the present polynucleotides are locally delivered as described below, and persist in target cells. Once expressed, the self-complementing RNA molecule is processed and is guided to the endogenous target RNA, where it functions to degrade the target RNA.

Promoter sequences are the pol III promoters. The pol III promoters include those promoters selected from the group consisting of 5S rRNA, tRNAs, VA RNAs, Alu RNAs, H1, and U6 small nuclear RNA promoters.

In the vector construction, the polynucleotides of the present invention may be linked to one or more regulatory regions in addition to the promoter. Selection of the appropriate regulatory region or regions is a routine matter, within the level of ordinary skill in the art. Regulatory regions other than promoters include enhancers, suppressors, etc.

In addition to recombinant adenovirus (dsDNA), systems, other viral packaging systems such as ssDNA viruses, for example, adenovirus-associated virus (AAV), are suitable as for use as vector backbone in the present invention. Furthermore, other dsDNA viruses, such as for example, Epstein Barr virus, herpes simplex virus, baculovirus or vaccinia viruses are useful as vector backbone constructs in the present invention. Each of these systems has a different host range..

For expression in AAV, the polynucleotide is cloned into an AAV expression vector. To produce recombinant AAV particles, 293 cells are infected with adenovirus type 5; 4 hours later the infected cells are co-transfected with the AAV expression plasmid-oligonucleotide DNA construct and an AAV helper plasmid, pAAV/Ad (Samulski et al., (1989) J. Virol. 63:3822-3828). As recombinant AAV is produced, the 293 cells undergo cytopathology, becoming spherical and lose their ability to adhere to a tissue culture surface. Following development of maximal cytopathology the supernatant is harvested and, if necessary, concentrated (Halbert et al. 1997. J. Virol. 71:5932-5941).

For vaccinia virus expression, a replication competent vaccinia virus can be used. The polynucleotide is operatively linked to a vaccinia virus promoter, for example, P 11. Preferably, vaccinia virus strain MVA is used because it expresses recombinant genes but contains a deletion that renders it replication incompetent in many mammalian cells. Therefore, the polynucleotide can be expressed in target host mammalian cells without the development of vaccinia virus induced cytopathology. The recombinant vaccinia virus is produced by infecting chicken embryo fibroblasts (CEF) with vaccinia and co-transfecting a transfer vector into which has been ligated the polynucleotide of the invention and a marker gene (beta galactosidase) functionally linked to a vaccinia promoter, such as P11, and flanked by genomic sequences. The construct is inserted into the vaccinia genome by homologous recombination. Recombinant viruses can be identified by in situ staining for beta-galactosidase expression with X-gal (Wyatt et al. (1995) Virology 210:202-205).

Additional vector systems include the non-viral systems that facilitate introduction of DNA encoding the self- complementing single-stranded RNA, or the RNA itself into a patient. For example, a DNA vector encoding a desired sequence can be introduced in vivo by lipofection. Synthetic cationic lipids designed to limit the difficulties encountered with liposome mediated transfection can be used to prepare liposomes for in vivo transfection of a gene encoding a marker (Felgner, et. al. (1987) Proc. Natl. Acad. Sci. USA 84:7413-7); see Mackey, et al. (1988) Proc. Natl. Acad. Sci. USA 85:8027-31; Ulmer, et al. (1993) Science 259:1745-8). The use of cationic lipids may promote encapsulation of negatively charged nucleic acids, and also promote fusion with negatively charged cell membranes (Felgner and Ringold, (1989) Nature 337:387-8). Particularly useful lipid compounds and compositions for transfer of nucleic acids are described in International Patent Publications WO 95/18863 and WO 96/17823, and in U.S. Patent No. 5,459,127. The use of lipofection to introduce exogenous genes into the specific organs in vivo has certain practical advantages and directing transfection to particular cell types would be particularly advantageous in a tissue with cellular heterogeneity, for example, pancreas, liver, kidney, and the brain. Lipids may be chemically coupled to other molecules for the purpose of targeting. Targeted peptides, e.g., hormones or neurotransmitters, and proteins for example, antibodies, or non-peptide molecules could be coupled to liposomes chemically. Other molecules are also useful for facilitating transfection of a nucleic acid in vivo, for example, a cationic oligopeptide (e.g., International Patent Publication WO 95/21931), peptides derived from DNA binding proteins (e.g., International Patent Publication WO 96/25508), or a cationic polymer (e.g., International Patent Publication WO 95/21931).

The more preferred viral vectors useful in the practice of the present invention are the E1-deleted adenoviral vectors, with the E1, E2A deleted vectors being most preferred. The more preferred adenoviral vectors include the E1- deleted adenoviral serotype 5 vectors, with the E1, E2A deleted vectors being most preferred. Vectors may also be prepared from other adenoviral serotypes and corresponding packaging cells that include sequences for viral proteins deleted from such vector backbones. The most preferred adenoviral vector/packaging cell combinations are those combinations where the packaging cell and vector do not include any overlapping adenoviral sequences, which overlap would provide the statistical possibility of the production of replication competent adenoviral particles. Preferred packaging cells useful in the production of such vectors include the 293 and 911 cells, with the most preferred cells being the PER.C6 cell line. The modified PER.C6/E2A cell line is a special embodiment complementing the E1, E2A deleted adenoviral vector constructs, with non-overlapping adenoviral E1, E2A sequences, and is most preferred in the practice of the present invention.

The vectors of the present invention as described herein are also useful in methods of lowering the amounts of RNA or protein translated from RNA in a host cell, or subject, comprising transfecting said cell or subject with a vector according to the invention. Preferred vectors according to the present invention comprise the aforesaid first nucleotide sequence and a third nucleotide sequence covalently linked by a second nucleotide sequence capable of forming a stem-loop structure such that all nucleotides in said first sequence and said third sequences are capable of base pairing with each other, and wherein said second nucleotide sequence comprises a stem-loop forming region derived from naturally occurring RNA sequences found in RNA molecules other than mRNA, such as for example, tRNA, snRNA, rRNA, mtRNA, or structural RNA sequences. Preferred stem loop sequences are derived from the let-7 nucleotide sequence, or some portion thereof, or an artificially generated polynucleotide sequence based thereon. The administration of the aforesaid vector to a subject comprises the administration of an amount of vector effective to lower the amounts of said RNA in said transfected cells of said subject.

The preferred vector of the present invention including a polynucleotide comprising a promoter operably linked to a sequence of a self-complementing polynucleotide, may be prepared by denaturing the self-complementing polynucleotide, converting the resulting denatured polynucleotide into a double stranded polynucleotide, and ligating the double stranded polynucleotide into a vector capable of transfecting a host cell and transcribing said polynucleotide. Alternatively the self-complementing polynucleotide may be chemically synthesized as two single stranded polynucleotides, which are capable of being annealed to each other followed by ligation into the vector. The ligation may be accomplished preferably into an adapter plasmid that may be used to form a transfectable viral vector particle by co-transfection with a helper molecule in a packaging cell line.

A further embodiment of the vector construct encoding the self-complementing polynucleotide is wherein said second nucleotide sequence contains at least one nucleotide sequence capable of being cleaved enzymatically. A more preferred embodiment comprises a second sequence have at least two enzymatic cleavage sites. In this embodiment, said second sequence, which can be any length, can be removed by enzymatic cleavage and replaced by a stem-loop sequence. In a further embodiment the second nucleotide sequence encodes for a gene useful for the facilitation of cloning stem-loop sequences into the vector. In a further special embodiment the gene useful for the facilitation of cloning stem-loop sequences into the vector is the E. coli ccdB death gene.

The present vectors may be administered to a patient by a variety of methods. They may be added directly to target tissues, complexed with cationic lipids, packaged within liposomes, or delivered to target cells by other methods known in the art. Localized administration to the desired tissues may be done by catheter, infusion pump or stent, with or without incorporation of the self-complementing polynucleotide in biopolymers. Alternative routes of delivery include, but are not limited to, intravenous injection, intramuscular injection, subcutaneous injection, aerosol inhalation, oral (tablet or pill form), topical, systemic, ocular, intraperitoneal and/or intrathecal delivery.

Preferably, the viral vectors used in the gene therapy methods of the present disclosure are replication defective. Such replication defective vectors will usually lack at least one region that is necessary for the replication of the virus in the infected cell. These regions can either be eliminated (in whole or in part), or be rendered non-functional by any technique known to a person skilled in the art. These techniques include the total removal, substitution, partial deletion or addition of one or more bases to an essential (for replication) region. Such techniques may be performed in vitro (on the isolated DNA) or *in situ*, using the techniques of genetic manipulation or by treatment with mutagenic agents. Preferably, the replication defective virus retains the sequences of its genome that are necessary for encapsidating the viral particles.

In other embodiments of the present invention, adeno-associated viruses ("AAV") are utilized. The AAV viruses are DNA viruses of relatively small size that integrate, in a stable and site-specific manner, into the genome of the infected cells. They are able to infect a wide spectrum of cells without inducing any effects on cellular growth, morphology or differentiation, and they do not appear to be involved in human pathologies.

It is also possible to introduce a DNA vector in vivo as a naked DNA plasmid (see U.S. Patents 5,693,622, 5,589,466 and 5,580,859). Naked DNA vectors for gene therapy can be introduced into the desired host cells by methods known in the art, e.g., transfection, electroporation, microinjection, transduction, cell fusion, DEAE dextran, calcium phosphate precipitation, use of a gene gun, or use of a DNA vector transporter (see, e.g., Wilson, et al. (1992) J. Biol. Chem. 267:963-7; Wu and Wu, (1988) J. Biol. Chem. 263:14621-4; Hartmut, et al. Canadian Patent Application No. 2,012,311, filed March 15, 1990; Williams, et al (1991). Proc. Natl. Acad. Sci. USA 88:2726-30). Receptor-mediated DNA delivery approaches can also be used (Curiel, et al. (1992) Hum. Gene Ther. 3:147-54; Wu and Wu, (1987) J. Biol. Chem. 262:4429-32).

The present invention, in a particular embodiment, relates to a composition comprising a vector of the invention including self-complementing polynucleotide that is used to down-regulate or block the expression of specific polypeptides or specific non-coding RNA molecules. The nucleic acid encodes a self-complementing sRNA molecule covalently linked by a stem-loop RNA sequence. In this embodiment, the nucleic acid is operably linked to signals enabling expression of the nucleic acid sequence and is introduced into a cell utilizing recombinant vector constructs, which will express the polynucleic acid once the vector is introduced into the cell. Examples of suitable vectors include plasmids, adenoviruses, adeno-associated viruses, retroviruses, and herpes viruses.

The present invention provides biologically compatible compositions comprising the vectors of the present invention. A biologically compatible composition is a composition, that may be solid, liquid, gel, or other form, in which the vector of the invention is maintained in an active form, e.g., in a form able to effect a biological activity. For example, a vector would be able to transfect a target cell. A preferred biologically compatible composition is an aqueous solution that is buffered using, e.g., Tris, phosphate, or HEPES buffer, containing salt ions. Usually the concentration of salt ions will be similar to physiological levels. Biologically compatible solutions may include stabilizing agents and preservatives. In a more preferred embodiment, the biocompatible composition is a pharmaceutically acceptable composition.

Such compositions can be formulated for administration by topical, oral, parenteral, intranasal, subcutaneous, and intraocular, routes. Parenteral administration is meant to include intravenous injection, intramuscular injection, and intraarterial injection or infusion techniques. The composition may be administered parenterally in dosage unit formulations containing standard, well-known nontoxic physiologically acceptable carriers, adjuvants, and vehicles as desired.

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient. Pharmaceutical compositions for oral use can be prepared by combining active com- pounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethylcellulose, or sodium carboxymethyl-cellulose; gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate. Dragee cores may be used in conjunction with suitable coatings, such as concentrated sugar solutions, which may also contain gum arabic, talc, polyvinyl-pyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations that can be used orally include push-fit capsules made of gelatin, as well as soft, sealed capsules made of gelatin and a coating, such as glycerol or sorbitol. Push-fit capsules can contain active ingredients mixed with filler or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Preferred sterile injectable preparations can be a solution or suspension in a nontoxic parenterally acceptable solvent or diluent. Examples of pharmaceutically acceptable carriers are saline, buffered saline, isotonic saline (e.g. monosodium or disodium phosphate, sodium, potassium, calcium or magnesium chloride, or mixtures of such salts), Ringer's solution, dextrose, water, sterile water, glycerol, ethanol, and combinations thereof. 1,3-butanediol and sterile fixed oils are conveniently employed as solvents or suspending media. Any bland fixed oil can be employed including synthetic mono- or di-glycerides. Fatty acids such as oleic acid also find use in the preparation of injectables.

The composition medium can also be a hydrogel, which is prepared from any biocompatible or non-cytotoxic homo- or hetero-polymer, such as a hydrophilic polyacrylic acid polymer that can act as a drug absorbing sponge. Certain of them, such as, in particular, those obtained from ethylene and/or propylene oxide are commercially available. A hydrogel can be deposited directly onto the surface of the tissue to be treated, for example during surgical intervention.

Preferred pharmaceutical compositions of the present invention comprise a replication defective recombinant viral vector and the polynucleotide identified by the present invention. A special embodiment of the composition invention includes also a transfection enhancer, such as poloxamer. An example of a poloxamer is Poloxamer 407, which is commercially available (BASF, Parsippany, NJ) and is a non-toxic, biocompatible polyol. A poloxamer impregnated with recombinant viruses may be deposited directly on the surface of the tissue to be treated, for example during a surgical intervention. Poloxamer possesses essentially the same advantages as hydrogel while having a lower viscosity.

The formulation herein may also contain more than one active compound as necessary for the particular indication being treated, preferably those with complementary activities that do not adversely affect each other. Alter- natively, or in addition, the composition may comprise a cytotoxic agent, cytokine or growth inhibitory agent. Such molecules are suitably present in combination in amounts that are effective for the purpose intended. The formulations to be used for in vivo administration must be sterile. This is readily accomplished by filtration through sterile filtration membranes.

The active ingredients of the present invention may also be entrapped in microcapsules prepared, for example, by interfacial polymerization, for example, hydroxymethylcellulose or gelatin-microcapsules and poly-(methylmeth- acylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences (1980) 16th edition, Osol, A. Ed.

Sustained-release preparations may be prepared. Suitable examples of sustained-release preparations include semipermeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Patent No. 3,773,919), copolymers of L- glutamic acid and. gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

The present disclosure provides methods of treatment that comprise the administration to a human or other animal of an effective amount of a composition of the invention. A therapeutically effective dose refers to that amount of the present polynucleotide that ameliorates the symptoms or condition. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical compositions that exhibit large therapeutic indices are preferred. The data obtained from cell culture assays and animal studies is used in formulating a range of dosage for human use. The dosage of such compounds lies preferably within a range of circulating concentrations that include the ED50 with little or no toxicity. The dosage varies within this range depending upon the dosage form employed, sensitivity of the patient, and the route of administration.

For any polynucleotide of the present disclosure, the therapeutically effective dose can be estimated initially either in cell culture assays or in animal models, usually mice, rabbits, dogs, or pigs. The animal model is also used to achieve a desirable concentration range and route of administration. Such information can then be used to determine useful doses and routes for administration in humans. The exact dosage is chosen by the individual physician in view of the patient to be treated. Dosage and administration are adjusted to provide sufficient levels of the active moiety or to maintain the desired effect. Additional factors which may be taken into account include the severity of the disease state, age, weight and gender of the patient; diet, desired duration of treatment, method of administration, time and frequency of administration, drug combination(s), reaction sensitivities, and tolerance/response to therapy. Long acting pharmaceutical compositions might be administered every 3 to 4 days, every week, or once every two weeks depending on half-life and clearance rate of the particular formulation.

As discussed hereinabove, recombinant viruses may be used to introduce DNA encoding the self-complementing single stranded polynucleotides, as well as the self-complementing single stranded RNA. Recombinant viruses according to the invention are generally formulated and administered in the form of doses of between about 10⁴ and about 10¹⁴ pfu. In the case of AAVs and adenoviruses, doses of from about 10⁶ to about 10¹¹ pfu are preferably used. The term pfu ("plaque-forming unit") corresponds to the infective power of a suspension of virions and is determined by infecting an appropriate cell culture and measuring the number of plaques formed. The techniques for determining the pfu titre of a viral solution are well documented in the prior art.

Self-complementing polynucleotides according to the present disclosure may be administered in a pharmaceutically acceptable carrier. Dosage levels may be adjusted based on the measured therapeutic efficacy.

In another aspect of the present invention, the polynucleotide vector is transferred into the target tissue using one of the vector delivery systems herein. This transfer is carried out either ex vivo in a procedure in which the nucleic acid is transferred to cells in the laboratory and the modified cells are then administered to the human or other animal, or in vivo in a procedure in which the nucleic acid is transferred directly to cells within the human or other animal. In preferred embodiments, an adenoviral vector system is used to deliver the expression vector. If desired, a tissue specific promoter is utilized in the expression vector as described above.

Non-viral vectors may be transferred into cells using any of the methods known in the art, including calcium phosphate co-precipitation, lipofection (synthetic anionic and cationic liposomes), receptor-mediated gene delivery, naked DNA injection, electroporation and bio-ballistic or particle acceleration.

The present invention may be used in *in vitro* validation of drug targets, screening for novel drug targets by knocking down genes in cellular assays, and in animal studies for *in vivo* target validation development of therapeutics.

The subject invention relates also to methods and compositions for the high throughput delivery and expression in a host of guide nucleic acid(s) targeting RNA of known or unknown function. Methods are described for infecting a host with the adenoviral vectors that express the self-complementing RNA molecules including the guide nucleic acid(s) in the host, identifying an altered phenotype induced in the host by the knockdown of the target RNA nucleic acids, and thereby assigning a function to the product(s) encoded by the target nucleic acids. The methods can be fully automated and performed in a multiwell format to allow for convenient high throughput analysis of sample nucleic acid libraries, which samples code for the guide sequences for use in this method.

The present invention may be used to prepare libraries of vectors according to the invention. These libraries may be prepared as single element, compartmentalized, or discrete elements, wherein each element consists essentially of a vector coding for a unique nucleotide sequence. Alternatively, a library comprising pools of vectors may be prepared.

The libraries may be used to assist in the elucidation of the functions of host cell RNA molecules including the unique polynucleotide residing in each compartment of said library, or in other words, determining the function of a naturally occurring polynucleotide sequence comprising transfecting a host cell with a vector according to the invention, said vector including a polynucleotide sequence complementary to a portion of said naturally occurring polynucleotide and detecting a change in cellular phenotype. Each vector in the library may be introduced into one or more cells and changes in protein expression, or phenotype observed. The vectors may comprise plasmids or be included in a viral vector construct. Alternatively, more than one vector thereby introducing more than one guide sequence can be introduced into a single host cell. Preferred viral vectors include the adenoviral, retroviral and AAV-vectors. More preferred are the adenoviral vectors, and most preferred are the adenoviral vectors that comprise a replication deficient construct that may be multiplied in a packaging cell having complementary sequences to the sequence contained in the vector itself.

The present invention provides for the temporary knock-down of proteins, such as lethal proteins, during virus production, thereby allowing the replication and packaging of virus that include sequences encoding for lethal proteins. sRNA can be used to knock-down gene expression during viral production with any virus and in any viral packaging cell line. Accordingly, the present disclosure relates to a method of producing viral vectors encoding a toxic protein comprising
(a) introducing into a cell a polynucleotide sequence as described herein having a first sequence that is complementary to the mRNA coding for said toxic protein,
(b) introducing said viral vector into said cell,
(c) culturing said cells under conditions allowing expression of said polynucleotide sequence and replication of said viral vector, and
(d) recovering said viral vectors.

A preferred method of the present disclosure uses viral packaging cells that are stably transfected with said polynucleotide.

Viral production using adenovirus retrovirus or alphavirus benefit from such knock down methods, and examples of packaging cells include adenoviral packaging cells, such as PER.C6 cells, and derivatives thereof, HEK293 cells, 293 and 911 cells, among others. Furthermore, sRNA knock-down methodology is useful for improving recombinant protein production. Such protein production methods benefit from the down-modulation of heterologous protein expression prior to achieving the optimal production cell titre for protein production.

The present invention may be applied to every viral packaging and protein production system without a need for optimization. Knock-down constructs described herein may be transfected into any selected packaging cell and such transfected cells are used directly. The present invention uses virus constructs that are used directly to infect cells and no further compound is required by the system to induce virus or protein production.

The sequences between transcription start and the 5' end of the expression cassette that is used to express exogenous genes can be used to knock down the expression of those exogenous genes during virus production. Poly- nucleotides of the present invention, which polynucleotides includes guiding sequences targeted against the sequence between transcription start and the 5' end of the expression cassette, are co-transfected with the viral plasmid(s) carrying the sequence for the toxic protein, into the packaging cells. Alternatively, a vector according to the invention, encoding polynucleotides that down-modulate expression of target sequences by break down of the mRNA, are used. The vector can be used transiently or used to generate a stable derivative of the packaging cell line.

The various aspects of the present invention are further described in the following non-limiting examples.

### EXAMPLES

### Example 1: A reporter assay system based on let-7 target sequence to monitor repression

Example 1 describes development of a reporter assay system that provides a method for measuring knockdown of a readily assayed gene. This system is used to determine if siRNAs and chimeric RNAs can decrease expression of the readily assayed luciferase gene. The system consists of two components. The first component is a reporter DNA molecule based on the pGL3 luciferase reporter vector (available from Promega), which has been modified to include a let-7 target sequence derived from the human let-7 sequence found on chromosome 22. These reporter constructs are designated as follows: The names start with a 'p' indicating that the construct is in a plasmid, then the name of the reporter gene follows (e.g. GL3 or GL2), after that the target sequence is mentioned starting with a 't' to indicate that it is the target sequence. For example: pGL3-tLet7 describes a plasmid containing the GL3 gene as reporter and Let7 sequences as target for the knockdown RNA. The second component is a siRNA or a plasmid expressing siRNA or chimeric RNAs. siRNAs are double stranded short interfering RNA molecules no larger than about 23 nucleotides in length. Chimeric RNAs as used herein refer to an RNA molecule constructed from at least two polynucleotide sequences that are covalently linked together and derived from at least two different. RNA molecules that may be from the same or different species. The scientific literature describes siRNA as mediating the sequence specific degradation of a target mRNA. In the present application these siRNAs are designated as follows: siRNA followed by the name of the target gene, e.g. siRNA GL3.1 is a duplex siRNA targeted against the GL3 gene. In this example a reporter construct, a siRNA, and an internal control used to normalize luciferase activity (Renilla: pRL-TK) are combined together and used to transfect host cells and the luciferase activity measured. If the siRNA knocks down expression of luciferase mRNA, a reduction of luciferase activity is seen relative to controls. The reporter system is described below. The description is meant only as an example and is in no way limiting to the invention.

The reporter system is based on the pGL3 luciferase reporter vector (Promega). The let-7 target DNA sequence (5'-ACTATACAACCTACTACCTCA-3' SEQ ID NO: 1) is introduced just outside of the GL3 coding region, in a pGL3-reporter vector, such that it expresses a GL3 mRNA that contains the let-7 target sequence.

### A. Construction pGL3-tLet-7 reporter constructs,

The pGL3-control vector (GenBank Accession Number U47296) is linearized at its unique Xba I site, which is immediately 3' of the GL3 coding sequence. The double stranded let-7 target DNA sequence is generated using complementary DNA oligonucleotides (Oligo 1 and Oligo 2). In order to facilitate closing into the Xba I site of pGL3, Oligo 1 and Oligo 2 are designed such that upon annealing, the double stranded let-7 target DNA thus generated has 5' overhangs on each end that are compatible with an Xba I restriction site. Annealing of Oligo 1 and Oligo 2 is accomplished by mixing the oligos in equimolar amounts to a final concentration of 0.5 nmole/D1 each in annealing buffer [10 mM Tris-HCl (pH 7.9), 10 mM MgCl₂, 50 mM NaCl], followed by incubation of the mixture for 1 minute at 90°C and 60 minutes at 37°C. The annealed oligos are then ligated into the Xba I site of the linearized pGL3 vector using common techniques as described in Sambrook et al.

The Let-7 target DNA sequence is SEQ ID NO: 1:
5'-ACTATACAACCTACTACCTCA-3'
Sequences DNA Oligos (5' to 3'):

| | |
|---|---|
| OLIGO 1 | 5'-CTAGTACTATACAACCTACTACCTACTACCTCA-3' (SEQ ID NO: 2) |
| OLIGO 2 | 5' -CTAGTGAGGTAGTAGGTTGTATAGTA-3' (SEQ ID NO: 3) |

The annealed Oligos 1 and 2 give the following double stranded structure:

Underlined nucleotides denote the Xba I compatible ends. The cloning of the oligos into the vector results in an extra Sca I site (bolded nucleotides), which facilitates the selection of clones with the insert and enables the discrimination between clones with an insert in the forward (F) orientation or clones with an insert in the reverse (R) orientation. The original Xba I site from the starting pGL3-control vector is destroyed by the cloning process and thus is absent in the clones with inserts. The clones are tested for presence of the insert by performing PCR on the transformed bacteria directly, using the following primers:
OLIGO 3 5' -CATCTTCGACGCAGGTGTCGCA-3' (SEQ ID NO: 4) (position 1668-1689 according to Promega catalog and U47296 sequence)
OLIGO 4 5'-CCATCGTTCAGATCCTTATCGA-3' (SEQ ID NO: 5) (position 2210-2189 according to Promega catalog and U47296 sequence)

The given positions are based on the pGL3-control vector (Accession number U47296).

using Oligo 3 (SEQ ID NO: 4) and Oligo 4 (SEQ ID NO: 5) as primers and colony DNA as template, PCR products generated from clones without an insert are 543 base pairs; with an insert in either forward (F) or reverse (R) orientation PCR products are 569 base pairs.

The orientation of the insert is analyzed further by a second round of PCR using two primer combinations;
1) Oligo 1 (SEQ ID NO: 2) and Oligo 3 ( SEQ ID NO: 4) : PCR with let-7 target sequence in R orientation produces a DNA fragment of 297 bp; F orientation will produce no DNA product.
2) Oligo 1 (SEQ ID NO: 2) and Oligo 4 ( SEQ ID NO: 5) ; PCR with let-7 target sequence in F orientation gives a DNA fragment of 302 bp; R orientation will produce no DNA product.

The plasmid generated by successful cloning of let-7 target DNA sequences in the forward orientation into the Xba I site of pGL3 will be known as pGL3-tlet-7F. Similarly, the plasmid generated by successful cloning of let-7 target DNA sequences in the reverse orientation into the Xba I site of pGL3 will be known as pGL3-tlet-7R. Both clones pGL3-tlet-7F and pGL3-tlet-7R are used in further experiments.

### B. siRNAs

The siRNAs that target pGL3 and pGL2 (used as a negative control; Accession Number X65324) are as described in Elbashir et al. (2001) Nature 411:494-498. siRNAs are double stranded RNAs that include the target sequence and its complement. Two uridine residues are added to the 3' end of the RNAs.

### siRNA-GL2.1:

GL2 Target DNA Sequence
5'..CGTACGCGGAATACTTCGA..3' (SEQ ID NO: 6)

### siRNA-GL2.1-

-antisense 3'-UUGCAUGCGCCUUAUGAAGCU-5' (SEQ ID NO: 8)

### siRNA-GL3.1:

GL3 Target DNA Sequence
5'..CTTACGCTGAGTACTTCGA..3' (SEQ ID NO: 9)

### siRNA-GL3.1-

The siRNA that targets the let-7 target sequence is:

### siRNA-let7.1:

Let-7 Target DNA Sequence
5'.. TATACAACCTACTACCTCA..3' (SEQ ID NO: 12)

### siRNA-let7.1-

Each RNA oligo pair is annealed as described in Elbashir et al. (2001) Nature 411:494-498 in order to obtain the duplexed siRNA.

### C. Co-transfection of pGL3-reporter constructs and siRNAs

The let-7 targeting system is tested by transfecting a DNA/RNA mixture consisting of three components into host cells (for example HeLa or PER.C6/E2A cells):
1. Luciferase-based reporter construct
   a. pGL3-control (Promega), or
   b. pGL3-tLet-7F, or
   c. pGL3-tLet-7R
2. Internal control for normalization
   a. pRL-TK (Promega; Acc. Number AF025846)
3. Duplexed siRNA
   a. siRNA-GL3.1, or
   b. siRNA-let-7.1, or
   c. siRNA-GL2.1

### Day 1:

HeLa or PER.C6/E2A cells are seeded 20 hrs prior to transfection in 96-well format at 4.5 x 10⁴ cells/100 µl medium (DMEM +10% heat inactivated Fetal Bovine Serum for HeLa cells; DMEM +10% non-heat inactivated Fetal Bovine Serum for PER.C6/E2A cells)/well.

### Day 2:

Per well DNA/RNA mixtures are prepared in 25 µl (total volume) OptiMEM containing:
1. 0.25 µg pGL3-control, or
   0.25 µg pGL3-tLet-7F, or
   0.25 µg pGL3-tLet-7R
2. 25 ng pRL-TK
3. 66.5 ng siRNA-GL3.1, or
   66.5 ng siRNA-let-7.1, or
   66.5 ng siRNA-GL2.1, or
   no siRNA

LipofectAMINE2000 (0.8 µl) and OptiMEM (24.2 µl) are incubated for 7-10 minutes at room temperature and added to each DNA/RNA mixture. This mixture (final volume 50 µl) is incubated for 15-25 minutes at room temperature and subsequently added to the cells from which the medium has been removed. The cells are incubated for 48 hrs in a 37°C incubator under 10% CO₂.

### Day 4:

The cells are harvested, lysed, and firefly luciferase and renilla luciferase activities measured using the Dual Luciferase kit (Promega) according to the manufacturer's instructions. The absolute firefly luciferase value (luc) of each sample is divided by its internal absolute renilla luciferase value (ren) to obtain the relative luc/ren-value. These relative luc/ren-values are compared to the control sample where no siRNA is included.

The results of transient transfection on PER.C6/E2A are shown in Figure 1. It shows the repression of luciferase activity of pGL3-fusion constructs containing let-7 target sequences by let-7 siRNAs in PER.C6/E2A cells. PER.C6/E2A cells are transiently transfected with the pGL3-fusion constructs containing let-7 target sequences in either orientation (pGL3-tLet-7F, pGL3-tLet-7R) or pGL3-control lacking let-7 sequences, in combination with each of the siRNA duplexes siRNA GL3.1, siRNA let-7.1, siRNA GL2.1, or no siRNA. Co-transfection of siRNA let-7.1 specifically represses luciferase activity of the reporters pGL3-tLet7F and pGL3-tLet7R, but not the pGL3-control. Co-transfection of the positive control siRNA GL3.1 shows repression of all the reporter constructs (pGL3-tLet-7F, pGL3-tLet-7R, pGL3-control).

### Example 2: Testing chimeric let-7 RNAs

This example describes preparation of let-7-based chimeric RNAs, which are tested for the ability to knock down gene expression in the system described in Example 1.

The two complementary RNA strands of the siRNA-duplexes of Example 1 are covalently linked using an RNA loop structure, making a single RNA molecule containing both siRNA strands. This results in a molecule folding into an RNA-duplex with a loop structure on one side of the duplex and a 3' overhang of 2 uridine residues on the other side of the duplex. Molecules containing this loop structure and the sequences are referred to as chimeric RNAs. The constructs are referred to as follows: loop RNA followed by the gene they are targeted against, e.g. loop RNA GL2.2 is a chimeric RNA molecule containing a loop directed against GL2. The extension '.2' is to indicate that the RNA contains a loop in contrast to the extension '.1' used in Example 1 indicating a duplex RNA without a loop structure. The loop structure used here is the let-7 loop and meant as an example and in no way intended to limit to the invention.

Such a chimeric RNA molecule is shown below (see also sR-hLet7.2-as below). The complementary RNA regions are shown in uppercase, while the loop region and 3' uridines are shown in lowercase. The loop region is also underlined.

Linear representation of a chimeric RNA (SEQ ID NO: 15):

During an annealing reaction, the complementary regions of the above chimeric RNA molecule anneal and form a stem-loop as shown below. A siRNA molecule is shown for comparison.

### Stem-loop structure of chimeric RNA:

### siRNA:

Additionally, chimeric RNAs having 5' and 3' extensions can be generated. The sequence of the 5' and 3' extensions are based upon adjacent let-7L RNA sequence:
5' terminus: 5'-GGCCUUUGGGG..(SEQ ID NO: 16)
3' terminus: ..CCGUGAAGUCCU-3' (SEQ ID NO: 17)

These extension sequences differ from the extensions described by Hutvagner et al. (2001) Science 293:834-838 by one base in order to increase the transcriptional efficiency of T7 RNA Polymerase.

A chimeric RNA molecule with 5' and 3' extensions is shown below. The 5' and 3' extensions are bolded.

Stem-loop structure of chimeric RNA with 3' and 5' extensions SEQ ID NO: 18):

Synthesis of chimeric let-7 siRNAs can be accomplished by *in vitro* transcription of the chimeric RNA using T7 RNA Polymerase, as described below. The template for the polymerase is DNA cloned into pUC19.

### A. Co-transfection of the pGL3 reporter constructs with siRNA, or chimeric RNAs

Similarly, as described in the previous example, here in Example 2 cells are transfected by a mixture of DNA and RNA. The RNA component also contains samples with Loop-RNA versions. The DNA/RNA mixture consists of the following components:
1. Luciferase-based DNA reporter construct
   a. pGL3-control, or
   b. pGL3-tLet-7F, or
   c. pGL3-tLet-7R
2. Internal control for normalization
   a. pRL-TK
3.Annealed RNA
   a. Duplexed siRNA-GL2.1, siRNA (small RNA duplex with overhangs) targeted against GL2, or
   b. Duplexed siRNA-GL3.1, siRNA (small RNA duplex with overhangs) targeted against GL3, or
   c. Annealed Loop RNA-GL3.2, siRNA with a connecting loop targeted against GL3 or
   d. Duplexed siRNA Let 7.1, siRNA (small RNA duplex with overhangs) targeted against Let-7 sequences, or
   e. Annealed Loop RNA-Let-7.2, siRNA with a connecting loop targeted against Let-7 sequences

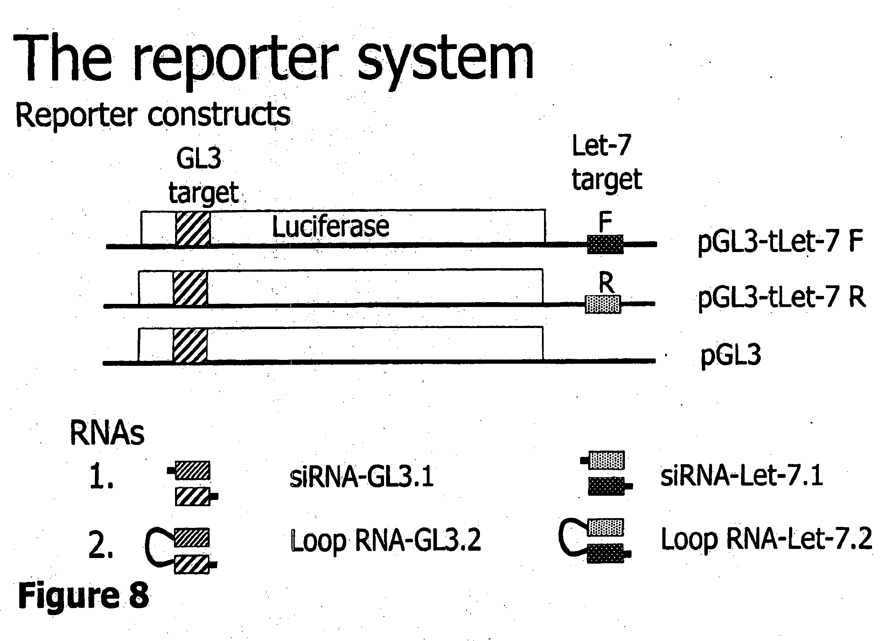

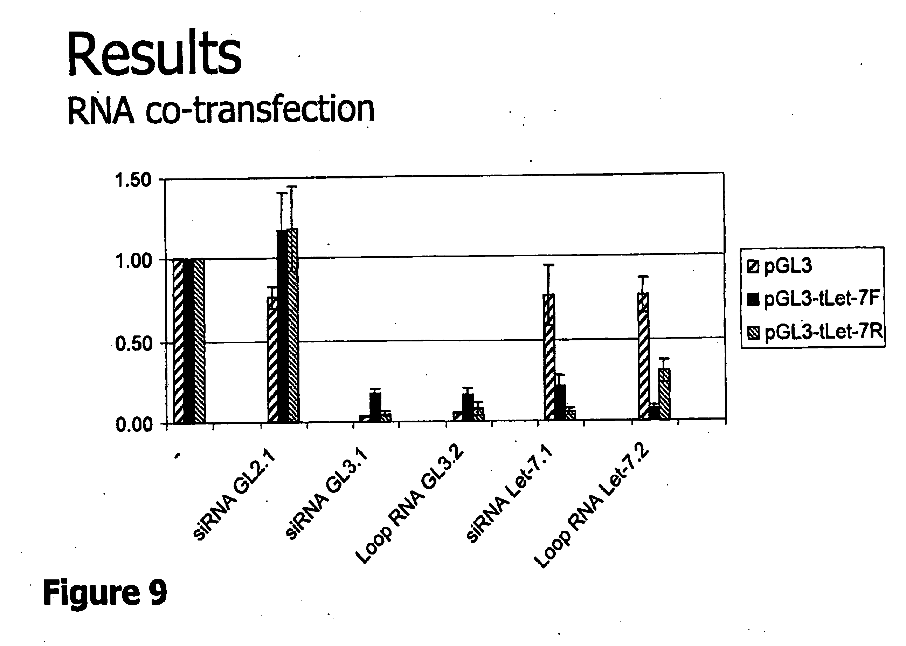

The luciferase-based reporter constructs and the RNAs are schematically represented in Figure 8. Figure 9 shows down-regulation of the reporters containing the target sequences that corresponds to the sequences of the co-transfected RNAs. Again, as shown in Example 1, the siRNA GL3.1 causes a specific reduction of the expression levels of all reporters since they all carry the target sequence. The siRNA Let-7.1 shows only repression of the reporters pGL3-tLet7F and pGL3-tLet7R, but not of the pGL3-control. The control siRNA GL2.1 shows no significant repression of any reporter construct. The chimeric RNA versions with the connecting loop between the duplexed regions, Loop RNA GL3.2 and Loop RNA Let7.2, show the same sequence specific reduction of the reporters as the corresponding siRNAs consisting of the independent RNA strands, siRNAs GL3.1 and Let-7.1, respectively. In conclusion, the RNAs with both strands connected with a loop work as well as the siRNA consisting of two independent strands.

### B. Generation of DNA Fragments Encoding Chimeric RNA Molecules

The template for RNA Polymerase is double stranded DNA. The DNA template is generated by PCR using overlapping oligos that are complementary at their 3' ends. The oligos are shown below.

### Oligonucleotides:

T7-pre-hLet7L.3-F (SEQ ID NO: 19):
Pre-hLet7L.3-R (SEQ ID NO: 20):
T7-pre-GL3.3-F (SEQ ID NO: 21):
Pre-GL3.3-R (SEQ ID NO: 22):

The oligonucleotide pairs (T7-pre-hLet7L.3-F with Pre-hLet7L.3-R; T7-pre-GL3.3-F with Pre-GL3.3-R) are mixed in a 1:1 molar ratio to 40 µM in 50 mM Tris-HCl pH 7.9, 10 mM MgCl₂, 100 mM NaCl buffer. The mixture is incubated for 5 min at 95 °C, three min at 65 °C and the 3' ends are allowed to anneal by slowly (30 min.) decreasing the temperature to 20°C. In the diagram below, forward (F) oligos are shown in uppercase and reverse (R) oligos are shown in lowercase.
Annealed Pre-hLet7L.3 Oligos:
Annealed Pre-GL3.3 Oligos:

Of the annealed oligos, 2 µl are incubated in the extension mixture (40 µl final volume) consisting of 10 mM Tris-HCl pH 7.5, 5 mM MgCl₂, 7.5 mM dithiothreitol, 33 µM of each dNTP, 20 U DNA Polymerase I, Large (Klenow) fragment for 15 minutes at room temperature.
Extension of Annealed Pre-hLet7L.3 Oligos:
Extension of Annealed Pre-GL3.3 Oligos:

The DNA fragments generated by the extension of the oligos are shown in their entirety below. The sequences are given as double stranded DNA; upper strand (uppercase) 5' to 3'; lower strand (lowercase) 3' to 5'. T7 promoter (nts 9-26); cloning sites: Hind III site (nts 4-9); EcoR I site (nts 119-124); restriction site for linearization Cac8I or Nae I (nts 113-118).
Pre-hLet7L.3:
   121 ATTCATGCG-3' (SEQ ID NO: 23)
   taagtacgc-5' (SEQ ID NO: 24)
Pre-GL3.3:
   121 ATTCATGCG-3' (SEQ ID NO: 25)
   taagtacgc-5' (SEQ ID NO: 26)

### C. Cloning of DNA Fragments into pUC19

The PCR products are digested with Hind III and EcoR I and cloned into pUC19 digested with the same enzymes using published methods (Sambrook et al.)

### D. In vitro Transcription using T7 RNA Polymerase

The templates are linearized with Cac8I or Nae I and used for *in vitro* transcription with T7 RNA Polymerase (Promega) according to the manufacturer's instructions. Afterwards the template DNA is removed with DNase (Promega) according to the manufacturer's instructions.

The *in vitro* synthesized RNA is self-annealed using the same annealing protocol as for the siRNA oligo-mixtures in Example 1. Transfections are performed as described in Example 1, except that the self-annealed RNA replaces the siRNA used in Example 1.

The linear sequences of the chimeric RNAs generated using this method are shown below along with a stem-loop representation of each chimeric RNA. The uppercase sequences are the regions that are able to basepair within the RNA, one of the sequences is able to anneal to the target sequence; in these cases, the first uppercase region.

In the next section the names for the RNA molecules are composed as follows: 's' stands for siRNA, 'R' stands for RNA, and 'h' indicates human sequence. Whenever Pre is present in the name it indicates that the molecules contains 5'- and 3'-extensions.

### sR-hLet7.2-as (Loop RNA Let7.2)

let-7 siRNAs connected by the let-7 loop, lacking 5' and 3' extensions:

### sR-GL3.2-as (Loop RNA GL3.2)

GL3 siRNAs connected by the let-7 loop, lacking 5' and 3' extensions:

### Pre-hLet7L.3

let-7 siRNAs connected by the let-7 loop and containing 5' and 3' extensions:

### Pre-GL3.3

GL3 siRNAs connected by the let-7 loop and containing 5' and 3' extensions:

The uppercase sequences are the regions of complementarity within the RNA molecule, one of the sequences is able to base pair with the target sequence; in these cases, the first uppercase region.

The RNAs sR-hLet7.2-as and sR-GL3.2-as are chemically synthesized, and can also be generated using the above procedure.

### E. Analysis of functional connecting loop sequences

The size of the wild type Let-7 connecting loop is 30 nts (5'-GUUUGGGGCUCUGCCCUGCUAUGGGAUAAC-3' (SEQ ID NO: 75).

Ideally one prefers to generate synthetic oligos and clone them into an expression vector (see below). However, the size of the oligo is preferably not too long to make efficient oligo synthesis possible. One cannot change dramatically the size of the guide sequences, however the loop sequence is 30 nt and can be shortened (see Figure 10 and Figure 11). Different loop sequences are selected, based on the wild type Let-7 loop sequence or other microRNA loop sequences (Lagos-Quintana, M et al. Science (2001) 294: 853, Lau, et al. Science (2001) 294: 858, Lee and Ambros Science (2001) 294: 862

The selected derivatives of the Let-7 loop are based on the predicted secondary folding of the Let-7 loop. Two stable structures of the Let-7 loop are predicted (see SEQ ID NOS: 35 to 41, and 45 below). The bulging 12 nucleotides and the predicted GGG/CCC stem in the loop are deleted in structure 2 resulting in loop L12, (SEQ ID NO: 30) (see also Figure 10 and Figure 11). Deleting the UGGG/CCUG part of the predicted stem and the bulged nucleotides in the loop in structure 1 results in loop L11 (SEQ ID NO: 31).

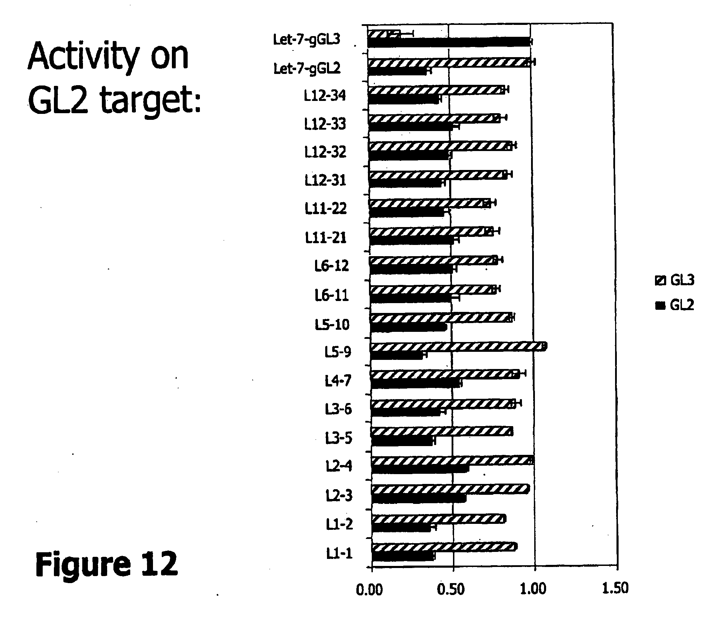

The selected loops of the micro RNAs are chosen since they are shorter and have the same organization of 5'-guide-loop-basepairing sequence-3' as the Let-7 RNA. Loops of microRNAs with the reverse organization, 5'- basepairing sequence-loop-guide-3', might also be functional. Constructs are synthesized in the context of a GL2 guide sequence using PCR as described above and their knock-down efficiencies on GL2 reporter are measured in a transient transfection experiment as described in Example 3 and compared to the wild type Let-7 loop (Figure 12). All loops show knock-down effects, which are specific for GL2 and do not effect GL3 reporter. Loop sequences as short as 12 nucleotides (e.g. L12) still specifically knock-down the GL2 reporter.

### Example 3: Let-7 promoter for expression

This example describes a DNA expression construct producing siRNA, and the identification and cloning of the human let-7 promoter and human let-7 genomic sequence in a DNA vector. The let-7 promoter is used in the expression construct to produce siRNAs. However, as described below, other promoters can be used as well.

Included in this Example are:
1. Results of a DNA database search using let-7 guide sequence as a probe;
2. Predicted secondary structures of RNAs transcribed from let-7 genomic clones;
3. Description of isolation of human let-7 promoter;
4. Description of isolation of human let-7 genomic clone; and
5. Methods for modifying let-7 genomic constructs.

### A. Cloning of the Let-7 promoter

A DNA database search using let-7 guide sequence as a probe results in three perfect matches on the human genome, on chromosomes 9, 11, and 22, and five near perfect matches on chromosomes 9, 21, X, 19 and 5 (see also Pasquinelli et al. (2000) Nature 408:86-89):
1) >ref|NT_011523.4|Hs22_11680 Homo sapiens chromosome 22
2) >ref|NT_009215.3|Hs11_9372 Homo sapiens chromosome 11
3) >ref|NT_025808.2|Hs9_25964 Homo sapiens chromosome 9
4) >ref|NT_011512.3|Hs21_11669 Homo sapiens chromosome 21
5) >ref|NT_011799.5|HsX_11956 Homo sapiens chromosome X
6) >ref |NT_011091.5| Hs19_11248 Homo sapiens chromosome 19
7) >ref | NT_027021.1 | Hs5_27181 Homo sapiens chromosome 5

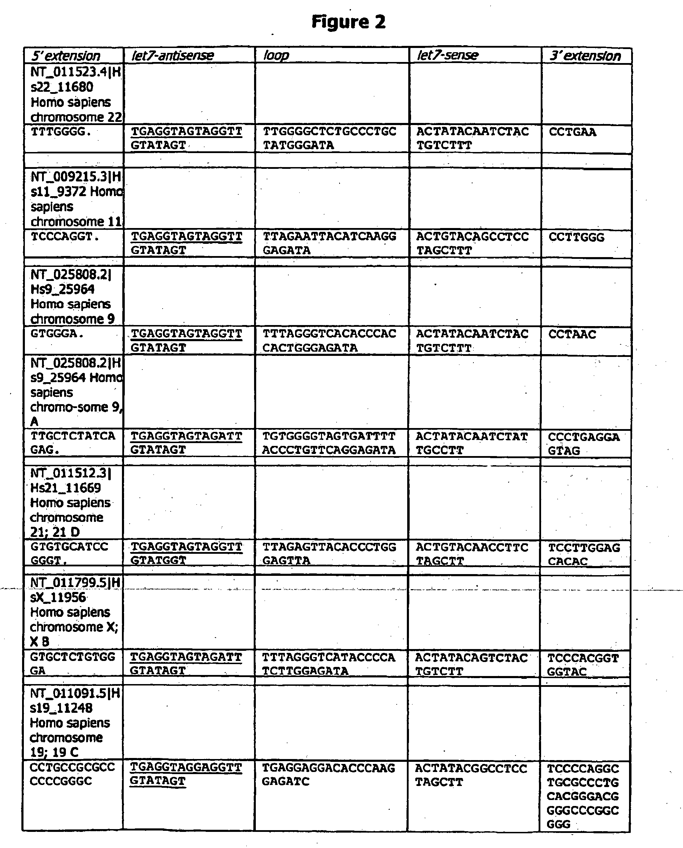

All locations are potential candidates for the let-7 gene. From these locations RNA structures are predicted that fold into RNA duplex structures similar to let-7 RNA (schematically shown below). For comparison, in Figure 2 the potential structure is divided into the segments: 5' extension, let-7-antisense, loop, let7-sense, and 3' extension. The let-7-antisense sequence is underlined.

The following references provide computer programs to predict the secondary structure of the three predicted RNAs transcribed from the above human chromosomal DNA:
M. Zuker, D.H. Mathews & D.H. Turner, "Algorithms and Thermodynamics for RNA Secondary Structure Prediction: A Practical Guide", In RNA Biochemistry and Biotechnology (1999) 11-43, J. Barciszewski & B.F.C. Clark, eds, NATO ASI Series, Kluwer Academic Publishers, (mfold version 3.1*)*
D.H. Mathews, J. Sabina, M. Zuker & D.H. Turner, "Expanded Sequence Dependence of Thermodynamic Parameters Improves Prediction of RNA Secondary Structure", J. Mol. Biol. (1999) 288:911-940.

The following prediction of secondary structure results from the use of the *mfold* version 3.1 program by Zuker and Turner (the let7-antisense sequence is underlined):
1) NT_011523.4|Hs22_11680 Homo sapiens chromosome 22
   Structure 1
   Folding bases 1 to 81
   Initial dG = -36.1 Structure 2
   Folding bases 1 to 81
   Initial dG = -34.4 In Hutvagner et al. (2001) Science 293:834-838, predictions and experimental data for longer precursors (4 extra base pairs at the termini) derived from this genomic location are reported.
2) NT_009215.3|Hs11_9372 Homo sapiens chromosome 11
   Structure 1
   Folding bases 1 to 80
   Initial dG = -33.7
3) NT_025808.2|Hs9_25964 Homo sapiens chromosome 9
   Structure 1
   Folding bases 1 to 83
   Initial dG = -36.6 Structure 2
   Folding bases 1 to 83
   Initial dG = -35.8
4) NT_025808.2|Hs9_25964 Homo sapiens chromosome 9, A (302576-302677)
   Structure 1
   Folding bases 1 to 102
   Initial dG = -49.1
5) NT_011512.3|Hs21_11669 Homo sapiens chromosome 21:21 D Structure 1
   Folding bases 1 to 92
   Initial dG = -42.1 Structure 2
   Folding bases 1 to 92
   Initial dG = -40.8
6) NT_011799.5|HsX_11956 Homo sapiens chromosome X working draft sequence segment: X B
   Structure 1
   Folding bases 1 to 96
   Initial dG = -48.0 Structure 2
   Folding bases 1 to 96
   Initial dG = -47.5
7) NT_011091.5 |Hs19_11248 Homo sapiens chromosome 19; 19 C Structure 1
   Folding bases 1 to 122
   Initial dG = -65.8 Structure 2
   Folding bases 1 to 122
   Initial dG = -62.8

By limiting the length of the sequences discovered in chromosome 19 to about 80 nucleotides, a predicted stem-loop structure similar to those shown for the sequences found on the other chromosomes is produced by starting from the 5' direction with the first of the base-pairing nucleotides. The endogenous promoter for let-7 is cloned using PCR on human genomic DNA. Primers are designed for all three genomic locations flanking the let-7 sequences (e.g., 5'-TGAGGTAGTAGGTTGTATAGT-3' SEQ ID NO: 32). Two different reverse primers, which anneal 3' of the 3' let-7 extension sequence, are utilized. In addition, three different forward primers, which anneal 5' of the 5' let-7 extension, are used. In order to ensure that a fully functional, but yet minimal let-7 promoter is identified, the three forward primers anneal at different distances upstream from the let-7 transcription initiation site. The following examples use the sequences derived from chromosome 22. Similarly, the sequences from the other chromosomes can be used. The primers for chromosome 22 are listed below.
Oligonucleotides (5' to 3') for location
NT_011523.4|Hs22_11680 Homo sapiens chromosome 22:

| | |
|---|---|
| Let7gene22 F1 42) | 5'-GCACGTTCTAGAGAATCCCTGTGCCCTTGGTG (SEQ ID NO: |
| Let7gene22 F2 43) | 5'-GCACGTTCTAGACCGTGAAGCCGCTACTCAGC (SEQ ID NO: |
| Let7gene22 F3 44) | 5'-GCACGTTCTAGAGGGTTGACAGTCGTATCTGC (SEQ ID NO: |
| Let7gene22 R1 45) | 5'-CCGTGCAAGCTTTGTCAGACTTCTCAGTGTAG (SEQ ID NO: |
| Let7gene22 R2 46) | 5'-CCGTGCAAGCTTCCTGCCACTGAGCTGGCCAG (SEQ ID NO: |

The sequences matching genomic regions are underlined. The other 5' sequences are added to facilitate cloning (Xba I site in primers Let7gene22 F1-3, Hind III in primers Let7gene22 R1-2).

Different primer combinations are utilized to obtain genomic fragments:

### Name fragment Forward primer Reverse primer

| | | |
|---|---|---|
| Let7gene22A | Let7gene22 F3 | Let7gene22 R1 |
| Let7gene22B | Let7gene22 F2 | Let7gene22 R1 |
| Let7gene22C | Let7gene22 F1 | Let7gene22 R1 |
| Let7gene22D | Let7gene22 F3 | Let7gene22 R2 |
| Let7gene22E | Let7gene22 F2 | Let7gene22 R2 |
| Let7gene22F | Let7gene22 F1 | Let7gene22 R2 |

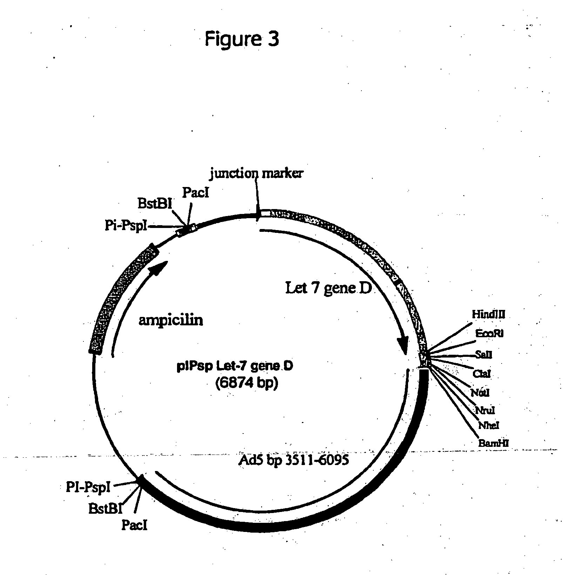

The PCR fragments are digested with the enzymes Xba I and Hind III and cloned into the Avr II site and the Hind III site of pIPspAdapt6-deltaPolyA, thereby replacing the CMV promoter sequence (see Figure 3). pIPspAdapt6-deltaPolyA was constructed from pIPspAdapt6 as follows. pIPspAdapt6 was grown in the methylase negative E. coli strain DM1 to prevent methylation of the second Xba-site. The DNA was isolated and digested with Xba I, thereby excising a 142 bp fragment containing the poly A signal. The religated vector is called pIPspAdapt6-deltaPolyA.

These constructs with the genomic fragments Let7gene22A-F are transfected into mammalian cells (as described in Example 1) and tested for expression of let-7 RNA and its effect on repressing pGL3-tlet7. This is done by different means:

### Repression of luciferase activity from pGL3-tLet7-F, or pGL3-tLet7-R reporter.

The fragments Let7gene22A, Let7gene22B, and Let7gene22C represent different lengths of the genomic sequences of the Let7 gene on chromosome 22. These fragments are cloned into pIPspAdapt6-deltaPolyA plasmids. Reporter constructs are used to test the Let-7 expression plasmids Let7gene22A, Let7gene22B, Let7gene22C in an experiment similar to Example 1, but with the following deviation:

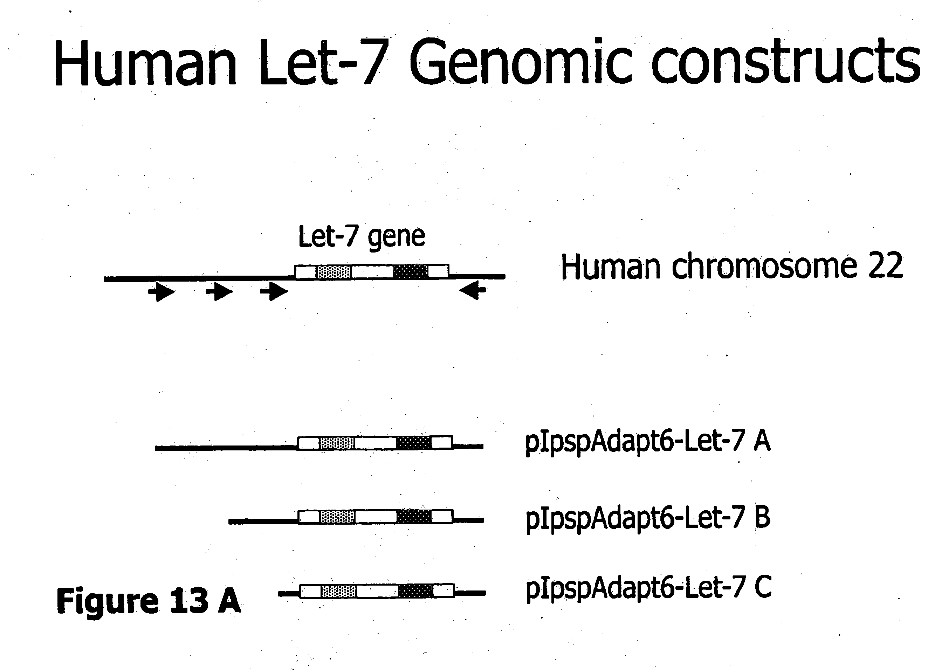

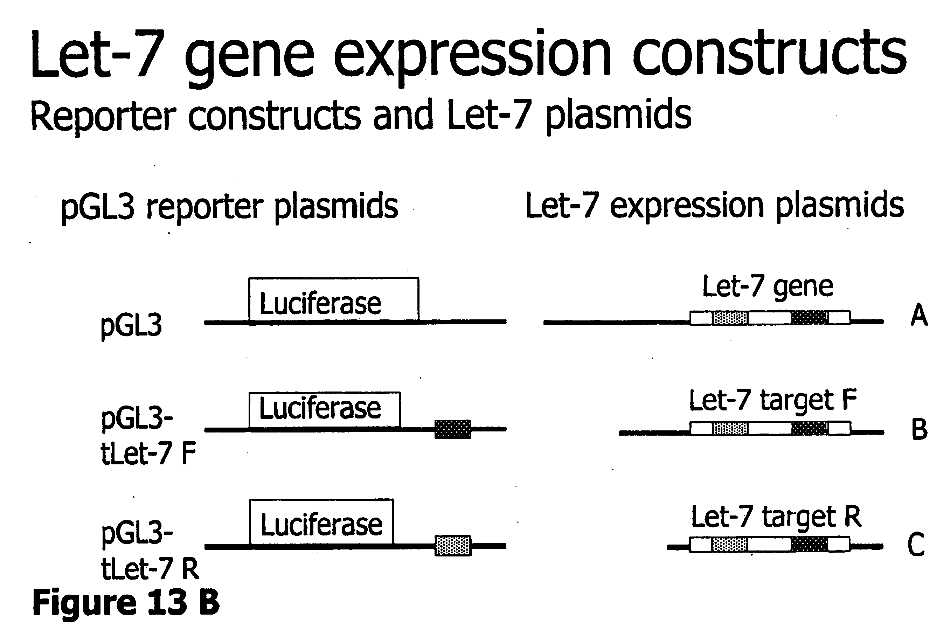

The Let-7-based expression plasmids replace the RNAs, as schematically illustrated in Figure 13A-B.

The DNA mixture consisting of the following three components is transfected into host cells (for example HeLa or PER.C6/E2A cells):
4. Luciferase-based reporter construct
   a. pGL3-control (Promega), or
   b. pGL3-tLet-7F, or
   c. pGL3-tLet-7R
5. Internal control for normalization
   a. pRL-TK (Promega; Acc. Number AF025846) 6. Let-7 based expression constructs
   a. pIPspAdapt-Let-7gene A (containing fragment Let7gene22A), or
   b. pIPspAdapt-Let-7gene B (containing fragment Let7gene22B), or
   c. pIPspAdapt-Let-7gene C (containing fragment Let7gene22C)

### Day 1:

HeLa or PER.C6/E2A cells are seeded 20 hrs prior to transfection in 96-well format at 4.5 x 10⁴ cells/100 µl medium (DMEM +10% heat inactivated Fetal Bovine Serum for HeLa cells; DMEM +10% non-heat inactivated Fetal Bovine Serum for PER.C6/E2A cells)/well.

### Day 2:

The treatment per well is: DNA mixtures are prepared in 25 µl (total volume) OptiMEM containing:
1. 200 ng pGL3-control, or
   200 ng pGL3-tLet-7F, or
   200 ng pGL3-tLet-7R
2. 20 ng pRL-TK
3. 100 ng Let-7 based expression construct, pIPspAdapt-Let-7geneA, or pIPspAdapt-Let-7geneB, or pIPspAdapt-Let-7geneC.

LipofectAMINE2000 (0.8 µl) and OptiMEM (24.2 µl) are incubated for 7-10 minutes at room temperature and added to each DNA mixture. This mixture (final volume 50 µl) is incubated for 15-25 minutes at room temperature and subsequently added to the cells from which the medium has been removed. The cells are incubated for 48 hrs in a 37°C (HeLa) or 39°C (Per.C6/E2A) incubator under 10% CO₂.

### Day 4:

The cells are harvested, lysed, and firefly luciferase and renilla luciferase activities measured using the Dual Luciferase kit (Promega) according to the manufacturer's instructions. The absolute firefly luciferase value of each sample is divided by its internal absolute renilla luciferase value to obtain the relative luc/ren-value. These relative luc/ren-values are compared to the control sample where no or non-related plasmid is used.

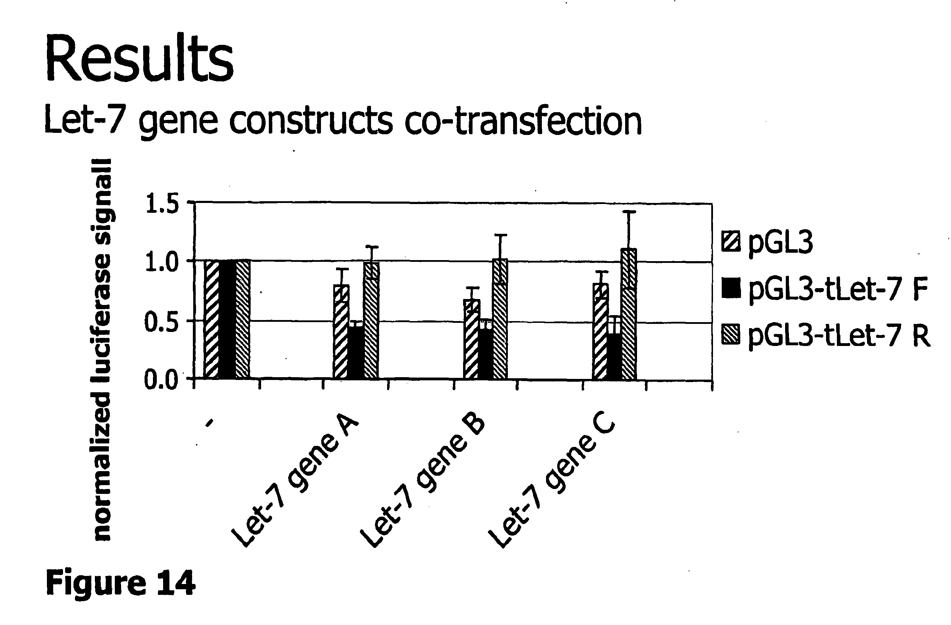

The results of the transient transfection on PER.C6/E2A cells are shown in Figure 14.

Cotransfection of reporter constructs and Let-7 expression constructs (pIPspAdapt-Let-7gene A-C) show the activity of the Let-7 expression constructs on the reporter construct containing the let-7 target sequence in the F-orientation; pGL3-tLet-7-F. The pGL3-tLet-7-R does not show a knock-down, probably due to asymmetric processing of the Let-7 RNA. The pGL3 reporter, lacking Let-7 target sequence, is not affected. The smallest region (338 bps), Let-7 gene-C, is sufficient to have knock down activity as shown in this experiment.

### Reduction of the luciferase levels.

The procedure is the same as described in Example 1, with the major difference being that the siRNA-Let7.1 is replaced by the plasmid constructs containing Let7gene22A-F. The mRNA levels of GL3 luciferase are measured for sequence specific degradation using Taqman PCR (PE Applied Biosystems) according to the manufacturer's instructions. RNA is isolated using TRIzol Reagent (Life Technologies), according to the manufacturer's protocol, followed by DNase treatment using 0.1 U/µl RQ1 RNase (DNase free; Promega) for 20 min at 37 °C. A single tube reaction is performed for the cDNA synthesis and the subsequent Taqman PCR reaction containing 100 ng total RNA in a total volume of 25 µl consisting of 1x TaqMan buffer A (PE Applied Biosystems), 5 mM MgCl₂, 300 µM total dNTPs, 300 nM luc-1-For, 300 nM luc-1-Rev, 150 nM luc-1-probe, 0.025 U/µl AmpliTaq Gold, 0.1 U/µl RNase Inhibitor, and 0.25 U/µl MultiScribe Reverse Transcriptase. The Taqman analysis is performed on an ABI Prism 7700 Sequence Detector apparatus.

The primers and probe combination used in this analysis are as follows:

### Primers:

| | |
|---|---|
| luc-1-For | 5' AAGCGACCAACGCCTTGAT 3' (SEQ ID NO: 47) |
| luc-1-Rev 5' | TTCGTCTTCGTCCCAGTAAGC 3' (SEQ ID NO: 48) |

### TaqMan probe (5' FAM Reporter Dye; 3' TAMRA):

| | |
|---|---|
| luc-1-probe | 5' ATGTCTCCAGAATGTAGCCATCCATCCTTG 3' (SEQ ID NO: 49) |

The reaction is performed using the following program:
30 min at 48°C, 10 min at 95°C followed by 40 cycles of [15 sec 95°C, 1 min 60°C].

### 1) Expression of let-7 RNA.

The plasmid constructs containing Let7gene22A-F are transfected into mammalian cells and harvested for RNA isolation. The RNA is analyzed by Northern blotting for let-7 RNA expression as described in Pasquinelli et al. (2000) Nature 408:86-89.

### B. Reprogramming Let-7 RNA to another target sequence.

The term "reprogramming" refers to the replacement of let-7 guide sequences with guide sequences based upon some other RNA sequence. The GL3 sequence is used in this example. The reprogrammed constructs are made by removing the let-7 guide sequence and its complement and replacing them with GL3 guide sequence and its complement. Such a reprogrammed chimeric RNA would target RNA containing the GL3 target sequence as opposed to RNAs containing the let-7 target sequence. These constructs are based on the plasmids having the Let7gene22A-F fragments.

The new guide sequence and a sequence able to basepair with the guide sequence are introduced into the constructs using PCR. For example, for the let-7 genomic location on chromosome 22, a GL3 sequence is introduced; two primers consisting of a forward (Let-7.N19-F4) and reverse primer (Let-7.N19-R4) are designed anti-parallel and pointing away from each other, each containing let-7 sequences at their 3' end, the new target sequence internally (N19), and let-7 loop sequences at their 5' end. The 5' sequences of the primers are complementary for 14 bases.
Let-7.N19-F4:
Let-7.N19-R4:
N19 is the target sequence of interest; for GL3 the N19 is:
   5'-CTTACGCTGAGTACTTCGA-3'. (SEQ ID NO: 9)

The process for replacing guide sequences of let-7 genomic clones is shown in Figure 4. These primers are used in separate PCR reactions on the plasmid constructs containing Let7gene22A-F DNA. Primer Let-7.N19-R4 is used in combination with a forward primer, for instance, Let7gene22 F1-3. Primer Let-7.N19-F4 is used in combination with a reverse primer, for instance, Let7gene22 R1-2. The mixture of the two PCR products thus obtained is used as template in a subsequent PCR reaction using the outside primers Let7gene22 F1-3 and Let7gene22 R1-2. The PCR products obtained from this step are cloned into pIPspAdapt6-deltaPolyA using the same strategy as described for the Let7gene22A-F fragments. The expression of this chimeric let-7 RNA with new guide sequence is analysed by the three methods described above for the let-7 RNA itself:
1. Repression of luciferase activity from pGL3-control, or pGL3-tLet7-F, or pGL3-tLet7-R reporter constructs.
2. Reduction of luciferase mRNA levels.
3. Expression of chimeric let-7 RNA with new target sequence.

In this example, new guide sequences and sequences that can base pair with the guide sequences are introduced into the construct pIPspAdapt-Let-7gene C using PCR with a similar strategy as described in Example 3A.

The new N19 guide sequences are:
Let-7-100%, 5'-TATACAACCTACTACCTCA-3'; (SEQ ID NO: 52)
GL3, 5'-CTTACGCTGAGTACTTCGA-3'; (SEQ ID NO: 9)
GL2, 5'-CGTACGCGGAATACTTCGA-3'; (SEQ ID NO: 6) and
EGFP, 5'-GCTGACCCTGAAGTTCATC-3'; (SEQ ID NO: 53)

The clones generated have the names: plasmid_name-promoter_name-guide sequence, e.g., pIPspAdapt-Let-7-gLet7-100%, pIPspAdapt-Let-7-gGL3, pIPspAdapt-Let-7-gGL2, and pIPspAdapt-Let-7-gEGFP, respectively. The 'g' before the guide sequence is introduced to differentiate promoter sequences (e.g. let-7) from guide sequences (e.g. gLet-7)

The Let-7 target and guide sequences are made 100% complementary in the clone pIPspAdapt-Let-7-gLet7-100% to enable complete base pairing in this region within the RNA. The natural let-7 RNA is not 100% complementary in this region; it contains 2 non-canonical base pairs (G-U base pairs) and a bulged-out U.

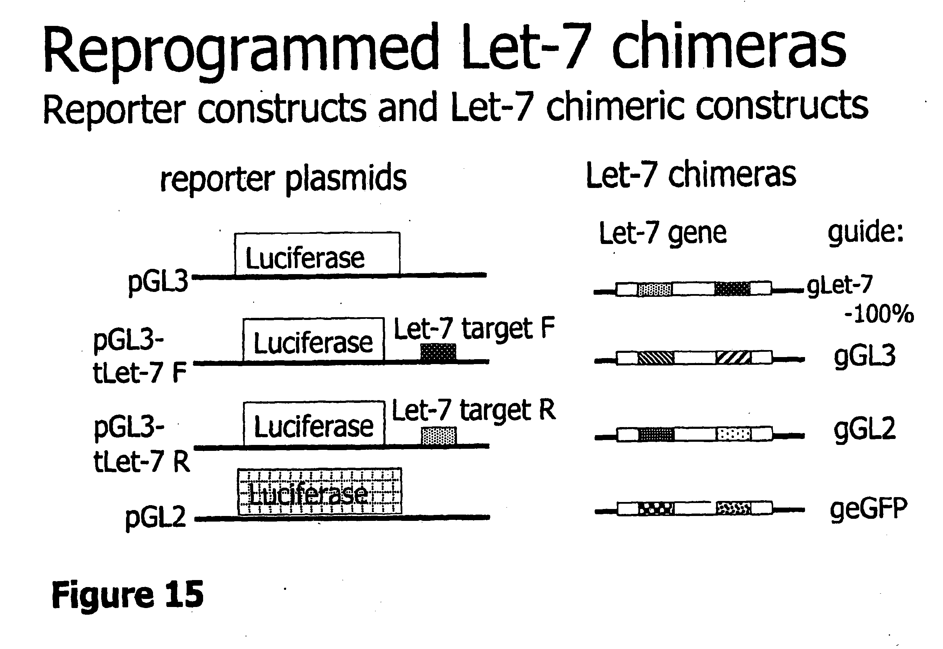

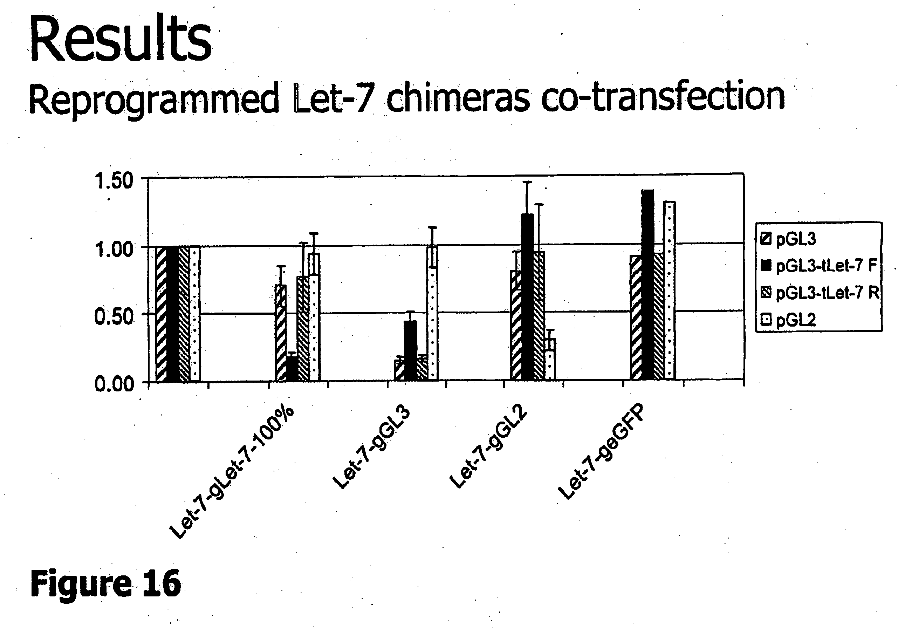

The activity of the clones is tested as described above in the luciferase reporter assay, using luciferase-based reporter constructs (pGL3-control, or pGL3-tLet-7F, or pGL3-tLet-7R, or pGL2). A schematic representation of the components used in this experiment is given in Figure 15. The results are shown in Figure 16. Reduction of the luciferase reporter levels are only observed in the reporters that contain the cognate target sequences, i.e.: plasmid pIPspAdapt-Let-7-gLet-7-100% shows reduction of pGL3-tLet-7-F, plasmid pIPspAdapt-Let-7-gGL3 shows reduction of all three reporters containing the GL3 target sequence (pGL3-control, pGL3-tLet-7F, pGL3-tLet-7R), plasmid pIPspAdapt-Let-7-gGL2 shows reduction of only the pGL2 reporter. No non-specific effects are observed, also confirmed by the plasmid pIPspAdapt-Let-7-gEGFP, which did not repress expression of any of the reporters.

### C. RNA expression of the expression constructs by Northern analysis

In order to show that RNA is transcribed from the DNA expression constructs, expression analysis is performed using Northern blotting.

Cells are transfected with the chimeric expression DNA constructs and analyzed by Northern blotting, exemplified as follows:

### Day 1:

HeLa, U20S, PER.C6/E2A cells are seeded 24 hrs prior to transfection in 6-well format at 30 x 10⁴ cells in 2 ml medium per well. For infection experiments 15 x 10⁴ cells in 2 ml medium per well is used. (DMEM +10% heat inactivated Fetal Bovine Serum for HeLa cells and U2OS cells; DMEM +10% non-heat inactivated Fetal Bovine Serum +10 mM MgCl2 for PER.C6/E2A cells)/well.

### Day 2:

Per well DNA mixtures are prepared in 250 µl (total volume) OptiMEM containing:
3 ug per well in a 6-well plate of the Let-7 based expression construct

LipofectAMINE2000 (4 µl) and OptiMEM (246 µl) are incubated for 7-10 minutes at room temperature and added to each DNA mixture. This mixture (final volume 500 µl/well) is incubated for 15-25 minutes at room temperature and subsequently added to the cells. The cells are incubated for 24, 48, or 72 hrs in a 37°C incubator under 10% CO₂.

### Day 3 and/or 4 and/or 5:

The cells are harvested using TriZol (Invitrogen) according to the manufacturer's descriptions. Briefly, cells are washed with 1-2 ml PBS, and lysed in 1 ml TriZol. Samples of 6 wells are pooled and glycogen is added to 50 ug/ml. Chloroform is added (0.2 ml per ml TriZol used). The samples are vortexed, incubated for 5 min., spun at 9300 g for 15 min. The aqueous phase is transferred to a fresh tube. Isopropanol (0.5 ml per ml TriZol) is added and spun at 7500 g for 5 min. Pellet is washed with 80% ethanol, and dissolved in RNase-free water. The concentration of the RNA samples is determined by UV absorption (A260) or by RiboGreen (Molecular Probes) measurement using the manufacturer's descriptions.

Of each sample 0.5-15 ug RNA is prepared for gel analysis. The RNAs are mixed with the denaturing loading dye (TBE Urea sample buffer; Biorad)) and denatured by heating for 3 minutes at 90°C just before loading onto the 15% polyacrylamide 1xTBE-gel (Biorad). The gel is run for 50 min at 200 V. The gel is blotted onto Hybond N+ filter (Amersham) using the Transblot semi-dry Electroblotter (Biorad) for 30 min. at 0.2 A/blot with a maximum of 25 V. The blotted filter is hybridized with a probe using the Rapid Hyb solution (Amersham) according to the manufacturer's descriptions. The probes are made by 5' end labeling using standard procedures with [³²P]□-ATP of the oligonucleotides complementary to the guide sequences.

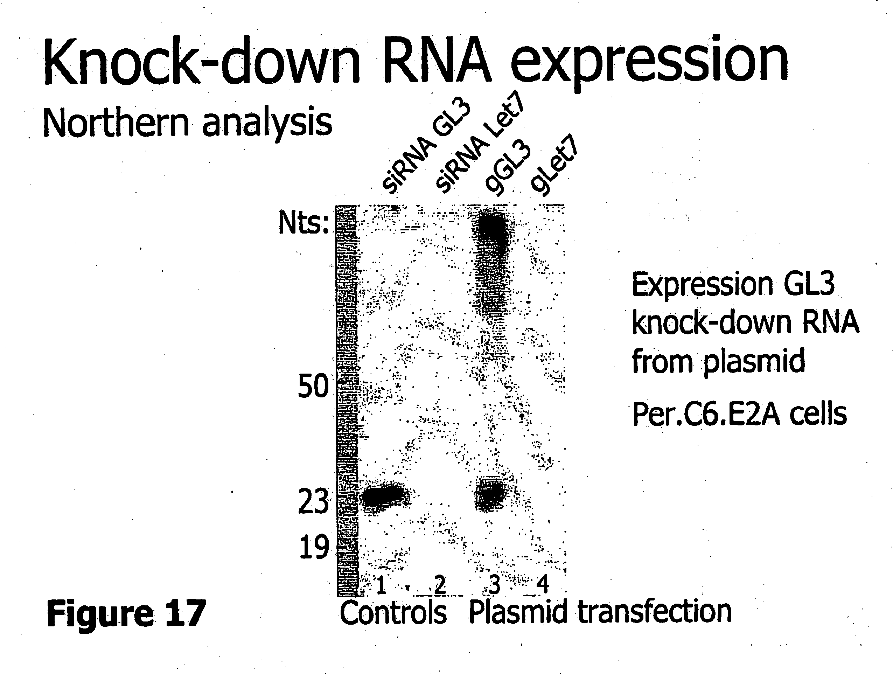

In Figure 17 the expression of the RNA species derived from the construct pIPspAdapt-Let-7-gGL3 is shown. Northern blots of RNA preparations of PER.C6.E2A cells transfected with Let-7-based expression constructs are hybridized with a ³²P-end labelled probe recognizing the GL3 guiding sequences (GL3 probe sequence: 5'-CTTACGCTGAGTACTTCGA-3' SEQ ID NO: 9). The processed RNA species co-migrates with the synthetic GL3 siRNA duplex (21 nts) that has been loaded as a size marker. No signal is detectable in control samples with unrelated sequences.

Similar expression studies have been performed for a number of other Let-7 based constructs with different guiding sequences and their base pairing sequences. The target sequences of these constructs are designed against various targets and are identical to the sequences of the oligonucleotide used in the hybridizations. The target sequences are listed:
PPAR gamma (acc. Number NM_005037; nts 173-191;
   5'-CAGATCCAGTGGTTGCAGA-3', SEQ ID NO: 54)
IGF2R (acc. Number NM_000876; nts 510-528;
   5-GGAGGTGCCATGCTATGTG-3' SEQ ID NO: 55)

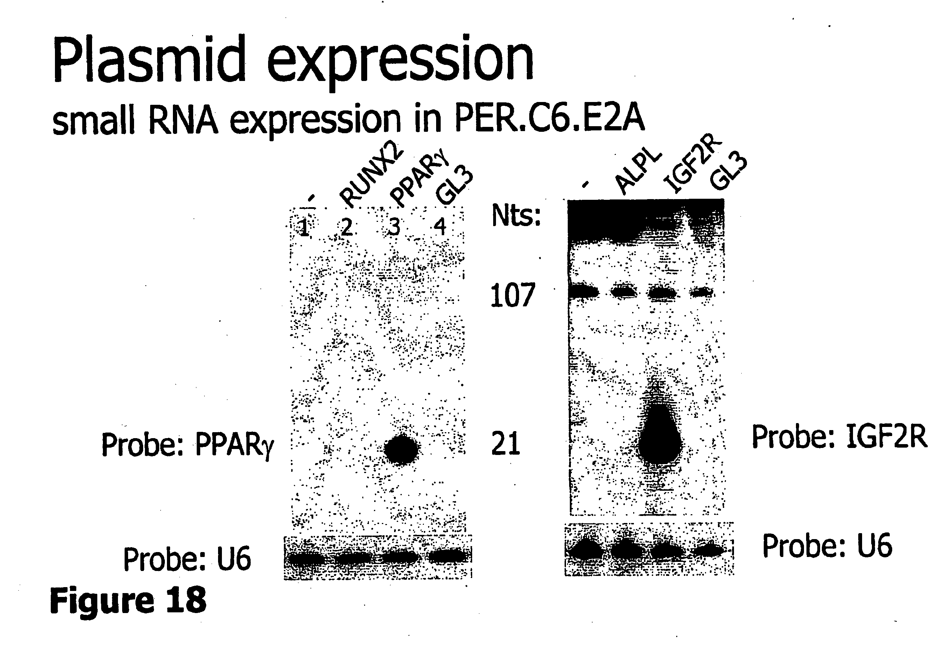

Figure 18 shows that expression plasmids containing sequences other than let-7 or GL3 also express chimeric RNA molecules with the correct length and expected sequence.

### Example 4: RNA polymerase III promoter-based expression

In the examples discussed so far, the expression of the chimeric RNAs are dependent upon the activity of the let-7 promoter. In this example, the let-7 promoter is replaced with a heterologous promoter to be used for chimeric RNA expression. The preferred promoter is ubiquitously active. This example describes the cloning of the constructs from Example 3 into a vector containing a ubiquitously active promoter, in particular, a promoter recognized by RNA polymerase III (pol III promoter).

In eukaryotes, pol III promoters endogenously produce various small, structured RNAs, e.g.: 5S rRNA, tRNAs, VA RNAs, Alu RNAs, H1, and U6 small nuclear RNA. Other suitable promoters that also can be used include CMV, RSV, MMLV, tet-inducible, and IPTG-inducible promoters. Pol III promoters are frequently chosen for expression of therapeutic antisense RNAs or ribozyme RNAs (Medina and Joshi (1999)). The pol III promoters from Medina and Joshi are used to determine the appropriate promoter for transcription of siRNAs, stRNAs and chimeric RNAs.

This approach is used for expression of siRNA, stRNAs and chimeric RNAs using expression constructs based on pol III promoters. An example of this approach is a fusion of a siRNA to the 3' end of a tRNA transcribed from a pol III promoter. The siRNA consists of either an stRNA sequence or a chimeric RNA, which consists of a guide sequence connected via a loop structure to the complementary guide sequence. The relative order of guide sequence and complementary guide sequence can be reversed. Also, the loop structure may be derived from the let-7 loop derived from *C. elegans, drosophila,* or human origin, such as found on chromosomes 9, 11 and 22. The chimeric RNA molecule is processed by the endogenous cellular processing machinery; for example, an siRNA moiety fused to the 3' end of a tRNA would be processed by endogenous molecules including tRNA 3' processing endoribonuclease, thereby releasing the siRNA moiety. The site for cleavage can also artificially be introduced, e.g. a ribozyme target sequence in combination with introduction of the cognate ribozyme.

The activity of the promoter is artificially adapted to obtain an inducible pol III expression system. For example, as described in Meissner et al. (2001) Nuc Acids Res 29:1672-1682.

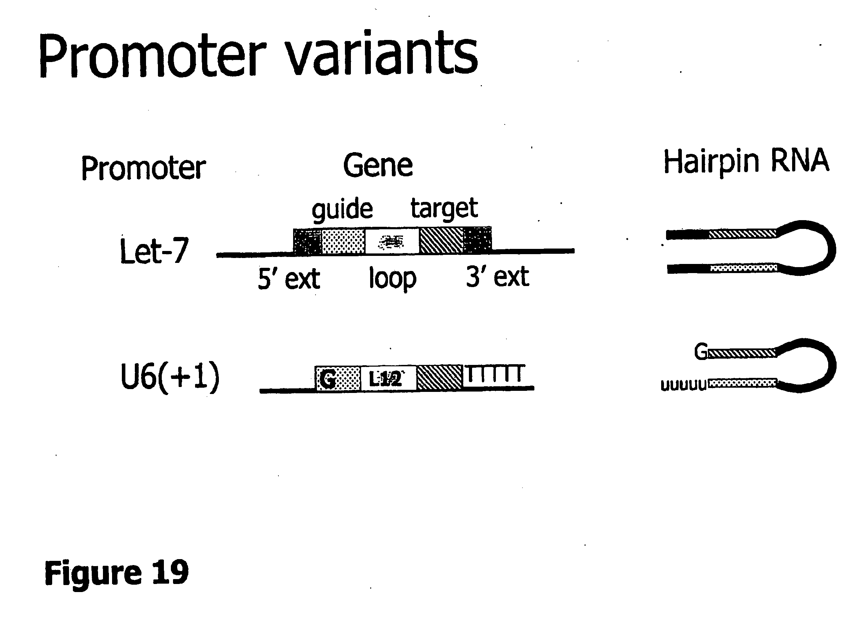

The Let-7 promoter is replaced by a RNA polymerase III promoter. As an example, the Let-7 promoter is replaced by elements of the human U6 snRNA promoter. (Figure 19). For efficient promoter activity the first nucleotide of the transcript is a G and the transcription termination signal is a string of 5 or more Ts. The expressed RNA contains a guide sequence of 19-21 nts (directed against a target) and a sequence able to basepair with the guide sequence connected by a loop sequence.

The genomic human U6 gene (Accession number M14486) is cloned by a PCR based strategy using human genomic DNA. The region to be cloned starts at nucleotide -265 upstream of the transcription start site until nucleotide +198 downstream of the transcription start site. The primers used are:
hU6 (-265)-F1-XbaI 5'-ccgtgcAAGCTTTGGTAAACCGTGCACCGGCGTA-3' (SEQ ID NO: 57)

The PCR product is cloned into the Xba I and Hind III sites of pIPspAdapt6-deltaPolyA in a strategy similar to that described for the cloning of the Let-7 gene (see Example 3).

Starting from this clone, the transcribed U6 snRNA sequence is replaced from nt +2 to nt +102 by an RNA sequence capable of down-regulating target sequences. These RNA sequences preferably contain a guiding sequence, connecting loop, and sequence able to basepair with the guiding sequence, using an assembly PCR strategy, similar to the strategy for replacing the guide/target sequences of the Let-7 genes (see Example 3 above). The first 5' G-nucleotide and the 3' termination signal UUUUU are kept intact.

As.an example, the target sequence directed against the pGL3-tLet-7 reporters is used. The guide sequence described for the Let-7 based promoter (TGAGGTAGTAGGTTGTATA, SEQ ID NO: 58) is shifted 1 nucleotide compared to the guide sequence for the U6-based promoter (GAGGTAGTAGGTTGTATAG; SEQ ID NO: 59) in order to obtain a G-nucleotide at position 1 in the U6 context. Transcription is much more effective if the starting nucleotide is a G. The guide sequence used is completely complementary to the target sequence.

Expression plasmid:

pIPspAdapt-U6(+1) -L12-glet7 is a pIPspAdapt plasmid with a U6(+1) promoter followed by a let7 guiding sequence and containing the L12 loop sequence (SEQ ID NO: 30).

### Primers used for cloning:

GJA 141 (SEQ ID NO: 60)
   5'-GTTTGCTATAACTACAACCTACTACTTTTTACATCATCAGGTTG-3'
GJA 142 (SEQ ID NO: 61)
   5'-GTTATAGCAAACTATACAACCTACTACCTCGGTGTTTCGTCCTTTC-3'

Resulting RNA sequence (guide underlined):
5'-GAGGUAGUAGGUUGUAUAGUUUGCUAUAACUAUACAACCUACUACUUUUU-3' (SEQ ID NO: 62)

The predicted RNA folding:
Structure 1
   Initial dG = -29.8 pIPspAdapt-U6(+1) -L13-glet7 is a pIPspAdapt plasmid with a U6(+1) promoter followed by a let7 guiding sequence and containing the L13 loop sequence (SEQ ID NO: 66).

### Primers used for cloning:

GJA143 (SEQ ID NO:63)
   5'-GTTGTGCTACAACTACAACCTACTACTTTTACATCAGGTTG-3'
GJA144 (SEQ ID NO:64)
   5'-GTTGATAGCACAACTATACAACCTACCTCGGTGTTTCGTCCTTTC-3'
RNA sequence (guide underlined):
   5'-GAGGUAGUAGGUUGUAUAGUUGUGCUAUCAACUAUACAACCUACUACUUUUU-3' (SEQ ID NO: 65)

The predicted RNA folding:
Structure 1
   Initial dG = -32.6

In the two examples given above, the RNA sequence starts with 5'-G(G/A)(G/A) to enable base pairing with the 3' terminus of the RNA (...UUUUU-3'). The underlined Us do basepair with the extreme 5' end of the RNA. This results in a 2 nts overhang at the 3' end. Constructs that do not have G(G/A)(G/A) as starting sequence are also possible; they have 5 nts overhang at the 3' end (see below).
Briefly, two PCR reactions are performed on the human U6 clone to generate a 'left fragment' and a 'right fragment'. As an example, the cloning of pIPspAdapt-U6(+1) -L12-glet7 is given. However in principle any guiding sequence can be constructed following the instructions below:

The primers used are:
For the 'Left fragment':
   hU6(-265)-F1-XbaI (SEQ ID NO: 56)
      5'-GTTATAGCAAACTATACAACCATCATCCTCGGTGTTTCGTCCTTTC-34
   U6-R2: GJA 142 (SEQ ID NO: 61)
      5'-GTTATAGCAAACTATACAACTACTACCTCGGTGTTTCGTCCTTTC-3'
For the 'Right fragment':
   U6-F2: GJA 141: (SEQ ID NO: 60)
      5'-GTTTGCTATAACTATACAACCTACTACTTTTTACATCAGGTTG-3'
   hU6(0198)-R1-HindIII (SEQ ID NO: 57)'
      5'-ccgtgcAAGCTTTGGTAAACCGTGCACCGGCGTA-3

Two primers consisting of a forward (U6 -F2/GJA141, SEQ ID NO: 60) and reverse primer (U6 -R2/GJA142, SEQ ID NO: 61) are designed anti-parallel and pointing away from each other, each containing U6 sequences at their 3' end, the new target sequence internally (N19), and connecting loop sequences at their 5' end. The 5' sequences of the primers are complementary for 12 bases.

The obtained 'left fragment' and 'right fragment' are gel isolated and combined in a one to one ratio to be the template in the subsequent assembly PCR reaction with the outside primers.

### Assembly PCR:

hU6(-265)-F1-XbaI (SEQ ID NO: 56)
   5'-GcacgTTCTAGAAGGTCGGCAGGAAGAGGGCCT-3'
hU6(+198)-R1-HindIII (SED ID NO: 57)
   5'-ccgtgcAAGCTTTGGTAAACCGTAAACCGTGCACCGGCGTA-3'

The PCR products obtained from this step are cloned into pIPspAdapt6-deltaPolyA using the same strategy as described for the Let7gene22A-F fragments (Example 3).

The obtained plasmids are tested in the reporter system described in Examples 1 to 3. The experiment is performed similarly as described in Example 3 for the Let-7 based plasmids, but in this example these plasmids are replaced by the U6-promoter based plasmids (pIPspAdapt-U6(+1) - glet7-L12 and pIPspAdapt-U6(+1) - glet7-L13)

Northern blots of samples of cells transfected with the U6-based promoter expression plasmids show expression of the RNA species and processing into a species of a size comparable to that of synthetic siRNAs.

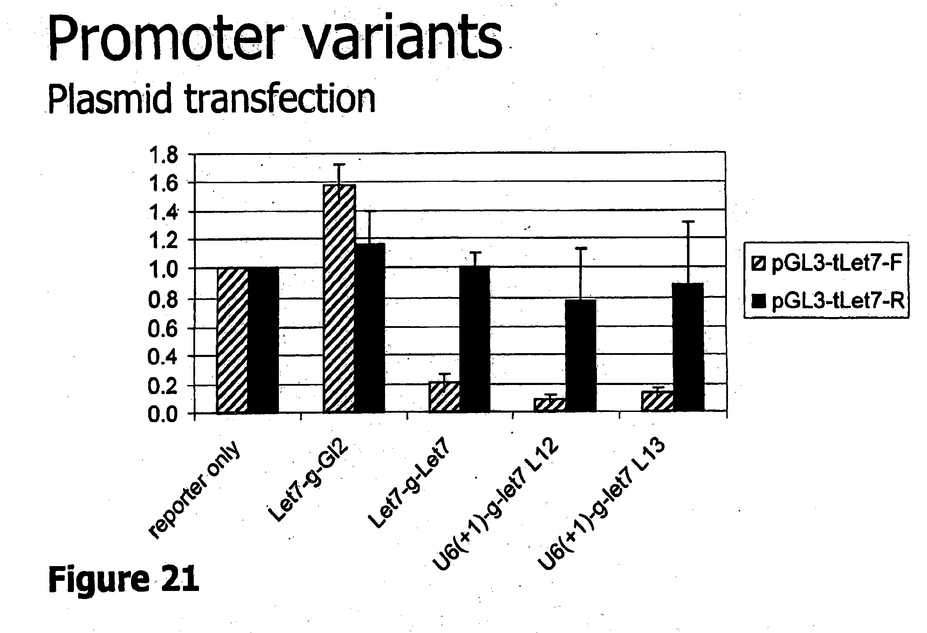

The results given in Figure 21 clearly show repression of the reporter plasmids with the target Let-7 sequences (pGL3-tLet-7F and to some extent pGL3-tLet-7R). In this experiment the U6 promoter-based expression plasmids show stronger knockdown effects than the let-7 promoter-based expression plasmids. Both loop sequences tested show similar activity.

### Example 5: Co-infection with virus

The experiments described above are all transient transfection experiments, i.e. plasmid DNA is transfected into PER.C6/E2A cells. In order to test the activity of the knockdown in the presence of adenovirus in the cell, the following experiment is performed. PER.C6 cells, which do not complement E2A deleted adenovectors, are transfected with either siRNA or expression plasmids as described previously in Examples 2 and 3, but with one deviation: 20-24hrs prior to transfection the cells are infected with adenovirus. The viruses, which cannot replicate in these cells, express EGFP so it can be determined that the transduction efficiency is over 90% using an MOI of 3. Different viral backbones are tested. (See EP 1191105)

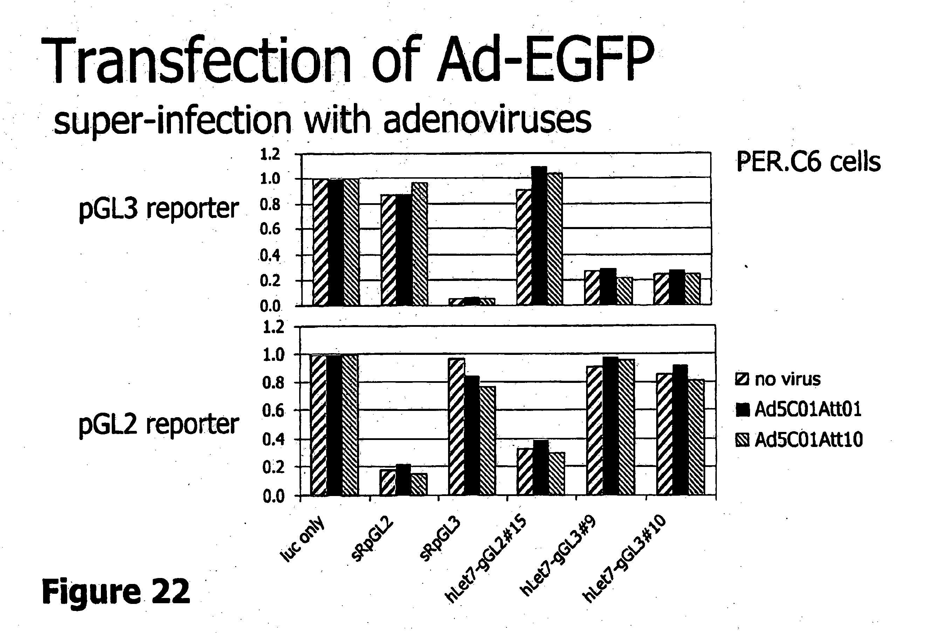

Infection of adenovirus per se has no effect on the knock-down activity obtained by the transiently transfected plasmids under the conditions used in this example. As Figure 22 shows, there is no difference in knockdown effects between cells infected with virus and cells not infected with virus.

### Example 6: Viral knock-down

The reporter genes with the promoters of the plasmids pGL3-control, pGL3-tLet-7F, pGL3-tLet-7R, and pRL-TK have been recloned into the pIPspAdapt6-deltapolyA-construct in order to be able to generate adenoviral variants of the reporters. Standard recloning procedures are performed using the following restriction-sites: HindIII and BamHI for the fragments of pGL3-control, pGL3-tLet-7F, and pGL3-tLet-7R; BglII(blunt) and BamHI for the fragment of pRL-TK cloned in AvrII(blunt)/BamHI of the vector pIPspAdapt6.

The pIPspAdapt versions of the constructs are tested for functionality in a transient transfection experiment and appear to be active as a plasmid.

All the described pIPspAdapt-based reporter constructs are transiently transfected together with helper DNA into PER.C6/E2A. Adenoviruses are formed using the described procedure (US 6,340,595). Virus constructs are designated with 'Ad', e.g. AdGL3-tLet-7F is an adenovirus with the GL3 reporter gene and the Let-7 target sequence in forward orientation.

A co-infection experiment is performed with a combination of adenoviruses containing:
1) One of the luciferase reporter constructs (AdGL3-control, AdGL3-tLet-7F, AdGL3-tLet-7R), and
2) Internal control (AdRL-TK), and
3) U6 promoter-based expression construct (e.g. Ad-U6(+1)-L12-gLet-7)

In this experiment the infected cells are HeLa or U2OS.

The HeLa cells or U2OS cells are pre-infected at MOI 1500 for the luciferase reporter constructs together with the internal control (AdRL-TK) at MOI 500. After 4 hours the U6 promoter-based expression constructs are added at MOI 3000 or 8000. These viruses are crude lysates (US 6,340,595). The cells are harvested at 24, 48 and 72 hours post-infection. Northern blots are made to measure the expression level of the knock-down RNA molecules

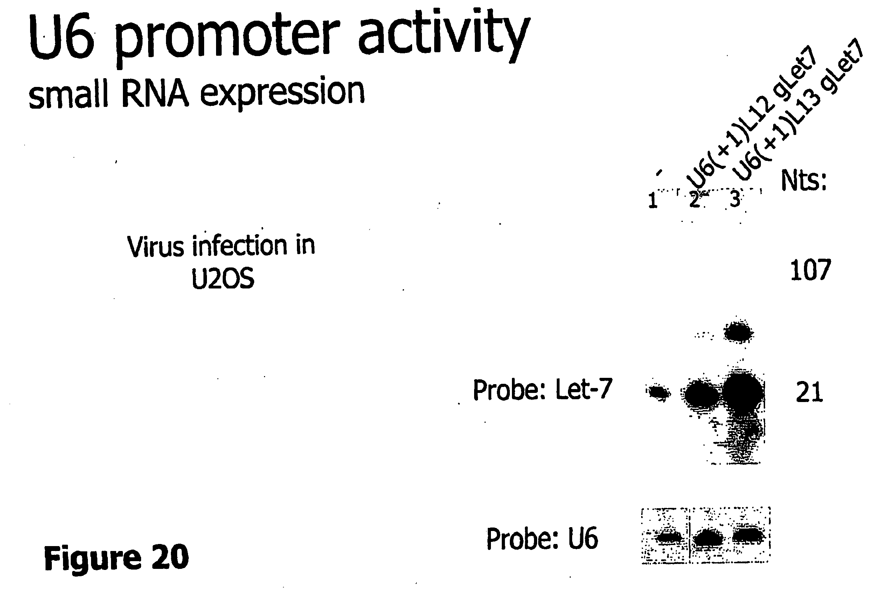

Northern blots of samples of cells infected with the adenoviral U6-based promoter expression constructs show expression of the RNA species and processing into a species of a size comparable to that of endogenous Let-7 RNA. (Figure 20).

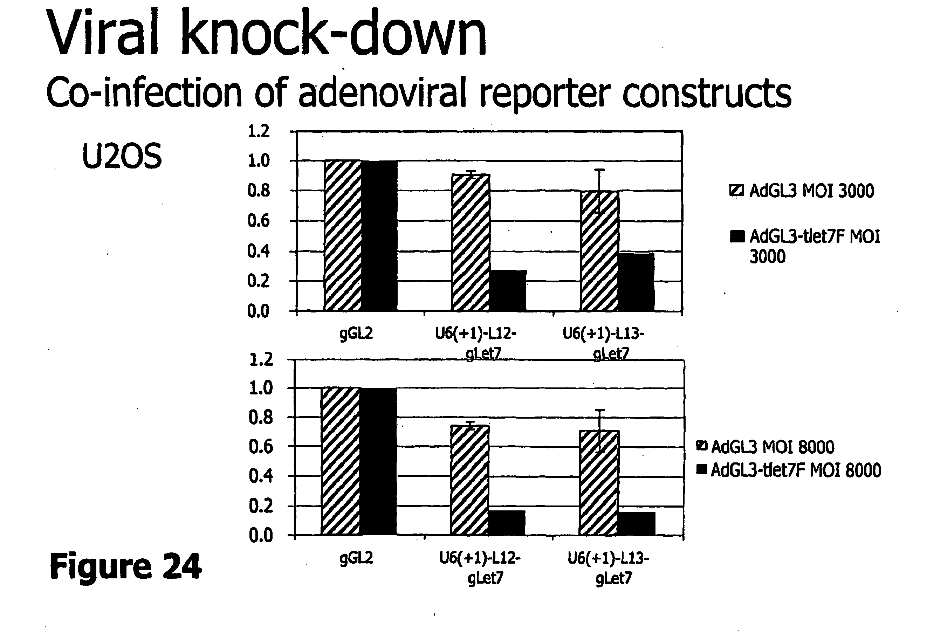

The luciferase activity is measured using the Dual Luciferase Kit as described above. The results are shown in Figure 24. Two U6-promoter based expression constructs with different loop sequences are tested. Both U6 promoter-based expression constructs show a clear sequence specific knock-down of the reporter virus AdGL3-tLet-7F that contains the Let7 target sequence. No significant repression is seen for AdGL3-virus lacking the let-7 target sequence. This clearly shows that U6-based viral expression constructs have knock-down activity.

Preferably, the expression construct contains a pol III promoter (more preferably an U6-based promoter), preferably followed by the 19-21 nucleotides guiding sequence (e.g. gGL3 or gLet7), preferably followed by a connecting loop sequence (e.g. L12 or L13), preferably followed by a 19-21 nucleotides sequence that is able to base pair with the guiding sequence at the RNA level, followed by a pol III transcription termination signal consisting preferably of 5 or more T residues.

In order to obtain transcriptional activity of the U6 promoter the first nucleotide of the transcript is preferably a G nucleotide. This G nucleotide can be the first nucleotide of the guide sequence. Preferably an internal sequence of more than 5 T- or A residues should be avoided since this will cause premature transcription termination by pol III. The loop sequence can be L12: 5'-GUUUGCUAUAAC-3' (SEQ ID NO: 30); or L13: 5'-GUUGUGCUAUCAAC-3' (SEQ ID NO: 66); or another connecting sequence (see for example the data described in Example 2). Preferably the sequence downstream of the connecting loop can base pair with the guiding sequence. The termination signal consisting of 5 U-residues at the 3' terminus in the transcript can be made partially complementary to the 5' terminus of the guiding sequence in such a manner that the transcript is predicted to fold into a duplexed structure having at the 3' terminus an overhang of 2-4 nucleotides. For example to create a 3' overhang of 2 nucleotides, the starting nucleotides of the guiding sequence have to be at position 1 (G), at position 2 (G or A), at position 3 (G or A) (see also above).

Alternatively, the sequence downstream of the connecting loop is made complementary to the guiding sequence. In this case the stretch of 3' terminal Us form the overhang.

The 19-21 nt guiding sequence is a sequence able to base pair to the target sequence in the target gene. In the RNA molecules described above, the order of the RNA sections is, going from 5' to 3': guide sequence, connecting loop, sequence able to base pair with the guide sequence. However this order can be reversed to produce (5' to 3'): sequence able to base pair with the guide sequence, connecting loop, guide sequence. Constructs based on GL2.2 guides in reverse order as described above show efficient expression and processing to RNAs of approx. 21 nts on Northern blots.

For knock-down of endogenous RNAs, a sequence of about 17 to about 22 nucleotides is selected corresponding to the target RNA. Multiple sequences from a target mRNA can be selected in order to identify a target sequence that is specific for a particular mRNA. Sequence alignments with other mRNA sequences that have high homology to the target mRNA are utilized to identify those regions of the target mRNA that are unique. Alternatively, if the goal is to knock-down the expression of more than one mRNA, a sequence alignment can be used to identify those regions of high homology to other genes. A target sequence selected from a region of high homology would knock-down all mRNAs comprising that sequence.

Here the GNAS gene (NM_000516) is taken as an example but is not limited to the invention as other RNAs can be knocked down using this method.

An U6-promoter based construct is generated containing a GNAS target sequence (e.g. 5'-CGATGTGACTGCCATCATC-3' (SEQ ID NO: 80). The generation of the construct can be performed e.g. by using PCR-based methods as described in example 4 or using direct cloning of oligonucleotides as in example 11 or any technique known to someone skilled in the art. From the resulting vector adenoviruses, Ad-U6(+1)-gGNAS, are obtained by methods as described previously (US 6,340,595, US 6,413,776). After introduction into vertebrate cells, RNA is transcribed from the vector with the following predicted sequence:
5'-GAUGAUGGCAGUCACAUCGGUUUGCUAUAACCGAUGUGACUGCCAUCAUC(U)₂₋₅-3' (SEQ ID NO: 81)

And the following predicted RNA folding:

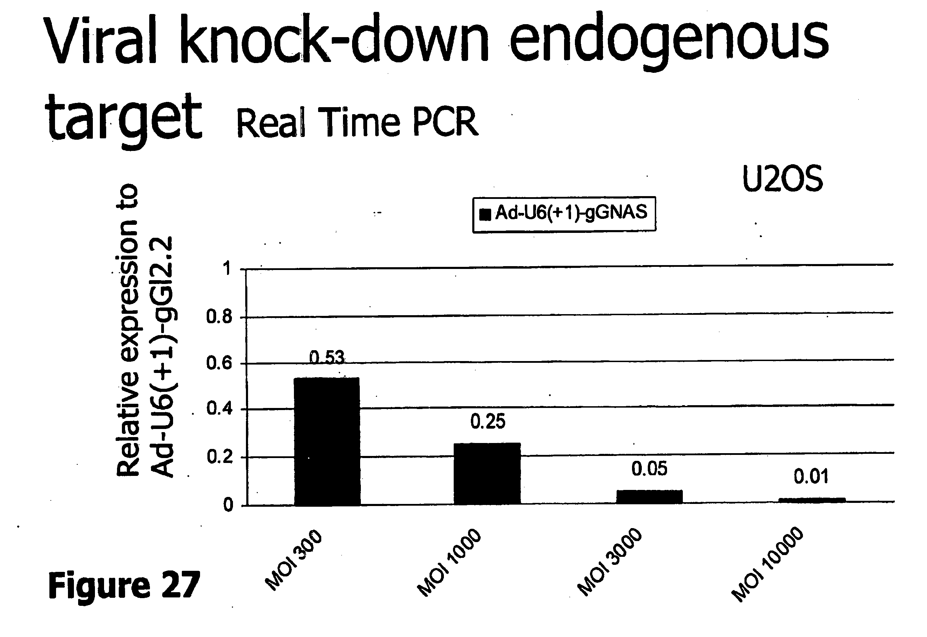

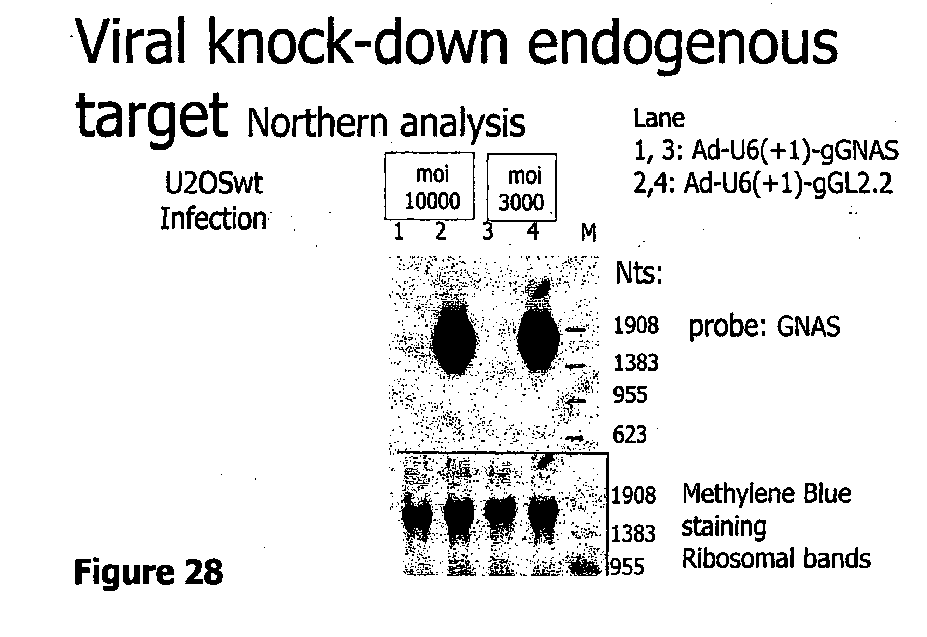

In order to demonstrate reduction of expression levels of the target RNA, a similar experiment is performed as described above, however we do not use a reporter virus. Briefly, for example HeLa cells or U20S cells are infected with the Ad-U6(+1)-gGNAS virus or the control virus Ad-U6(+1)-gGL2.2 with varying MOIs (MOI 300, 1000, 3000, 10000). After 72 hours post-infection, RNA was isolated from the infected cells. The expression levels of the GNAS mRNA are analyzed by real time PCR analysis as well as by Northern blotting (See figures 27 and 28).

For the real time PCR analysis, detection can performed e.g. by with SYBR Green or with Taqman probes. Here, SYBR Green detection is performed using standard techniques with the following primers:

| | |
|---|---|
| HPA-03 For | TCCCTCCCGAATTCTATGAGC (SEQ ID NO: 82) |
| HPA-03 Rev | GGCACAGTCAATCAGCTGGTAC (SEQ ID NO: 83) |

The results of the real time analysis are shown in Figure 27. The values are normalized to the internal GAPDH values of the samples to correct for variations in concentrations of the samples. The values are plotted relative to the control samples with Ad-U6(+1)-gGL2.2.

The analysis by Northern blotting is performed using standard techniques with a GNAS cDNA probe obtained by RT-PCR. The results are shown in Figure 28. Equal loading is checked by a methylene blue staining of the blot prior to hybridization as shown at the bottom of the figure.

The above experiments exemplify that endogenous genes can be efficiently knocked down by adenoviral knock-down constructs of the invention as described herein.

In a more extensive analysis, additional endogenous genes are selected to demonstrate sequence-specific reduction of the mRNA after viral infection with knock-down viral constructs according to the present invention. The selected genes and the selected sequences that are targeted are identified below.

More than one target sequence is selected for two of these genes.

Selected genes: IKK beta (AF080158), PPAR gamma (NM_005037), M6PR (NM_002355)
Selected target sequences:

| | | |
|---|---|---|
| IKK beta -1 | CUUAAAGCUGGUUCAUAUC | (SEQ ID NO: 84) |
| IKK beta -2 | AGAAUCAUCCAUCGGGAUC | (SEQ ID NO 85) |
| IKK beta -3 | CGUCGACUACUGGAGCUUC | (SEQ ID NO: 86) |
| PPAR gamma -2 | UACUGUUGACUUCUCCAGC | (SEQ ID NO: 87) |
| PPAR gamma -3 | UUGAACGACCAAGUAACUC | (SEQ ID NO: 88) |
| PPAR gamma -4 | AUUCAAGACAACCUGCUAC | (SEQ ID NO: 89) |
| M6PR-2 | GGAAGUAAUUGGAUCAUGC | (SEQ ID NO: 90) |

Adenoviruses are produced as described above. U2OS cells are infected with MOI 300, 1000 and 3000, and harvested after 24, 48 and 144 hrs post infection. The expression levels of the corresponding mRNAs are determined with real time PCR analysis as described above using the following primer combinations:
PPAR gamma (NM_005037) :

| | | |
|---|---|---|
| HPA-01 Rev | GGAGCGGGTGAAGACTCATG | (SEQ ID NO: 91) |
| HPA-01 For | ATTGTCACGGAACACGTGCA | (SEQ ID NO: 92) |

M6PR (NM_002355):

| | | |
|---|---|---|
| HPA-04 For | CAGTGGTGATGATCTCCTGCAA | (SEQ ID NO: 93) |
| HPA-04 Rev | TGCCACGCTCCTCAGACAC | (SEQ ID NO: 94) |

IKK beta (AF080158):

| | | |
|---|---|---|
| HPA-07 For | GCAGACCGACATTGTGGACTTAC | (SEQ ID NO: 95) |
| HPA-07 Rev | CCGTACCATTTCCTGACTGTCA | (SEQ ID NO: 96) |

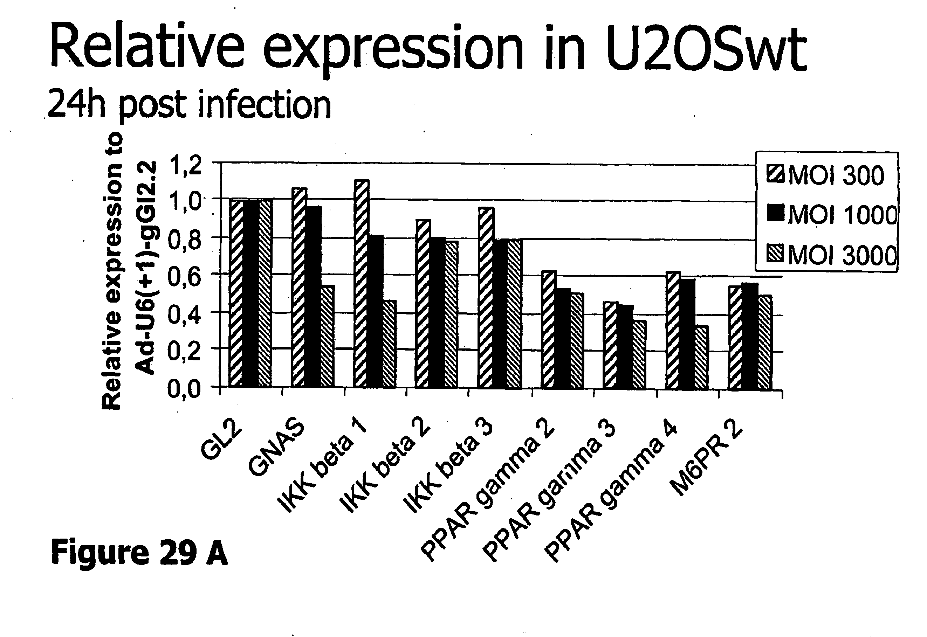

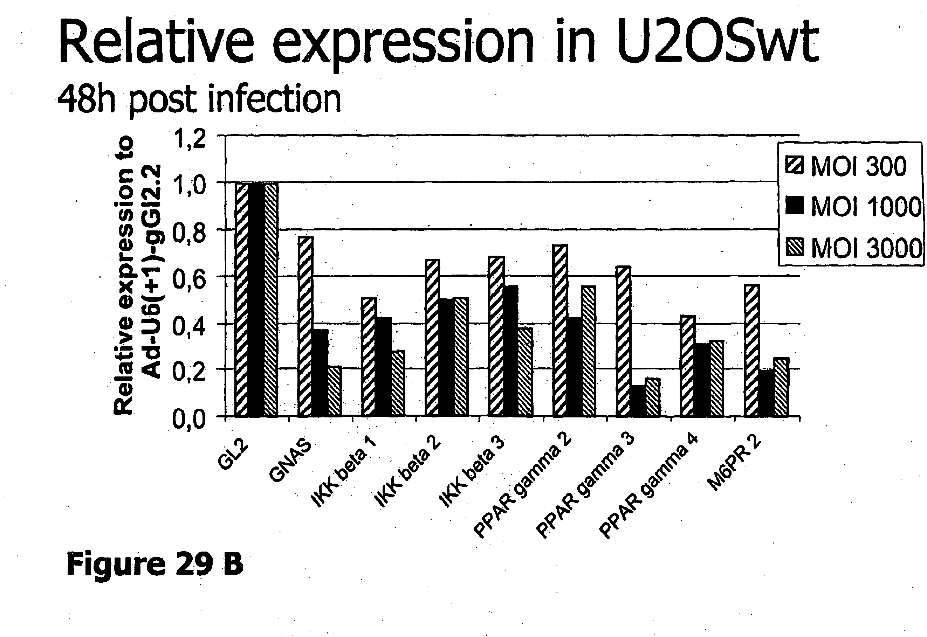

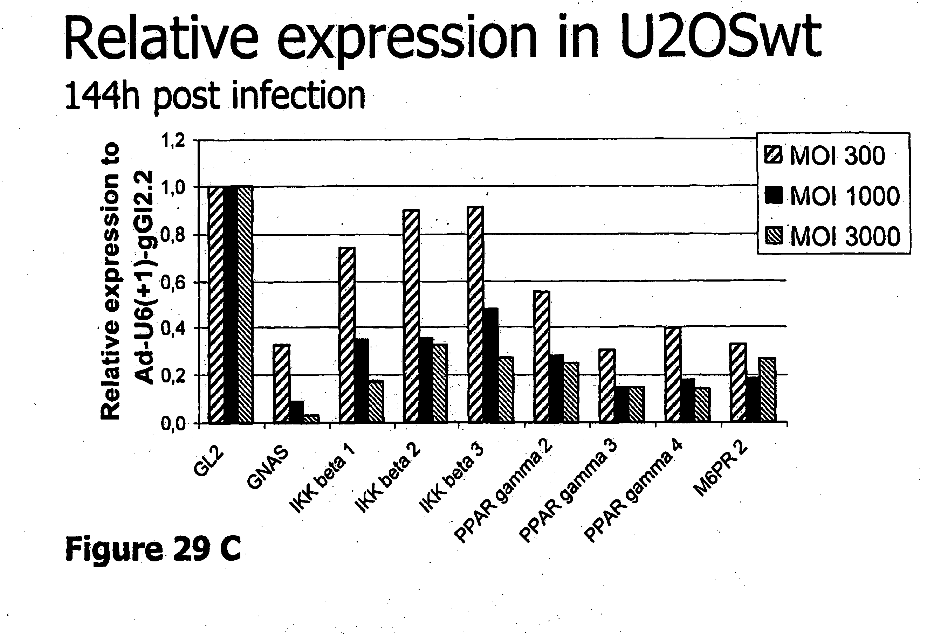

Figures 29 A-C show a specific reduction of the mRNA levels for the tested targets in a MOI and time dependent manner.

### GNAS:

GNAS encodes the Gα subunit of Gₛ, a heterotrimeric G-protein (α, β, γ). G proteins interact with 7 transmembrane receptors, the so-called G-protein coupled receptor (GPCR). Binding of a ligand to the GPCR induces a conformational change of the receptor that results in activation of the G-protein. The activated G protein will dissociate into its α subunit and the βγ subunit. In the case of Gαs the α subunit will interact with adenylate cyclase and in turn activate this enzyme. Adenylate cyclase converts ATP into cAMP. Activation of GPCRs that are coupled to Gₛ will therefore upon activation elevate the cellular cAMP levels. Variation in cAMP levels in the cell can be measured by cAMP responsive elements (CRE). cAMP responsive element activates transcription when bound by activated cAMP responsive element binding protein (CREBP). CREBP is activated by Protein Kinase A (PKA) that in turn is activated by cAMP. When the CRE is coupled to a luciferase gene, variations in cAMP levels in a cell can be visualized. An increase in cellular cAMP will result in an increase in the amount of luciferase and a decrease of cAMP results in a decrease of luciferase content.

The following assay is designed to measure the knock-down of GNAS at a functional level.

U20S cells are infected with an adenoviral construct encoding a GPCR (β2 adrenergic receptor) that couples to Gₛ (MOI 500) together with an adenoviral reporter construct carrying CRE elements upstream of a luciferase gene (MOI 750). The infected cells are co-infected with either an adenoviral construct encoding sRNA (SEQ ID NO: 81) targeted against GNAS or an empty virus (MOI 1500).

Two days post infection, the β2 adrenergic receptor is activated with isoproterenol, which is an agonist for the β2 adrenergic receptor. Six hours later the luciferase activity is measured.

After activation of the receptor, successful knock-down of GNAS results in a much lower cAMP levels compared with cAMP levels observed in the control cells transfected with empty virus.

To ensure that the decreased cAMP levels are due to the knock-down of GNAS and not due to a non-specific effect on cAMP levels, forskolin is added to the triply infected cells. Forskolin increases intracellular cAMP levels by direct activation of adenylate cyclase and therefore is independent of G-protein (GNAS).

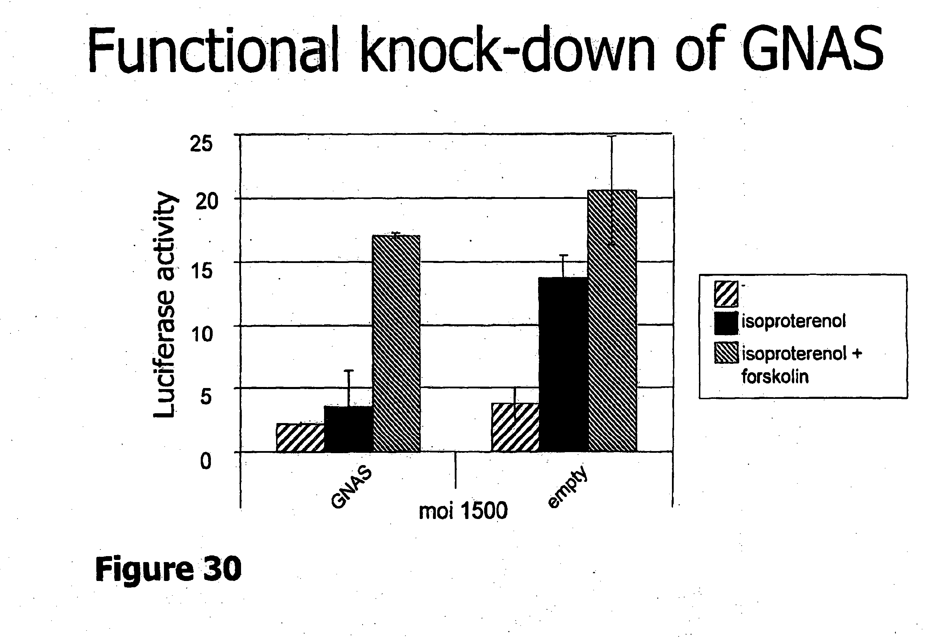

Figure 30 shows the results of the functional knock-down measurements of GNAS. The luciferase activity in cells infected with the adenoviral GNAS knock-down construct are much lower than the luciferase levels of the cells that are not infected with the GNAS knock-down construct. The forskolin results show that the cAMP reduction is due to the knock-down of GNAS rather then a general down-stream effect on the signal transduction route leading to the activation of CREBP.

### Example 7: Co-expression of human Dicer

This example describes the cloning and expression of human Dicer cDNA. Cloning of Dicer cDNA is accomplished using RT-PCR with mRNA as template. Dicer is co-expressed with the constructs of the previous examples thereby improving processing of RNA transcribed from the constructs. Co-expression of human Dicer is beneficial to the system, since it allows the system to be independent of endogenous Dicer activity that might be low or even absent in certain situations, tissues or developmental stages.

Biochemical and genetic data show the involvement of a member of the RNase III family of nucleases, Dicer, in the accurate processing of the double stranded RNA into the active siRNA species (Bernstein et al. (2001) Nature 409:363-366; Knight et al. (2001) Science 293:2269-71). The human homologue of Dicer functions to form approximately 22 nts RNAs from dsRNA substrates. Dicer is also required for the maturation of the let-7 sRNA in humans (Hutvagner et al. (2001) Science 293:834-838).

The human Dicer homologue is cloned using reverse transcriptase- PCR and primers based on the sequence Acc. Number NM_030621. Position relative to start of NM_030621 sequence.

| Primer | Sequence 5' to 3' | Position | SEQ ID NO |
|---|---|---|---|
| hDicer F1 | ccgAAGCTTGGGCATGCTGTGAT | 42-61 | SEQ ID NO: 67 |
| hDicer F2 | ccgaagcttATGAAAAGCCCTGCTTTGCAA | 183-203 | SEQ ID NO: 68 |
| hDicer F3 | ccgaagctTCCAGAGCTGGCTTATATCAG | 1592-1612 | SEQ ID NO: 69 |
| hDicer F4 | ccgaagcttACTGATTCTGCATATGAATGG | 4611-4631 | SEQ ID NO: 70 |
| hDicer R1 | ctgagctagcTTGTTAGAGCAACAGCCTAGA | 6960-6940 | SEQ ID NO: 71 |
| hDicer R2 | ctgagctagcTCAGCTATTGGGAACCTGAGGT | 5957-5936 | SEQ ID NO: 72 |
| hDicer R3 | ctgagctagcAATACACTGCTCAGTGTGCAA | 4850-4830 | SEQ ID NO: 73 |
| hDicer R4 | ctgagctagcGATTGAACATAGGATCGATAT | 1834-1814 | SEQ ID NO: 74 |

Due to the large size of the human Dicer cDNA, cloning can be accomplished by first isolating smaller regions of the cDNA. PCR fragments are subcloned and then ligated together in order to obtain the full-length human Dicer cDNA. Different primer combinations are used to generate different sized DNA fragments in order to facilitate cloning of the full-length cDNA. This avoids the problem of obtaining the long cDNA of Dicer. However, cloning in a single round using primers outside the coding region remains possible. The forward primers contain a Hind III site, and the reverse primers contain an Nhe I site for cloning purposes. The full-length cDNA is cloned into the Hind III and Nhe I sites of the pIPspAdapt6 vector. This vector is used for expression in mammalian cells by direct transfection or by making an adenoviral vector carrying the dicer cDNA and expressing it upon (co)infection.

The cloned Dicer cDNA expression plasmid is combined with constructs described in other examples and introduced into mammalian cells. The co-expression of constructs with Dicer cDNA or its homologues facilitates the processing of the transcribed sRNA.

### Example 8: Identification of 21-23 nts RNA sequences that are endogenously expressed and construction of a defined expression library of small RNAs

This example describes the identification and isolation of sRNAs.

Let-7 RNAs of 21-23 nts are conserved in a variety of animal species, including *Drosophila* and humans (Pasquinelli et al. (2000) Nature 408:86-89). Let-7 RNA is derived from a longer precursor RNA (Hutvagner et al. (2001) Science 293:834-838). It has been reported that upon incubation of long dsRNA in *Drosophila* extracts, 21-23 nts siRNAs are produced from the dsRNA. These 21-23 nts siRNAs have been isolated, cloned, and sequenced (Elbashir et al. (2001) Genes Dev 15:188-200). Novel endogenous 21-23 nts siRNAs have also been identified in *Drosophila* using this approach (Elbashir et al. (2001) Genes Dev 15:188-200).

The sRNAs belonging to the let-7 RNA-family are identified as follows. Sequences of human 21-23 nts-RNAs that are endogenous. (from different RNA sources; tissues, cell types, etc.) are obtained by cloning them by the method described for *Drosophila* (Elbashir et al. (2001) Genes Dev 15:188-200).

The obtained sequence information is used for the following aspects:
1. They are used to construct an expression library containing these sequences. The library is based, as described in the previous examples, on the expression of chimeric RNAs that have let-7 precursor segments (let-7 5' extension, let-7 loop, and let-7 3' extension sequences) combined with the 21-23 nt RNA sequences. The functions of these 21-23 nt RNAs and their target RNAs are studied by performing cellular assays.
2. The sequence identification of naturally occurring 21-23 nts RNAs facilitates the search for their genomic locations. Identification and characterization of the genomic sequences provides information regarding the production and processing of the members of the family of these sRNAs. In analogy to the let-7 RNA, the founding member of this family, new family member information is used for generating a chimeric RNA expressing system as explained for let-7 in previous examples. In addition, genomic sequence information is useful for optimizing the let-7 system described in previous examples.

### Example 9: Construction of an undefined expression library of sRNA species

This example describes the cloning of the sRNAs identified in Example 8. It is not necessary to know the sequence of the 21-23 nucleotide RNAs. The method generates a DNA fragment that consists of (in this order):
1. A 5' linker sequence to aid in cloning;
2. The 21-23 nucleotide sequence, which is preferably antisense to the target sequence;
3. A central linker (loop) sequence;
4. A sequence able to base pair with the 21-23 nucleotide sequence of 2 above; and
5. A 3' linker sequence to aid in cloning.

These DNA fragments are digested with restriction endonucleases and cloned into vectors. The preferred vectors are expression plasmids that contain let-7 or pol III promoters.

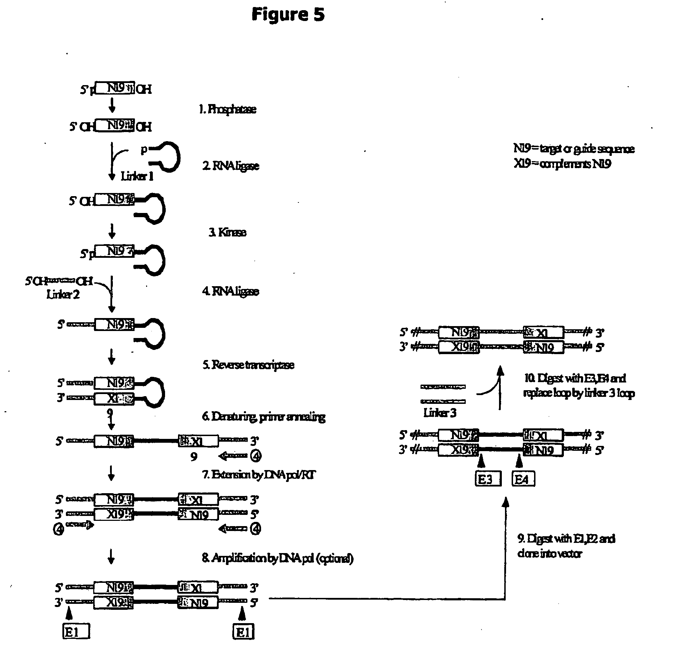

As described in the Example 8, endogenous sRNAs belonging to the family of let-7 RNA are identified by cloning and sequencing. In this example, the isolated sRNAs are cloned directly into an expression construct without first identifying their sequence (see Figure 5):
1. The sRNA is treated with Calf Intestinal Alkaline Phosphatase to remove the 5' phosphate group.
2. The product of step 1 is ligated at its 3' terminus to the 5' phosphate group of linker 1 to form an intermediate polynucleotide.
   Linker 1 is an oligonucleotide that can fold into a stem-loop structure and has a terminal 3' OH group that can be used as a primer. A preferred oligonucleotide contains a restriction site in the duplexed portion of the stem-loop. The presence of this restriction site facilitates the later excision and replacement of the linker sequence if necessary. The ligation is performed using T4 RNA ligase.
3. The product of step 2 is phosphorylated at its 5' terminus by T4 polynucleotide kinase.
4. The product of step 3 is ligated at its 5' phosphate terminus to the 3' OH terminus of linker 2. Linker 2 is an oligonucleotide having a 5' terminal OH group.
5. The product of step 4 is treated to allow the 3' terminus of linker 1 to anneal. This is used for an extension reaction using the 3' terminus of linker 1 as a primer with Reverse Transcriptase lacking the RNase H activity.
6. The product of step 5 is denatured and primer 4 is annealed. Primer 4 is complementary to the extreme 3' terminus of the product of step 5. Note: Linker 2 and primer 5 contain identical sequences.
7. The annealed primer 4 is extended by DNA polymerase or reverse transcriptase or a mixture of both.
8. An additional optional round of amplification is performed using primer 4. The amplification is performed by Klenow Fragment DNA polymerase, or T4 DNA polymerase, or a thermal stable DNA polymerase.
9. The product of step 7 (optionally step 8) is digested by restriction enzyme 1 and cloned into an expression vector with the appropriate site. The expression vector is based on the let-7 gene or pol III promoter based as described in previous examples and can be viral such as adenoviral or can be non-viral.
10. If the existing loop sequence (embedded in linker 1) is not functional to obtain the active sRNA species, this loop can be replaced by another loop sequence which gives rise to active sRNAs; e.g. the let-7 loop sequence. Cleavage sites are included in linker 1 to make this option possible.

### Example 10: Construction of an sRNA expression library directed against endogenous RNA targets

As it would be beneficial to be able to knock down expression of any gene, including currently unknown or uncharacterized genes, this example describes methods whereby different 19 nucleotide guide sequences are synthesized and cloned to make a library.

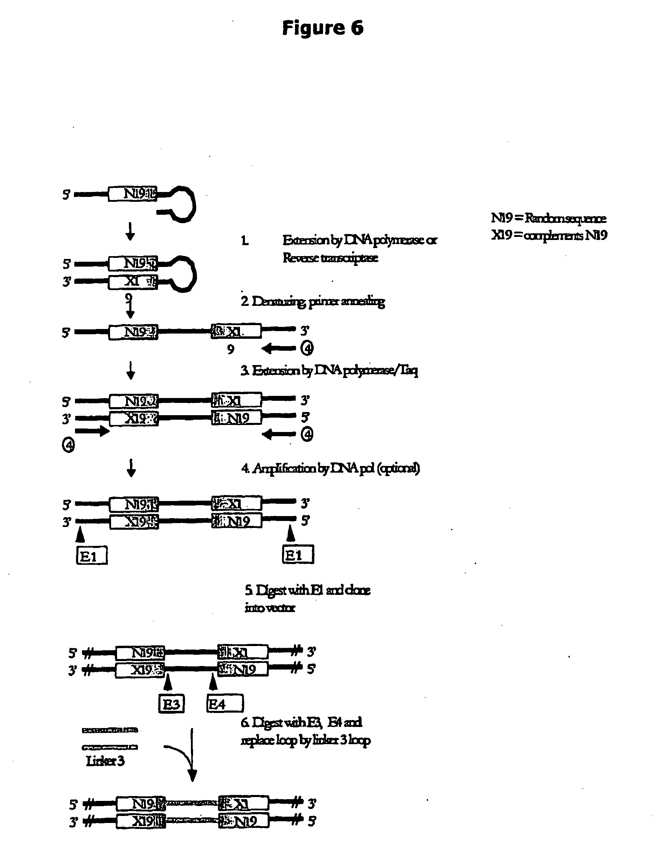

In analogy to Examples 8 and 9, an sRNA expression library is constructed. For example the sequences for the sRNA specifying their targets (mRNAs, structural RNAs, etc.) are obtained from databases, cellular extracts or chemically synthesized. For example, the sequences are obtained from databases containing expressed RNA sequences, including but not limited to mRNA, ESTs, SAGE, and may or may not encode proteins including but not limited to kinases, proteases, phosphodiesterases, phosphatases, G-protein coupled receptors, growth factors, lipases, ABC transporters, DNAses, RNAses etc. Also sequences from structural RNA sequences (e.g. snRNAs, rRNAs, tRNAs, snoRNAs, etc.) can be used. From these collections of oligonucleotides are produced containing these sequences. The mixture of the oligonucleotides is used to clone into the expression construct, using the strategy outlined in Figure 6. Another example is the random generation of 19 nt sequences that produces a library of 4¹⁹ combinations (2.7 x 10¹¹), which are similarly cloned into an expression construct in accordance with the procedure outlined in Figure 6. In step (1) of this procedure, an oligonucleotide is synthesized containing the specifying sequence (or random sequence), N19, flanked on the 5' end by a linker sequence and on the 3' end by a sequence that can fold into a stem-loop structure and has a terminal 3' OH group that can be used as a primer. The 3' terminus is used as a primer in an extension reaction with DNA polymerase or reverse transcriptase. Preferably, the duplexed portion of the stem-loop includes a restriction site. The 5' linker sequence could also have a restriction site in order to facilitate cloning.

In step (2), the product of step (1) is denatured and primer 4 is annealed. Primer 4 is complementary to the extreme 3' terminus of the product of step (1). The extreme 5' terminus of the initial oligonucleotide of step (1) and the primer 4 contain identical sequences. If the 5' linker sequence does not have a restriction site, primer 4 can be designed to have a restriction site in order to facilitate cloning.

In step (3) the annealed primer 4 is extended by DNA polymerase or a thermal stable DNA polymerase (e.g. Taq).

An optional step (4) provides for further amplification of the construct by a DNA polymerase.

In step (5), the product of step (3) or optionally step (4) is digested by restriction enzyme 1 (E1) and cloned into an expression vector with the appropriate restriction site. The preferred expression vector includes the let-7 or pol III promoter. The described method is especially suited for construction of pooled retroviral or lentiviral or AAV or integrating adenovirus sRNA expression libraries.

In optional step (6), if the loop sequence is not functional to obtain the active sRNA species, it can be replaced by another loop sequence which gives rise to active sRNAs; for example, the let-7 loop sequence. Restriction sites recognized by restriction enzymes 3 and 4 (E3 and E4 respectively) are included in the stem-loop sequence to permit replacement.

### Example 11: Production of an sRNA expression library

A preferred expression construct produces the siRNAs at levels with the strongest knock-down of the target RNA. For example, an expression construct based on the U6 promoter as described in previous examples.

Oligonucleotides can be designed directed against these targets and used for the construction of knock-down expression clones. Preferably, the forward (F) oligonucleotides contain the guiding sequences against the targets, followed by the connecting loop sequence, followed by the sequences that are able to basepair to the guiding sequences, however the order of guide sequence and complement can be switched. The reverse (R) oligonucleotides are complementary to the cognate forward oligonucleotide. A specific pair of forward and reverse oligonucleotides is annealed together forming a duplexed structure that is used for cloning into the expression vector.

The annealed F and R oligonucleotides either are cloned directly when designed with the appropriated overhangs matching these of the cloning vector, or they are digested with restriction enzymes prior to cloning to generate the appropriated overhangs matching these of the cloning vector.

The construction of a library directed against endogenous targets is preferably performed by using a DNA vector construct in which annealed oligonucleotides are cloned directly.

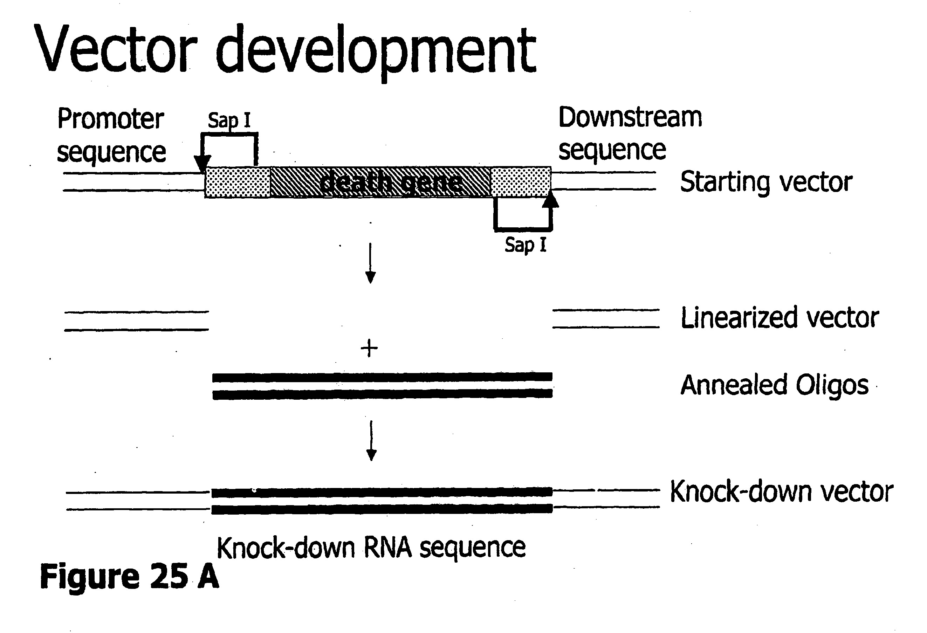

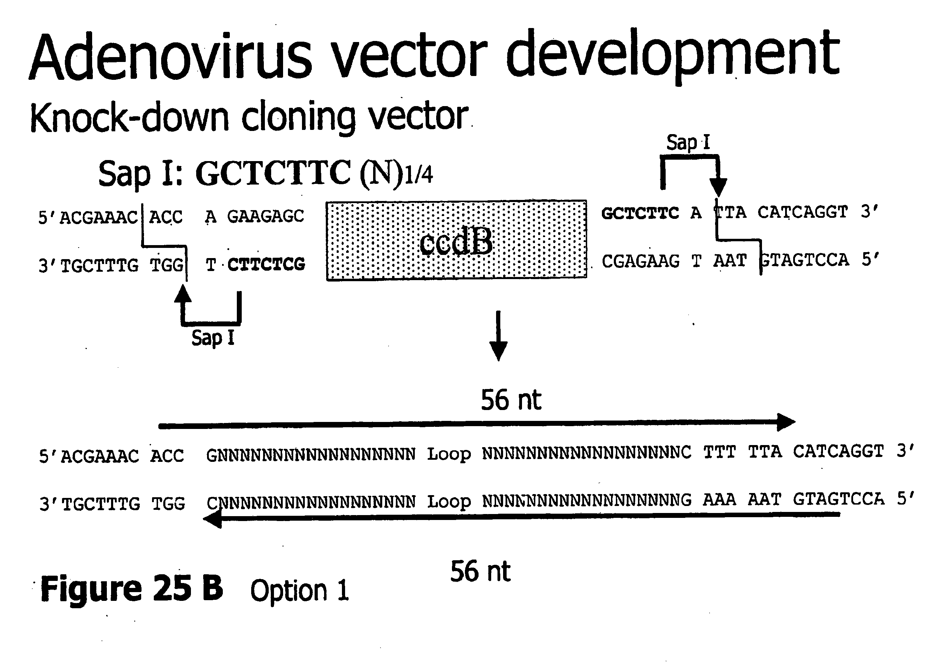

For example, to generate an expression construct based on the U6 promoter, a vector is created that is converted into a linear sequence with the enzyme Sap I. Sap I cuts adjacent to its recognition sites (GCTCTTC(N)_{1/4}). This has the advantage in that it cuts any sequence and that the recognition sequence is not present in the final construct since it is present on the excised fragment. As a consequence, any sequence can be chosen to linearize the vector. Therefore it is used for the construction of expression plasmids based on various promoters, without disturbing the sequence. Preferably, the U6 promoter is used to create both cloning sites with different overhangs. In addition, to improve cloning efficiency the e. coli lethal gene, ccdB, is included in the fragment to be excised. When the restriction fragment is not excised and the ccdB gene remains in the plasmid, after transfection no e. coli colonies are formed (Figure 25A). Only e. coli containing correct expression plasmids will form colonies. The sequences of the junctions are schematically given in Figure 25B.

The annealed oligonucleotides have the following sequence to enable direct cloning into the U6 promoter based vector after Sap I digestion as discussed above:
Forward oligonucleotide:
   5'-ACC-G-N18- -loop -N18*-C-TTT-3'
Reverse oligonucleotide:
   3'-C-N18*-loop*-N18 -GAAAAAT-5'

When using a pol III promoter, to facilitate transcription the first nucleotide is preferably a G-nucleotide. Therefore, in a preferred embodiment, the 19 nt guiding sequence is G-N18. N18*-C is a sequence able to base-pair to the 19 nt guiding sequence (G-N18), loop is the connecting loop sequence, loop* is the complement of loop. See also Figure 25B.

The oligonucleotides are designed such that they are even smaller, i.e. 51 nt instead of 56 nucleotides:
Forward oligonucleotide:
   5'-CC-G-N18- -loop -N18*-3'
Reverse oligonucleotide:
   3'-N18*-loop*-N18 -GAA-5'

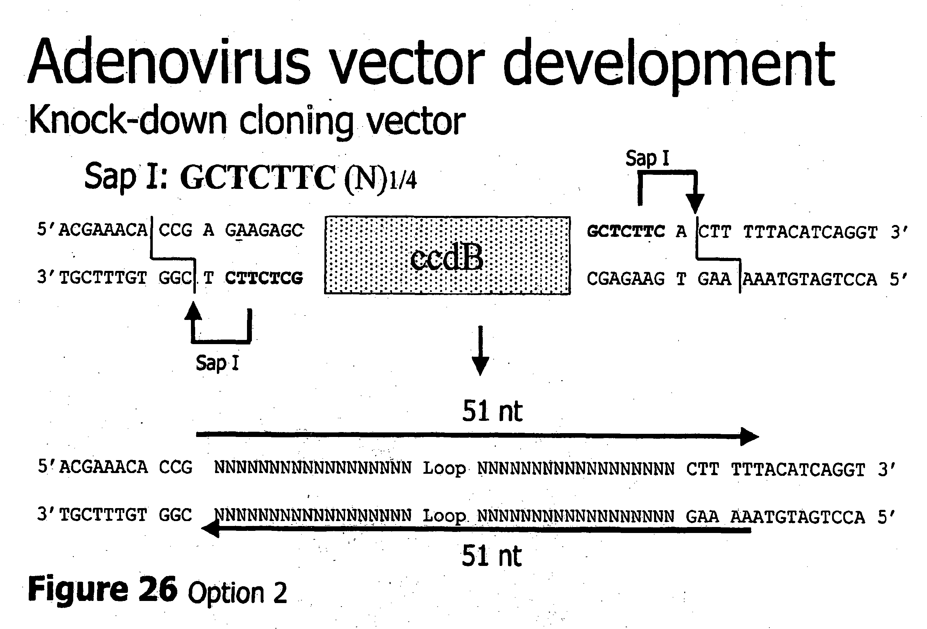

The expression vector has to be adjusted accordingly. See Figure 26

The present invention can also be used to generate random target sequences to produce an undefined library. The target sequences are not known, however they preferably start with a G to facilitate translation by Pol III. These oligonucleotides can produce a library with 4¹⁸ combinations (6.87 10¹⁰).

The non-viral DNA expression libraries generated as described above can be used in disease relevant cellular assays to screen for knockdown-induced phenotypes by transfecting the DNAs as arrayed collections using DNA transfer methods known in the art, such as lipofectamine or PEI. These libraries can also be used to generate collections of cell lines with individual or multiple genes stably knocked down by including a selectable marker in the expression plasmid prior to making the library in said vector.

The individual knockdown constructs can be pooled to various degrees, that is in one large pool of constructs, or subsets of such pool. The sets and subsets of pools can then be used in disease relevant assays where the phenotype is a selectable phenotype such as a flow cytometric phenotype (e.g. induction or repression of marker) or growth advantage phenotype.

### Example 12. Arrayed adenoviral library construction

This example describes construction of an arrayed adenoviral sRNA expression library. "Arrayed adenoviral sRNA expression library" refers to a collection of adenoviruses (contained in multiwell plates, for example 96 or 384 well plates) mediating the expression of various (human) sRNAs, in which every well contains a unique recombinant virus carrying a sRNA expression construct targeted against a gene, i.e. one target gene per well. Further details about the concept of arrayed adenoviral libraries can be found in WO 99/64582, US 6,340,595 and 6,413,776 (Arrayed adenoviral libraries for performing functional genomics). Arrayed adenoviral libraries expressing sRNAs that down-regulate specific endogenous mRNAs can be used to infect cells including disease relevant human primary cell types such as HUVECs. These assays may be used to screen for genes that specifically inhibit a metabolic pathway, such as the NfkappaB pathway, by an expressed sRNA, and thereby inhibit the up-regulation of a cytokine, such as IL8.

### A. Construction of Adenoviral Arrayed sRNA Library

Oligonucleotides are designed to be targeted against specific mRNAs transcribed from specific genes and used for the construction of knock-down adenoviral expression clones. The preferred oligonucleotides are designed such that the first sequence (forward (F)) contains the guide sequences against the targets, covalently linked to the stem-loop second sequence, which is covalently linked to the third sequence that is able to base-pair with the first sequence. Another embodiment reverses the guide function resulting in the third sequence as the guide sequence. The reverse (R) oligonucleotides are complementary to the cognate forward oligonucleotides. Specific pairs of forward and reverse oligonucleotides are annealed together forming a duplexed structure that is used for cloning into an expression vector based on pIPspAdapt6 (described in WO 99/64582) containing a promoter, for instance the polIII-dependant let-7 or U6 promoter. The expression cassette may further contain (a) unique restriction enzyme recognition sequence(s) to directionally ligate the annealed F and R oligonucleotides that encode for the sRNA sequences against an endogenous mRNA. The single stranded oligonucleotide components may be synthesized and annealed in 96 or 384 well plates to generate the double stranded oligonucleotides.

The array of sRNA Adapter plasmids is then, again in an arrayed format, transformed into host microorganisms, such as bacteria, preferably *Escherichia coli* (strain DH10B) or strains better suited for propagation of constructs containing adenoviral ITRs. Transformed bacteria can be plated and grown on 6 well LB-ampicillin plates, after which a colony picker picks individual bacterial colonies and further inoculates liquid LB growth medium (+ 100 µg/ml ampicillin) in 96 well plates.

The preferred method of this invention is to start the liquid cultures directly from the 96 well E. coli transformation plates without plating and picking individual colonies. In both cases the resulting 96 well plates are incubated in a rotary shaker (New Brunswick Scientific, Innova, floor model or equivalent) at 37°C, 300 rpm. After culturing bacteria for about 12 to about 24 hours, about 100 µl of bacterial cultures are then mixed with 100 µl of 50% glycerol using a Multimek robot (Beckman Coulter) or equivalent and stored at -80°C. These plates are defined as 'glycerol stock plates'.

### B Preparation of plasmid DNA

A second step in the construction of the adenoviral sRNA expression library is the arrayed purification of DNA of individual plasmids from the primary library in amounts sufficient for adenovirus generation. For this purpose, a bacterial culture is prepared as follows. The glycerol stock plates are thawed and 3 µl of the bacterial culture is transferred to a 96 well plate filled with 280 µl of liquid LB growth medium (+ 100 µg/ml ampicillin) using a CybiWell robot (CyBio). These inoculated plates are then incubated for 18 hrs in a rotary shaker (37°C, 300rpm) (New Brunswick Scientific, Innova, floor model). Centrifugation of the 96 well plates (3 min, 2700 rcf) is performed to pellet the bacteria. All centrifugations of 96 well plates are performed in an Eppendorf microtiterplate centrifuge (type 5810). The supernatant is then removed by decanting into a waste container. The lysis of bacterial cells and precipitation of proteins and genomic DNA is performed by applying the classical alkaline lysis protocol. The buffers used to perform alkaline lysis are purchased from Qiagen (P1, P2, P3). In a first step, the bacterial pellet is resuspended into 60 µl of buffer P1. In a second step, 60 µl of buffer P2 is added to the resuspended bacterial cells. The solution is gently mixed and a 5 min incubation time is applied to achieve complete cell lysis. Finally, 60 µl of buffer P3 is added and a mixing step applied for precipitation of proteins and genomic DNA. The 96 well plates are then centrifuged (40 min, 3220 rcf). The supernatant (100 µl) is collected and transferred to new V-bottom 96 well plates containing 80 µl of isopropanol (for precipitation of the plasmid DNA) using a CybiWell robot (CyBio). The plates containing the pellet are discarded. The 96 well plates are centrifuged (45 min, 2700 rcf) and the supernatant discarded by decanting in a waste container. To remove salt traces, the pellet is washed with 100 µl of 70% ethanol and the 96 well plates are centrifuged again (10 min, 2700 rcf). The supernatant is removed again by decanting in a waste container and the DNA pellets are allowed to dry for 1 h in a laminar airflow cabinet. Finally, the DNA is dissolved in 20 µl of sterile TE buffer (1 mM Tris-HCl (pH7.6), 0.1mM EDTA). The integrity of the constructs is assessed by restriction enzyme mapping and sequencing of either the whole array or parts thereof. Plates containing the dissolved DNA (further defined as DNA plates) are stored at - 20°C until further use.

### C. DNA quantification

Before use for transfection of PER.C6/E2A cells, the plasmid DNA preparations contained in 96 well plates are quantified. For this purpose, 5 µl of plasmid DNA is pipetted from the DNA plates and transferred to a 96 well plate containing 100 µl of TE buffer. 100 µl of 'quantification solution' is added. This solution is prepared by dissolving 2 µl of SybrGreen (Molecular Probes) into 10 ml of TE Buffer. After a mixing step, measurement is performed in a Fluorimeter (Fluostar, BMG) with the following settings: emission: 485 nm; excitation: 520 nm, gain: 35. A standard curve is generated by performing a measurement using different dilutions (in TE buffer) of a standard DNA sample (lambda DNA). By fitting results for the individual DNA samples on this curve, DNA concentration per well is calculated. The mean DNA concentration per well for each DNA plate is then calculated. On average, a DNA concentration of 20-100 ng/µl of DNA is obtained.

### D. Transfection of Per.C6/E2A cells

The pIPspAdApt6 plasmid versions contain the 5' part (bp 1-454 and bp 3511-6093) of the adenovirus serotype 5 genome in which the E1 gene is deleted and a promoter (let-7- or pol III-derived) is introduced. In contrast, to the plasmid pIPspAdApt6, the sRNA expression derivatives of pIPspAdapt lack the CMV promoter and the SV40 polyadenylation site. Two other materials needed for the generation of recombinant adenovirus particles are a cosmid and a packaging cell line (WO99/64582. US 6,340,595). The cosmid (pWE/Ad.AflII-rITRΔE2A) contains the main part of the adenovirus serotype 5 genome (bp 3534-35953) from which the E2A gene is deleted. The Per.C6/E2A packaging cell line is derived from human embryonic retina cells (HER) transfected with plasmids mediating the expression of the E1 and E2A genes. The adenoviral genes that are integrated into the genome of the PER.C6/E2A cell line share no homology with the adenoviral sequences on the adapter plasmid and the cosmid. Consequently, vector stocks that are free of replication competent adenoviruses (RCAs) are prepared.

To obtain viruses, this adapter plasmid is co-transfected with the cosmid into a packaging cell line PER.C6/E2A. Once the adapter and helper plasmids are transfected into the PER.C6/E2A cell line, the complete Ad5 genome is reconstituted by homologous recombination. The helper and adapter plasmids contain homologous sequences (bp 3535-6093), which are a substrate for this recombination event. The DNA plates, which are prepared and quantified as described above, are used for transfection of the PER.C6/E2A cell line. Prior to this transfection, the plasmids contained in these plates are linearized by digestion with the PI-PspI restriction enzyme (New England Biolabs). For this purpose, a certain volume of plasmid DNA (representing 66.7 ng of DNA on average, as calculated from the average DNA concentration of each DNA plate) is pipetted from the DNA plates into a V-bottom 96 well plate containing a restriction mix composed of 1X restriction buffer (New England Biolabs: 10mM Tris-HCl (pH 8.6), 10mM MgCl₂, 150 mM KCl, 1 mM DTT), 100µg/ml BSA and 6 units of PI-PspI restriction enzyme (from a stock of 20 U/µl). For each DNA plate, an identical volume of plasmid is used for all wells. Transfer of the DNA samples from the DNA plate to the plate containing the restriction mix and subsequent mixing is performed with a JoBi Well robot (CyBio). The plates containing the restriction mix are then put in plastic boxes containing humidified paper towels (to avoid evaporation) and incubated at 65°C for 4 hrs. The helper plasmid (pWE/Ad.AflII-rITRΔE2A) (which is prepared in batch using the Qiagen Maxi-prep kits) is also linearized with the PacI restriction enzyme (New England Biolabs).

The transfection of the PER.C6/E2A cells with the linearized adapter and helper plasmids is set up as described below. 0.1867 µl of linearized helper plasmid (containing 93 ng of DNA) is mixed with 1.11 µl of serum free 2XDMEM (Life Technologies) to form a "helper mix". 0.597 µl of Lipofectamine (Life Technologies) is mixed to 1.11 µl of 2XDMEM to form a "lipo mix". In each well of 96 well plates containing the linearized adapter plasmids, 1.3 µl of "helper mix" and 1.7 µl "Lipo mix" are pipetted using a CyBi-Well robot (CyBio, equipped with a 'dropper'). The plates are then incubated for approximately 1 hour at room temperature before addition of 28.5 µl of serum-free DMEM. Mixing is performed by pipetting the mixture up and down three times (CyBi Well robot). Using the same device, 30 µl of the mix is transferred to 96 well plates containing PER.C6/E2A cells seeded at a density of 2.25x10⁴ cells/well. Cells are seeded into 100 µl of PER.C6/E2A medium (composed of DMEM (Life Technologies) containing 10% FBS (Life Technologies), 50 µg/ml gentamycin and 10 mM MgCl₂), but prior to addition of the 30 µl of the DNA/Lipofectamine mix, the medium is removed from all wells of the plates. An incubation time of three hours at 39°C, 10% CO₂ is then applied. 170 µl of PER.C6/E2A medium is then added to the plates and an overnight incubation at 39°C, 10% CO₂ is applied. The 96 well plates containing the transfected PER.C6/E2A cells are incubated at 34°C, 10% CO₂ during three weeks. This temperature allows the expression of the E2A factor, which is required for adenoviral replication. During this incubation time, viruses are generated and replicated, as revealed by the appearance of CPE (cytopathic effect). The percentage of the wells showing CPE is scored manually or using an automated image scanner and appropriate algorithm, which allows for the evaluation of the efficiency of virus production. The 96 well plates are stored at -80°C until further propagation of the viruses.

### E. Virus propagation

The final virus propagation step is aimed at obtaining a higher percentage of wells showing CPE and more homogenous virus titers. Viruses are propagated according to the following procedure. The transfection plates stored at -80°C are thawed at room temperature for about 1 hour. By means of a 96 channel Hydra dispenser (Robbins), 20 µl of the supernatant is transferred onto PER.C6/E2A cells seeded in 96 well plates at a density of 2.25x10⁴ cells/well in 180 µl of DMEM + 10% FBS. After handling of one series of 96 viruses, the needles of the dispenser are disinfected and sterilized by pipetting up 60 µl of 5% bleach three times. The traces of bleach present in the needles are removed by three successive washes with 70 µl of sterile water. Cells are incubated at 34°C, 10% CO₂ for approximately 10 days and the number of wells showing CPE is scored. In general, the number of wells showing CPE is increased after propagation. The plates are then stored at -80°C.

From the 200 µl of crude cell lysate containing the library viruses after the propagation step, six aliquots of 25 µl are prepared in 384 well plates using a 96-channel Hydra dispenser. From four 96 well plates, six identical 384 well aliquot plates are prepared. Disinfection of the needles in between the individual plates is achieved by a triple washing step with 200 µl 5% bleach and a triple washing step with 250 µl sterile water to remove bleach traces. The 384 well aliquot plates are then stored at -80°C until further use in an assay.

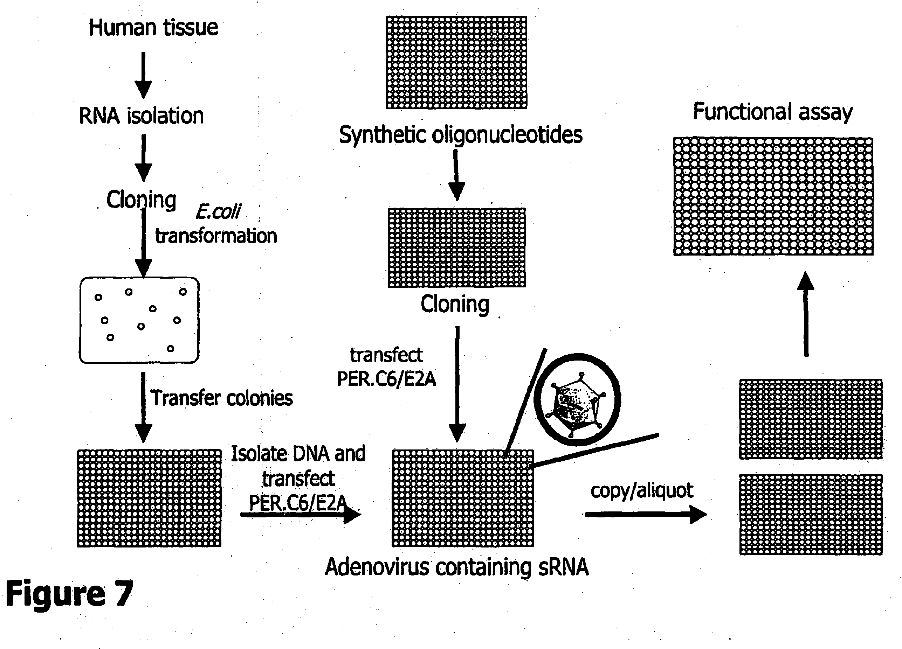

A schematic representation of the library construction is shown in Figure 7.

In addition, modifications to the viral coat proteins can be introduced to obtain a different or improved tropism (EP 1191105).

The arrayed adenoviral libraries described above can be used in disease relevant cellular assays to screen for knockdown induced cellular phenotypes. Such libraries are particularly useful when the adenoviral vector constructs are integrating adenoviral vectors, such as described in US 6,051,430 or WO 99/32647/EP 1042494 and Virology 2001 Sep 30; 288(2): 236-46. Such adenoviral sRNA expression libraries result in a library of cell lines in which individual or multiple genes are stably knocked down. The individual knockdown adenoviruses can be used as arrays but also can be pooled to various degrees i.e. sets of pools or one large pool. The pools can then be used in disease relevant assays including assays with a selectable phenotype such as a flow cytometric phenotype (screening for up-regulation or down regulation of a suitable marker by using, for example, fluorescence-labeled antibodies) or growth advantage phenotype.

### Example 13. Arrayed AAV library construction

This example describes construction of an arrayed AAV sRNA expression library "comprising a collection of adenovirus associated virus (AAV) vectors, that are contained in multiwell plates, for example 96 or 384 well plates, and that mediate the expression of various unique (human) sRNAs. Each well contains a unique recombinant AAV vector carrying an sRNA expression construct targeted against a specific gene. Arrayed AAV libraries expressing sRNAs that down regulate specific endogenous mRNAs can be used to infect cells including disease relevant human primary cell types such as HUVECs, for example to screen for genes that specifically inhibit a metabolic pathway, such as the NfkappaB pathway, by an expressed sRNA, and thereby down-regulate production of one or more cytokines, such as IL8.

### A. Construction of the AAV based sRNA library

Oligonucleotides are designed to be targeted against specific mRNAs transcribed from specific genes and used for the construction of knock-down AAV expression clones. The preferred oligonucleotides are designed as described above (Example 12). Specific pairs of forward and reverse oligonucleotides are annealed together forming a duplexed structure that is used for cloning into an AAV expression vector consisting of the AAV ITR sequences, an AAV packaging signal (see US 6,140,103) and a promoter, for instance the polIII-dependant let-7 or U6 promoter. The expression cassette may further contain (a) unique restriction enzyme recognition sequence(s) to directionally ligate the annealed F and R oligonucleotides

The array of sRNA-AAV ligations is then, again in an arrayed format, transformed into host microorganisms, such as bacteria, preferably *Escherichia coli* (strain DH10B) or strains better suited for propagation of constructs containing AAV ITRs. Transformed bacteria can be plated and grown on 6 well LB-ampicillin plates, after which a colony picker picks individual bacterial colonies and further inoculates liquid LB growth medium (+ 100 µg/ml ampicillin) in 96 well plates followed by plasmid DNA isolation.

The preferred method of this invention is to start the liquid cultures directly from the 96 well E. coli transformation plates without plating and picking individual colonies. In both cases the resulting 96 well plates are incubated in a rotary shaker (New Brunswick Scientific, Innova, floor model or equivalent) at 37°C, 300 rpm. After culturing bacteria for about 12 to about 24 hours, about 100 µl of bacterial cultures are then mixed with 100 µl of 50% glycerol using a Multimek robot (Beckman Coulter) or equivalent and stored at -80°C. These plates are defined as 'glycerol stock plates'. From these 'glycerol stock plates', plasmid DNA can be prepared and quantified as described in Example 12.

### B. Transfection of cells to generate AAV virions in arrayed format

For generation of arrayed AAV collections, AAV virions are produced from the arrayed sRNA-AAV plasmids DNAs described under A, using an AAV packaging cell line or a transient AAV packaging system. An AAV packaging cell line or derivative thereof, as described in US 6,140,103 may be used. Alternatively transient co-transfection of arrayed sRNA-AAV DNA with rep-cap expression constructs without a packaging signal or any other overlap with the sRNA-AAV constructs can be performed on cells infected with psoralene treated adenovirus to provide in trans the necessary helper genes of the helper adenovirus. For both approaches, the cell lines to be transfected are seeded in 96 well plates. Virus production, propagation and titering can be done with methods known to the art of AAV vector technology. After production of AAV sRNA-viruses in 96 well plates they can be re-aliquoted using a liquid handler into 384 well aliquot plates and stored at - 80°C until further use.

The arrayed AAV libraries described above can be pooled to various degrees, that is in one large pool of retroviruses, or subsets of such retrovirus pool. The sets and subsets of pools can be used in disease relevant cellular assays to screen for knock-down induced phenotypes. Also these AAV sRNA expression libraries can be used to generate collections of cell lines with individual or multiple genes that are stably knocked-down.

### Example 14

### Retroviral libraries expressing sRNA

In this example, the Moloney Murine Leukemia Virus-based retroviral plasmid LZRSpBMN-LacZ, is used (Kinsella TM and Nolan GP (1996). Episomal vectors rapidly and stably produce high-titer recombinant retrovirus. Hum. Gene Ther. 7:1405-13).

### A. Construction of the retrovirus-based sRNA library

In LZRSpBMN-LacZ, the LacZ gene replaces the env-pol sequences. Furthermore, the EBV EBNA-1 gene and EBV oriP origin of replication are present in the plasmid backbone. These allow episomal replication of the plasmid in the packaging cells. In this way, high titer supernatant can be generated after transient transfection of the packaging cells. Transfected cells can be selected since a puromycine resistance gene, under control of the Phosphoglycerol Kinase-1 promoter, is also present in the plasmid backbone. Culturing of the transfected packaging cells in the presence of puromycine usually generates high titer supernatant.

To express the chimeric RNA's as described above, the LZRSpBMN-LacZ vector is further modified by replacing the LacZ gene for an expression cassette. This expression cassette contains an ubiquitously active promoter, in particular, a promoter recognized by RNA polymerase III (pol III promoter) including but not limited to U6 and let7 promoters. The expression cassette can be cloned in either orientation at different positions within the viral backbone of the retroviral vector, as is well known to those skilled in the art (see for example, Medina MF, Joshi S. RNA-polymerase III-driven expression cassettes in human gene therapy. Curr Opin Mol Ther 1999 Oct; 1(5): 580-94. The expression cassette further contains (a) unique restriction enzyme recognition sequence(s) (see example 10) to directionally ligate and clone the annealed F and R oligonucleotides that encode the sRNA against an endogenous mRNA.

Oligonucleotides are designed as described above. Specific pairs of forward and reverse oligonucleotides are annealed together forming a duplexed structure that is used for cloning into a modified LZRSpBMN-LacZ vector or equivalent. The single stranded oligonucleotides components can be synthesized and annealed in 96 or 384 well plates to generate the double stranded oligonucleotides.

The arrayed library of retroviral plasmid-sRNA ligations is then, again in an arrayed format, transformed into host microorganisms, such as bacteria, preferably *Escherichia coli* (strain DH10B) or strains better suited for propagation of such constructs. Transformed bacteria can be plated and grown on 6 well LB-ampicillin plates, after which a colony picker picks individual bacterial colonies and further inoculates 300 µl of liquid LB growth medium (+ 100 µg/ml ampicillin) in 96 well plates followed by plasmid DNA isolation.

The preferred method of this invention is to start the liquid cultures directly from the 96 well E. coli transformation plates without plating and picking individual colonies. In both cases the resulting 96 well plates are incubated in a rotary shaker (New Brunswick Scientific, Innova, floor model or equivalent) at 37°C, 300 rpm. After culturing bacteria for about 12 to about 24 hours, about 100 µl of bacterial cultures are then mixed with 100 µl of 50% glycerol using a Multimek robot (Beckman Coulter) or equivalent and stored at -80°C. These plates are defined as 'glycerol stock plates'. From these 'glycerol stock plates', plasmid DNA can be prepared and quantified as described in Example 12.

### B. Transfection and virus collection

A suitable packaging cell line is subsequently used to generate an arrayed retroviral library from the purified retroviral plasmid-sRNA constructs. A suitable packaging cell line is provided by the Phoenix (ΦNX) packaging cells (Nolan GP and Shatzman AR (1998), Expression vectors and delivery systems, Curr. Opin. Biotechnol. 9: 447-450), but other packaging cells might be equally suitable. The Phoenix cells express the gag /pol and env proteins from two different constructs, thus minimizing the chance to generate replication competent retroviruses. Furthermore, the presence of a selection marker on each of these constructs allows the selection of cells that have retained them.

Ecotrophic packaging cells (ΦNX-E) produce retrovirus that can infect only rodent cells, while viruses produced in amphotrophic (ΦNX-A) packaging cells can infect cells from different origins. ΦNX cells do not adhere very well onto the tissue petri dishes. Care must be taken that trypsinisation is as short as possible and that the cells do not grow under confluent or superconfluent conditions.

Transfection of packaging cells is routinely performed using standard calcium phosphate co-precipitation, but other methods might be equally suited such as lipofectamine and PEI (also see WO 99/64582, US 6,340,595 and US 6,413,776). For this, the cell line is seeded in 96 well plates. Virus production, propagation and titering may be accomplished with methods known in the art of retroviral vector technology. After production of retroviral-sRNA viruses in 96 well plates, the transfected cells can be aliquoted using a liquid handler into 384 well aliquot plates.

To isolate the viral particles, collection of the retrovirus-containing supernatant may start 24 hours after transfection by replacing the medium with fresh culture medium. A 24 hours collection period is usually sufficient for high titer supernatant. For this, the culture medium is gently withdrawn from the dish, centrifuged at 3000xg, and aliquoted. Aliquots can be stored at -80°C and are stable for at least six months.

Alternatively, the cells may be trypsinized to select for transfected cells and transferred to a fresh plate containing medium supplemented with 2.5 mg/ml of puromycin. Selection must be performed for a minimum period of 8 days. After this period, medium is replaced with normal medium without puromycin. 24 hours the retrovirus-containing supernatant is collected by replacing the medium with fresh culture medium. Supernatant is centrifuged at 3000xg and stored in aliquots at -80°C.

The arrayed retrovirus libraries described above can be used in disease relevant cellular assays to screen for knock-down induced phenotypes. Also these retroviral sRNA expression libraries can be used to generate collections of cell lines with individual or multiple genes that are stably knocked-down.

The individual knockdown retroviruses, like the other vector-based members of the aforesaid libraries, can be pooled to various degrees, that is in one large pool of retroviruses, or subsets of such retrovirus pool. The sets and subsets of pools can then be used in disease relevant assays where the phenotype is a selectable phenotype such as a flow cytometric phenotype (e.g. induction or repression of marker) or growth advantage phenotype.

### Example 15 siRNA To Suppress Expression Of Exogenous Genes During Virus Production.

Non-limiting examples using sRNA to knock down expression of exogenous genes during adenovirus and retrovirus production are presented below.

### Adenovirus Production:

The unique sequences between the transcription start of the CMV promoter and the polylinker of the adenoviral vector pIPspAdapt 6 are used to down-modulate genes. These sequences are always part of the transcribed mRNA and independent of the sequence of the toxic protein and are depicted below (SEQ ID NO: 97)

Alternatively, unique nucleic acid sequences in the mRNA encoding the toxic protein can be used as target sequences for knock-down constructs. Polynucleotide sequences of the invention targeted against these sequences or a vector encoding the polynucleotide sequences are co-transfected with the viral plasmid(s) into the packaging cells to suppress exogenous protein production. Alternatively, the polynucleotide sequences of the invention, or a vector encoding the polynucleotide sequences, can be stably integrated into the packaging cells.

### Production of retrovirus:

The retroviral vector containing the exogenous gene contains a packaging signal surrounded by two splice sites, the splice donor site and the splice acceptor site. Replication of the viral RNA requires the transcription of the full vector. However, the packaging signal is spliced out to produce mRNA before protein production. To allow replication of the viral RNA, but suppress expression of the exogenous gene, the sequences adjacent to the splice donor site and splice acceptor site are used as a target for the polynucleotide sequences of the invention. The first sequence of said polynucleotide sequence consists of the complement of these two portions that are covalently linked and that form a unique sequence in the mRNA coding for the toxic protein. The first portion is complementary to about 9 to 11 nucleotides of the sequence upstream of the splice donor sequence, and the second portion is complementary to about 9 to 11 nucleotides of the sequence downstream of the splice acceptor sequence. When the RNA is spliced to produce a mRNA, the sequence upstream of the splice donor site and the sequence downstream of the splice acceptor site are joined together and will become the target complementing the above described polynucleotide thereby providing for the degradation and knock-down of the spliced mRNA sequence. The unspliced proviral RNA sequence will not be knocked-down, as the two target sequences are not linked.

Again, the polynucleotide sequences as described above or a vector encoding the polynucleotide sequences are co-transfected with the viral plasmid(s) into the packaging cells to suppress exogenous protein production. Alternatively, the polynucleotide sequences can be stably integrated into the packaging cells.

## Claims

1. A vector useful for transfecting host cells comprising an isolated polynucleotide useful for the degradation of a specific mRNA molecule in a host cell, comprising a first guide polynucleotide sequence consisting of 19 to 22 nucleotides and complementary to 19 to 22 nucleotides of said specific mRNA sequence in said host cell, said first sequence being covalently linked to a second sequence capable of forming a stem-loop structure when said second sequence is an RNA sequence, wherein said first sequence consists essentially of a single stranded DNA equivalent of an RNA sequence, and wherein said polynucleotide comprises further a third sequence consisting of 19 to 22 nucleotides complementary to said first sequence and covalently linked to the distal end of said second sequence and wherein all nucleotides in said first and third sequences base pair with each other, all of which nucleotides of said first and third sequences correspond to a sequence found in said specific mRNA, and wherein a pol III promoter is positioned upstream of said first sequence.

2. A vector as claimed in claim 1, wherein said second sequence contains at least one enzymatic cleavage site.

3. A vector as claimed in claim 2, having at least two enzymatic cleavage sites.

4. A vector as claimed in claims 2 or 3, wherein at least one of said enzymatic cleavage sites is located in the stem portion of Said stem-loop structure.

5. A vector as claimed in claims 2, 3 or 4, wherein at least one enzymatic cleavage site is inserted between said first sequence and said second sequence.

6. A vector as claimed in any of the claims 1 to 5, wherein the function of the expression products associated with said first sequence is unknown.

7. A vector as claimed in any one of claims 1 to 6, wherein said promoter is selected from the group consisting of 5S rRNA, tRNAs, VA RNAs, Alu RNAs, or U6 small nuclear RNA.

8. A vector as claimed in any of the claims 1 to 7, which is an adenoviral vector.

9. An in vitro method for reducing the amount of at least one RNA sequence present in a host cell comprising transfecting said cell with a vector as claimed in claims 1 to 8 wherein said first sequence is complementary with said RNA sequence.

10. An in vitro method of determining the function of a naturally occurring polynucleotide sequence comprising transfecting a host cell with a vector according to claims 1 to 8, said vector including a polynucleotide sequence complementary to said naturally occurring polynucleotide, and detecting a change in cellular phenotype.

11. A library of vectors according to claims 1 to 8.

12. A library as claimed in claim 11 wherein said vectors are viral vectors.

13. A library as claimed in claim 12, wherein said vectors are adenoviral vectors, preferably said adenoviral vectors are replication defective.

14. Vector as claimed in claims 1 to 5 for use as a medicament.

15. Use of a vector as claimed in claims 1 to 5 for the preparation of a medicament for lowering the amounts of specific RNA or protein translated from RNA.

## Patentansprüche

1. Vektor, der sich für die Transfektion von Wirtszellen eignet, umfassend ein isoliertes Polynukleotid, das sich für den Abbau eines spezifischen mRNA-Moleküls in einer Wirtszelle eignet, umfassend eine erste Leit-Polynukleotidsequenz, die aus 19 bis 22 Nukleotiden besteht und zu 19 bis 22 Nukleotiden der spezifischen mRNA-Sequenz in der Wirtszelle komplementär ist, wobei die erste Sequenz mit einer zweiten Sequenz, die zur Bildung einer Stamm-Schleife-Struktur fähig ist, wenn es sich bei der zweiten Sequenz um eine RNA-Sequenz handelt, kovalent gebunden ist, wobei die erste Sequenz im Wesentlichen aus einem Einzelstrang-DNA-Äquivalent einer RNA-Sequenz besteht und wobei das Polynukleotid weiterhin eine dritte Sequenz, die aus 19 bis 22 Nukleotiden besteht, die zu der ersten Sequenz komplementär ist und mit dem distalen Ende der zweiten Sequenz kovalent gebunden ist, umfaßt und wobei alle Nukleotide in der ersten und dritten Sequenz miteinander eine Basenpaarung eingehen wobei die Nukleotide der ersten und dritten Sequenz alle einer in der spezifischen mRNA gefundenen Sequenz entsprechen und wobei ein pol-III-Promotor stromaufwärts von der ersten Sequenz liegt.

2. Vektor nach Anspruch 1, wobei die zweite Sequenz mindestens eine Enzymspaltstelle enthält.

3. Vektor nach Anspruch 2, mit mindestens zwei Enzymspaltstellen.

4. Vektor nach Anspruch 2 oder 3, wobei mindestens eine der Enzymspaltstellen im Stammabschnitt der Stamm-Schleife-Struktur liegt.

5. Vektor nach den Ansprüchen 2, 3 oder 4, wobei mindestens eine Enzymspaltstelle zwischen der ersten Sequenz und der zweiten Sequenz insertiert ist.

6. Vektor nach einem der Ansprüche 1 bis 5, wobei die Funktion der mit der ersten Sequenz assoziierten Expressionsprodukte unbekannt ist.

7. Vektor nach einem der Ansprüche 1 bis 6, wobei der Promoter aus der Gruppe 5S-rRNA, tRNAs, VA-RNAs, Alu-RNAs oder U6 kleine nukleäre RNA stammt.

8. Vektor nach einem der Ansprüche 1-7, welcher ein Adenovirusvektor ist.

9. In-vitro-Verfahren zur Reduktion der Menge von mindestens einer in einer Wirtszelle befindlichen RNA-Sequenz, bei dem man die Zelle mit einem Vektor nach den Ansprüchen 1-8, wobei die erste Sequenz zu der RNA-Sequenz komplementär ist, transfiziert.

10. In-vitro-Verfahren zur Bestimmung der Funktion einer natürlich vorkommenden Polynukleotidsequenz, bei dem man eine Wirtszelle mit einem Vektor nach den Ansprüchen 1 bis 8 transfiziert, wobei der Vektor eine Polynukleotidsequenz, die zu dem natürlich vorkommenden Polynukleotid komplementär ist, beinhaltet, und man eine Veränderung des Zellphänotyps nachweist.

11. Bibliothek von Vektoren nach den Ansprüchen 1 bis 8.

12. Bibliothek nach Anspruch 11, wobei es sich bei den Vektoren um virale Vektoren handelt.

13. Bibliothek nach Anspruch 12, wobei es sich bei den Vektoren um Adenovirus-Vektoren handelt, wobei die Adenovirus-Vektoren vorzugsweise replikationsdefizient sind.

14. Vektor nach den Ansprüchen 1 bis 5 zur Verwendung als Arzneimittel.

15. Verwendung eines Vektors nach den Ansprüchen 1 bis 5 zur Herstellung eines Arzneimittels für die Verringerung der Mengen an spezifischer RNA oder an von RNA translatiertem Protein.

## Revendications

1. Vecteur utile pour la transfection de cellules hôtes comprenant un polynucléotide isolé utile pour la dégradation d'une molécule d'ARNm spécifique dans une cellule hôte, comprenant une première séquence de guidage polynucléotidique constituée de 19 à 22 nucléotides et complémentaire à 19 à 22 nucléotides de ladite séquence spécifique d'ARNm dans ladite cellule hôte, ladite première séquence étant liée de façon covalente à une deuxième séquence capable de former une structure tige-boucle lorsque ladite deuxième séquence est une séquence d'ARN, ladite première séquence étant essentiellement constituée d'un ADN simple brin équivalent à une séquence d'ARN, et ledit polynucléotide comprenant en outre une troisième séquence constituée de 19 à 22 nucléotides complémentaire à ladite première séquence et liée de façon covalente à l'extrémité distale de ladite deuxième séquence et tous les nucléotides dans lesdites première et troisième séquences s'appariant les uns avec les autres selon le principe de la complémentarité des bases la totalité desdits nucléotides desdites première et troisième séquences correspondant à une séquence trouvée dans ledit ARNm spécifique, et un promoteur pol III étant positionné en amont de ladite première séquence.

2. Vecteur selon la revendication 1, dans lequel ladite deuxième séquence contient au moins un site de clivage enzymatique.

3. Vecteur selon la revendication 2, ayant au moins deux sites de clivage enzymatique.

4. Vecteur selon les revendications 2 ou 3, dans lequel au moins l'un desdits sites de clivage enzymatique est situé dans la partie tige de ladite structure tige-boucle.

5. Vecteur selon les revendications 2, 3 ou 4, dans lequel au moins un site de clivage enzymatique est inséré entre ladite première séquence et ladite deuxième séquence.

6. Vecteur selon l'une quelconque des revendications 1 à 5, dans lequel la fonction des produits d'expression associés à ladite première séquence est inconnue.

7. Vecteur selon l'une quelconque des revendications 1 à 6, dans lequel ledit promoteur est choisi dans le groupe constitué des promoteurs de l'ARNr 5S, des ARNt, des ARN VA, des ARN Alu ou du petit ARN nucléaire U6.

8. Vecteur selon l'une quelconque des revendications 1 à 7, qui est un vecteur adénoviral.

9. Procédé in vitro pour la réduction de la quantité d'au moins une séquence d'ARN présente dans une cellule hôte, qui comprend l'étape consistant à transfecter ladite cellule avec un vecteur selon les revendications 1 à 8, ladite première séquence étant complémentaire à ladite séquence d'ARN.

10. Procédé in vitro de détermination de la fonction d'une séquence polynucléotidique naturellement présente, qui comprend les étapes consistant à transfecter une cellule hôte avec un vecteur selon les revendications 1 à 8, ledit vecteur incluant une séquence polynucléotidique complémentaire audit polynucléotide naturellement présent, et à détecter un changement de phénotype cellulaire.

11. Banque de vecteurs selon les revendications 1 à 8.

12. Banque selon la revendication 11, dans laquelle lesdits vecteurs sont des vecteurs viraux.

13. Banque selon la revendication 12, dans laquelle lesdits vecteurs sont des vecteurs adénoviraux, de préférence lesdits vecteurs adénoviraux sont défectifs pour la réplication.

14. Vecteur selon les revendications 1 à 5, destiné à être utilisé en tant que médicament.

15. Utilisation d'un vecteur selon les revendications 1 à 5 pour la préparation d'un médicament destiné à abaisser les quantités d'un ARN spécifique ou d'une protéine spécifique traduite à partir d'ARN.


### SEQUENCE LISTING

<110> Galapagos Genomics NV
<120> siRNA Knockout Assay Method and Constructs
<130> Gal 011
<150> US 60/317,229
   <151> 2001-09-01
<150> US 60/385,733
   <151> 2002-06-04
<160> 97
<170> PatentIn version 3.1
<210> 1
   <211> 21
   <212> DNA
   <213> Homo sapiens
<400> 1
   actatacaac ctactacctc a
   21
<210> 2
   <211> 26
   <212> DNA
   <213> Artificial sequence
<220>
   <223> human primer with restriction site
<400> 2
   ctagtactat acaacctact acctca
   26
<210> 3
   <211> 26
   <212> DNA
   <213> Artificial sequence
<220>
   <223> human primer with restriction site
<400> 3
   ctagtgaggt agtaggttgt atagta
   26
<210> 4
   <211> 22
   <212> DNA
   <213> Photinia pyrifolia
<400> 4
   catcttcgac gcaggtgtcg ca
   22
<210> 5
   <211> 22
   <212> DNA
   <213> Photinia pyrifolia
<400> 5
   ccatcgttca gatccttatc ga
   22
<210> 6
   <211> 19
   <212> DNA
   <213> Photinia pyrifolia
<400> 6
   cgtacgcgga atacttcga
   19
<210> 7
   <211> 21
   <212> RNA
   <213> Artificial sequence
<220>
   <223> siRNA against fire fly GL2
<400> 7
   cguacgcgga auacuucgau u
   21
<210> 8
   <211> 21
   <212> RNA
   <213> Artificial sequence
<220>
   <223> siRNA against fire fly GL2
<400> 8
   ucgaaguauu ccgcguacgu u
   21
<210> 9
   <211> 19
   <212> DNA
   <213> Photinia pyrifolia
<400> 9
   cttacgctga gtacttcga
   19
<210> 10
   <211> 21
   <212> RNA
   <213> Artificial sequence
<220>
   <223> siRNA against fire fly GL3
<400> 10
   cuuacgcuga guacuucgau u
   21
<210> 11
   <211> 21
   <212> RNA
   <213> Artificial sequence
<220>
   <223> siRNA against fire fly GL3
<400> 11
   ucgaaguacu cagcguaagu u
   21
<210> 12
   <211> 19
   <212> DNA
   <213> Homo sapiens
<400> 12
   tatacaacct actacctca
   19
<210> 13
   <211> 21
   <212> RNA
   <213> Artificial sequence
<220>
   <223> siRNA against human Let-7
<400> 13
   uauacaaccu acuaccucau u
   21
<210> 14
   <211> 21
   <212> RNA
   <213> Artificial sequence
<220>
   <223> siRNA against human Let-7
<400> 14
   ugagguagua gguuguauau u
   21
<210> 15
   <211> 70
   <212> RNA
   <213> Homo sapiens
<400> 15
<210> 16
   <211> 11
   <212> RNA
   <213> Homo sapiens
<400> 16
   ggccuuuggg g
   11
<210> 17
   <211> 12
   <212> RNA
   <213> Homo sapiens
<400> 17
   ccgugaaguc cu
   12
<210> 18
   <211> 91
   <212> RNA
   <213> Homo sapiens
<400> 18
<210> 19
   <211> 78
   <212> DNA
   <213> Artificial sequence
<220>
   <223> oligo based on human Let-7
<400> 19
<210> 20 '
   <211> 66
   <212> DNA
   <213> Artificial sequence
<220>
   <223> oligo based on human Let-7
<400> 20
<210> 21
   <211> 78
   <212> DNA
   <213> Artificial sequence
<220>
   <223> oligo based on fire fly GL3
<400> 21
<210> 22
   <211> 67
   <212> DNA
   <213> Artificial sequence
<220>
   <223> oligo based on fire fly GL3
<400> 22
<210> 23
   <211> 129
   <212> DNA
   <213> Artificial sequence
<220>
   <223> oligo based on human Let-7 with T7 promoter and restrict ion sites
<400> 23
<210> 24
   <211> 129
   <212> DNA
   <213> Artificial sequence
<220>
   <223> oligo based on human Let-7 with T7 promoter and restrict ion sites
<400> 24
<210> 25
   <211> 129
   <212> DNA
   <213> Artificial sequence
<220>
<223> oligo based on fire fly GL3 with T7 promoter and restric tion site s
<400> 25
<210> 26
   <211> 129
   <212> DNA
   <213> Artificial sequence
<220>
   <223> oligo based on fire fly GL3 with T7 promoter and restric tion site s
<400> 26
<210> 27
   <211> 70
   <212> RNA
   <213> Homo sapiens
<400> 27
<210> 28
   <211> 70
   <212> RNA
   <213> Artificial sequence
<220>
   <223> chimeric RNA against fire fly GL3 with let7 loop
<400> 28
<210> 29
   <211> 91
   <212> RNA
   <213> Artificial sequence
<220>
   <223> chimeric RNA against fire fly GL3 with let7 loop
<400> 29
<210> 30
   <211> 12
   <212> RNA
   <213> Artificial sequence
<220>
   <223> Loop sequence based on human Let-7 loop sequence
<400> 30
   guuugcuaua ac
   12
<210> 31
   <211> 14
   <212> RNA
   <213> Artificial sequence
<220>
   <223> loop sequence based on human Let-7
<400> 31
   guugcucugc uaac
   14
<210> 32
   <211> 21
   <212> DNA
   <213> Homo sapiens
<400> 32
   tgaggtagta ggttgtatag t
   21
<210> 33
   <211> 18
   <212> DNA
   <213> Homo sapiens
<400> 33
   tgaggtagta ggttgtat
   18
<210> 34
   <211> 17
   <212> DNA
   <213> Homo sapiens
<400> 34
   tgaggtagta ggttgta
   17
<210> 35
   <211> 81
   <212> RNA
   <213> Homo sapiens
<400> 35
<210> 36
   <211> 80
   <212> RNA
   <213> Homo sapiens
<400> 36
<210> 37
   <211> 83
   <212> RNA
   <213> Homo sapiens
<400> 37
<210> 38
   <211> 102
   <212> RNA
   <213> Homo sapiens
<400> 38
<210> 39
   <211> 92
   <212> RNA
   <213> Homo sapiens
<400> 39
<210> 40
   <211> 96
   <212> RNA
   <213> Homo sapiens
<400> 40
<210> 41
   <211> 122
   <212> RNA
   <213> Homo sapiens
<400> 41
<210> 42
   <211> 32
   <212> DNA
   <213> Artificial sequence
<220>
   <223> human primer with restriction site
<400> 42
   gcacgttcta gagaatccct gtgcccttgg tg
   32
<210> 43
   <211> 32
   <212> DNA
   <213> Artificial sequence
<220>
   <223> human primer with restriction site
<400> 43
   gcacgttcta gaccgtgaag ccgctactca gc
   32
<210> 44
   <211> 32
   <212> DNA
   <213> Artificial sequence
<220>
   <223> human primer with restriction site
<400> 44
   gcacgttcta gagggttgac agtcgtatct gc
   32
<210> 45
   <211> 32
   <212> DNA
   <213> Artificial sequence
<220>
   <223> human primer with restriction site
<400> 45
   ccgtgcaagc tttgtcagac ttctcagtgt ag
   32
<210> 46
   <211> 32
   <212> DNA
   <213> Artificial sequence
<220>
   <223> human primer with restriction site
<400> 46
   ccgtgcaagc ttcctgccac tgagctggcc ag
   32
<210> 47
   <211> 19
   <212> DNA
   <213> Photinia pyrifolia
<400> 47
   aagcgaccaa cgccttgat
   19
<210> 48
   <211> 21
   <212> DNA
   <213> Photinia pyrifolia
<400> 48
   ttcgtcttcg tcccagtaag c
   21
<210> 49
   <211> 30
   <212> DNA
   <213> Photinia pyrifolia
<400> 49
   atgtctccag aatgtagcca tccatccttg 30
<210> 50
   <211> 56
   <212> DNA
   <213> Artificial sequence
<220>
   <223> primer
<220>
   <221> misc_feature
   <222> (22) . . (40)
   <223> N means any nucleotide
<400> 50
   tctgccctgc tatgggataa cnnnnnnnnn nnnnnnnnnn tcctgaagtg gctgta 56
<210> 51
   <211> 56
   <212> DNA
   <213> Artificial sequence
<220>
   <223> primer
<220>
   <221> misc_feature
   <222> (24)..(42)
   <223> N means any nucleotide
<400> 51
   catagcaggg cagagcccca aacnnnnnnn nnnnnnnnnn nnccccaaag ggcagt 56
<210> 52
   <211> 19
   <212> DNA
   <213> Homo sapiens
<400> 52
   tatacaacct actacctca
   19
<210> 53
   <211> 19
   <212> DNA
   <213> Aequorea victoria
<400> 53
   gctgaccctg aagttcatc
   19
<210> 54
   <211> 19
   <212> DNA
   <213> Homo sapiens
<400> 54
   cagatccagt ggttgcaga
   19
<210> 55
   <211> 19
   <212> DNA
   <213> Homo sapiens
<400> 55
   ggaggtgcca tgctatgtg
   19
<210> 56
   <211> 34
   <212> DNA
   <213> Artificial sequence
<220>
   <223> human primer with restriction site
<400> 56
   gcacgttcta gaaggtcggg caggaagagg gcct 34
<210> 57
   <211> 34
   <212> DNA
   <213> Artificial sequence
<220>
   <223> human primer with restriction site
<400> 57
   ccgtgcaagc tttggtaaac cgtgcaccgg cgta 34
<210> 58
   <211> 19
   <212> DNA
   <213> Artificial sequence
<220>
   <223> guide sequence based on human let7
<400> 58
   tgaggtagta ggttgtata 19
<210> 59
   <211> 19
   <212> DNA
   <213> Artificial sequence
<220>
   <223> guide sequence based on human let7
<400> 59
   gaggtagtag gttgtatag 19
<210> 60
   <211> 43
   <212> DNA
   <213> Artificial sequence
<220>
   <223> primer based on human Let-7 and artifical loop
<400> 60
   gtttgctata actatacaac ctactacttt ttacatcagg ttg
   43
<210> 61
   <211> 46
   <212> DNA
   <213> Artificial sequence
<220>
   <223> primer based on human Let-7 with artificial loop
<400> 61
   gttatagcaa actatacaac ctactacctc ggtgtttcgt cctttc
   46
<210> 62
   <211> 50
   <212> RNA
   <213> Artificial sequence
<220>
   <223> chimeric RNA against human Let-7
<400> 62
   gagguaguag guuguauagu uugcuauaac uauacaaccu acuacuuuuu
   50
<210> 63
   <211> 45
   <212> DNA
   <213> Artificial sequence
<220>
   <223> primer based on human Let-7
<400> 63
   gttgtgctat caactataca acctactact ttttacatca ggttg
   45
<210> 64
   <211> 48
   <212> DNA
   <213> Artificial sequence
<220>
   <223> primer based on humanLet-7
<400> 64
   gttgatagca caactataca acctactacc tcggtgtttc gtcctttc
   48
<210> 65
   <211> 52
   <212> RNA
   <213> Artificial sequence
<220>
   <223> chimeric RNA against human Let-7
<400> 65
   gagguaguag guuguauagu ugugcuauca acuauacaac cuacuacuuu uu
   52
<210> 66
   <211> 14
   <212> RNA
   <213> Artificial sequence
<220>
   <223> Loop sequence based on human Let-7 loop sequence
<400> 66
   guugugcuau caac
   14
<210> 67
   <211> 23
   <212> DNA
   <213> Artificial sequence
<220>
   <223> human primer with restriction site
<400> 67
   ccgaagcttg ggcatgctgt gat
   23
<210> 68
   <211> 30
   <212> DNA
   <213> Artificial sequence
<220>
   <223> human primer with restriction site
<400> 68
   ccgaagctta tgaaaagccc tgctttgcaa
   30
<210> 69
   <211> 29
   <212> DNA
   <213> Artificial sequence
<220>
   <223> human primer with restriction site
<400> 69
   ccgaagcttc cagagctggc ttatatcag
   29
<210> 70
   <211> 30
   <212> DNA
   <213> Artificial sequence
<220>
   <223> human primer with restriction site
<400> 70
   ccgaagctta ctgattctgc atatgaatgg
   30
<210> 71
   <211> 31
   <212> DNA
   <213> Artificial sequence
<220>
   <223> human primer with restriction site
<400> 71
   ctgagctagc ttgttagagc aacagcctag a
   31
<210> 72
   <211> 32
   <212> DNA
   <213> Artificial sequence
<220>
   <223> human primer with restriction site
<400> 72
   ctgagctagc tcagctattg ggaacctgag gt
   32
<210> 73
   <211> 31
   <212> DNA
   <213> Artificial sequence
<220>
   <223> human primer with restriction site
<400> 73
   ctgagctagc aatacactgc tcagtgtgca a
   31
<210> 74
   <211> 31
   <212> DNA
   <213> Artificial sequence
<220>
   <223> human primer with restriction site
<400> 74
   ctgagctagc gattgaacat aggatcgata t
   31
<210> 75
   <211> 30
   <212> RNA
   <213> Homo sapiens
<400> 75
   guuuggggcu cugcccugcu augggauaac
   30
<210> 76
   <211> 28
   <212> DNA
   <213> Artificial sequence
<220>
   <223> oligo based on human Let-7
<400> 76
   gtatagtttg gggctctgcc ctgctatg
   28
<210> 77
   <211> 27
   <212> DNA
   <213> Artificial sequence
<220>
   <223> oligo based on human Let-7
<400> 77
   gtatagttat cccatagcag ggcagag
   27
<210> 78
   <211> 28
   <212> DNA
   <213> Artificial sequence
<220>
   <223> oligo based on fire fly GL3
<400> 78
   gtaaggtttg gggctctgcc ctgctatg
   28
<210> 79
   <211> 27
   <212> DNA
   <213> Artificial sequence
<220>
   <223> oligo based on fire fly GL3
<400> 79
   gtaaggttat cccatagcag ggcagag
   27
<210> 80
   <211> 19
   <212> DNA
   <213> Homo sapiens
<400> 80
   cgatgtgact gccatcatc
   19
<210> 81
   <211> 55
   <212> RNA
   <213> Artificial sequence
<220>
   <223> chimeric RNA against human GNAS
<400> 81
   gaugauggca gucacaucgg uuugcuauaa ccgaugugac ugccaucauc uuuuu
   55
<210> 82
   <211> 21
   <212> DNA
   <213> Artificial sequence
<220>
   <223> primer for human GNAS
<400> 82
   tccctcccga attctatgag c
   21
<210> 83
   <211> 22
   <212> DNA
   <213> Artificial sequence
<220>
   <223> primer for human GNAS
<400> 83
   ggcacagtca atcagctggt ac
   22
<210> 84
   <211> 19
   <212> RNA
   <213> Homo sapiens
<400> 84
   cuuaaagcug guucauauc
   19
<210> 85
   <211> 19
   <212> RNA
   <213> Homo sapiens
<400> 85
   agaaucaucc aucgggauc
   19
<210> 86
   <211> 19
   <212> RNA
   <213> Homo sapiens
<400> 86
   cgucgacuac uggagcuuc
   19
<210> 87
   <211> 19
   <212> RNA
   <213> Homo sapiens
<400> 87
   uacuguugac uucuccagc
   19
<210> 88
   <211> 19
   <212> RNA
   <213> Homo sapiens
<400> 88
   uugaacgacc aaguaacuc
   19
<210> 89
   <211> 19
   <212> RNA
   <213> Homo sapiens
<400> 89
   auucaagaca accugcuac
   19
<210> 90
   <211> 19
   <212> RNA
   <213> Homo sapiens
<400> 90
   ggaaguaauu ggaucaugc
   19
<210> 91
   <211> 20
   <212> DNA
   <213> Artificial sequence
<220>
   <223> primer for human PPAR gamma
<400> 91
   ggagcgggtg aagactcatg
   20
<210> 92
   <211> 20
   <212> DNA
   <213> Artificial sequence
<220>
   <223> primer for human PPAR gamma
<400> 92
   attgtcacgg aacacgtgca 20
<210> 93
   <211> 22
   <212> DNA
   <213> Artificial sequence
<220>
   <223> primer for human M6PR
<400> 93
   cagtggtgat gatctcctgc aa 22
<210> 94
   <211> 19
   <212> DNA
   <213> Artificial sequence
<220>
   <223> primer for human M6PR
<400> 94
   tgccacgctc ctcagacac 19
<210> 95
   <211> 23
   <212> DNA
   <213> Artificial sequence
<220>
   <223> primer for human IKKbeta
<400> 95
   gcagaccgac attgtggact tac 23
<210> 96
   <211> 22
   <212> DNA
   <213> Artificial sequence
<220>
   <223> primer for human IKKbeta
<400> 96
   ccgtaccatt tcctgactgt ca
   22
<210> 97
   <211> 117
   <212> DNA
   <213> Artificial sequence
<220>
   <223> parts of CMV promoter and the polylinker of the adenovir al vector pIPspAdapt 6
<400> 97